(19) Europäisches Patentamt

European Patent Office

Office européen des brevets

(11) **EP 4 501 320 A1**

(12) **EUROPEAN PATENT APPLICATION**

(43) Date of publication:
**05.02.2025 Bulletin 2025/06**

(21) Application number: **23189189.6**

(22) Date of filing: **02.08.2023**

(51) International Patent Classification (IPC):
***A61K 9/51*** (2006.01)  ***A61K 31/436*** (2006.01)

(52) Cooperative Patent Classification (CPC):
**A61K 9/5192; A61K 9/5138; A61K 31/436**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **IQ medical GmbH**
**82031 Grünwald (DE)**

(72) Inventors:
• **JOCHEM, Aljosha-Rakim**
**82031 Grünwald (DE)**
• **CAVELIUS, Christian**
**82031 Grünwald (DE)**
• **HORTSTKOTTE, Elke**
**82031 Grünwald (DE)**

(74) Representative: **Engelhard, Markus**
**Boehmert & Boehmert**
**Anwaltspartnerschaft mbB**
**Pettenkoferstrasse 22**
**80336 München (DE)**

(54) **A METHOD OF PREPARING AN AQUEOUS DISPERSION OF NANOPARTICLES**

(57) The present invention relates to a method of preparing an aqueous dispersion of nanoparticles of a hydrophobic active pharmaceutical ingredient (API). The present invention also relates to a plurality of nanoparticles produced by such method. Furthermore, the present invention relates to uses of such nanoparticles.

**EP 4 501 320 A1**

**Description**

The present invention relates to a method of preparing an aqueous dispersion of nanoparticles of a hydrophobic active pharmaceutical ingredient (API). The present invention also relates to a plurality of nanoparticles produced by such method.

Many pharmaceutically active ingredients have a very low water solubility. In order to show a therapeutic effect, an active pharmaceutical ingredient must be dissolved and thereafter reach the blood stream in sufficient concentrations. For a hardly soluble active pharmaceutical ingredient, only a very small portion, if any, reaches the bloodstream, and therefore, the bioavailability of such badly soluble active ingredient is low.

One way of increasing the bioavailability of a water-insoluble active pharmaceutical ingredient is to produce such ingredient in nanoparticulate form thereby increasing the surface and thus, as a consequence of such increased surface, their solubility. As a result thereof, the dosage of a pharmaceutically active ingredient that is necessary to reach a defined concentration in the bloodstream can be reduced.

One way of producing of producing pharmaceutical nanoparticles is by way of wet grinding. This process is iterative, discontinuous, limited to heat-resistant and water-resistant active agents and therefore costly. The active ingredient in nanoparticles produced by this method is typically in crystalline form which may, yet again, reduce their solubility.

One way of avoiding some of the disadvantages of wet grinding is to produce nanoparticles by means of precipitation. The active pharmaceutical ingredient (API) is typically dissolved in a non-aqueous solvent in which it is soluble. The resultant solution is subsequently mixed with a surplus of non-solvent for the API, i.e. typically water or an aqueous solution in which the API is not soluble, and this results in a precipitation of the API in particulate form, often times a nanoparticulate form. In precipitation, there is no heat or friction generated, as would be the case with grinding, and the method of precipitation is also useful for active ingredients that are heat-sensitive, such as peptides or hormones. However, problems remain with such production of nanoparticles, in that nanoparticles produced in such a manner may tend to form agglomerates that are not useful and need further work-up. Such problems occur even when, in addition to the active pharmaceutical ingredient, a stabilizer is included. This is because during the mixing step, various interfaces between the API, the stabilizer, and the surrounding solvent(s) as well as a hydration shell need to be formed which often results in the agglomeration of particles.

It is therefore an object of the present invention to provide for a methodology of producing nanoparticles of hydrophobic active pharmaceutical ingredients (API) that are poorly soluble or insoluble in water. It is furthermore an object of the present invention to provide a methodology of producing nanoparticles that comprise not only of a hydrophobic active pharmaceutical ingredient but also a stabilizing agent. It is furthermore an object of the present invention to provide for such methodology of producing API- and stabilizing agent-containing nanoparticles, that is easy to perform and results in nanoparticles comprising such API and a stabilizing agent and having a defined size with a limited size distribution. It is furthermore an object of the present invention to provide for such methodology of producing API- and stabilizing agent-containing nanoparticles, that reduces the formation of agglomerates.

All these objects are solved by a method of preparing an aqueous dispersion of nanoparticles of a hydrophobic active pharmaceutical ingredient (API) wherein said nanoparticles have a defined size range and comprise said hydrophobic active ingredient and at least one stabilizing agent, said stabilizing agent being a polymeric stabilizing agent having ionizable groups and/or ionic groups; said method comprising the steps:

a) Providing in a single first space a first composition comprising

- a hydrophobic active pharmaceutical ingredient (API) dissolved in a first amount of a suitable non-aqueous solvent;

- a polymeric stabilizing agent having ionizable groups that are ionizable by the addition of a suitable acid or suitable base, and/or having ionic groups, said polymeric stabilizing agent being also dissolved in said first amount of said non-aqueous solvent;

- a second amount of a suitable hydrophilic solvent, e.g. water or aqueous solution;

  wherein, in said first composition, said suitable non-aqueous solvent and said suitable hydrophilic solvent become mixed, and wherein said first amount and said second amount in said first composition have a volume ratio in the range of from 20:1 to 2:1, preferably from 10:1 to 4:1;

  wherein said second amount of a hydrophilic solvent additionally contains a third amount of a suitable acid or a suitable base, wherein said third amount of a suitable acid or suitable base is defined by the number of ionizable groups N in said polymeric stabilizing agent, such that said third amount of suitable acid or base achieves an ionization of 10% to 100% of N;

b) providing in a single second space a second composition comprising

- a suitable hydrophilic solvent, e.g. water or an aqueous solution; wherein, optionally, said suitable hydrophilic solvent further comprises one or several further solutes, such as a polymeric stabilizing agent which may be the same or different from the polymeric stabilizing agent provided in said first composition, and/or optionally further comprises one or several surfactants, and/or combinations of one or several further solutes and of one or several surfactants;

wherein said first and second space are separate from each other, and wherein the order of steps a) and b) is ab or ba or both steps are performed synchronously or concomitantly;

c) mixing said first composition and said second composition together in a volume ratio of from 1:2 to 1:50, preferably from 1:3 to 1:20; more preferably from 1:4 to 1:10, thereby generating a stable aqueous dispersion of nanoparticles of said active pharmaceutical ingredient (API) stabilized by said polymeric stabilizing agent.

In one embodiment, said polymeric stabilizing agent having ionizable groups and/or ionic groups is a poly(methacrylate), preferably selected from neutral protonizable poly(methacrylates), cationic poly(methacrylates), and anionic poly(methacrylates); or said polymeric stabilizing agent is a polyethyleneimine, poly(4-vinylpyridine), poly(L-lysine), or poly(L-arginine).

In one embodiment, said polymeric stabilizing agent is a poly(methacrylate), and said poly(methacrylate) is a neutral protonizable or cationic poly(methacrylate) selected from poly(butyl methacrylate-co-(2-dimethylaminoethyl) methacrylate-co-methyl methacrylate),methyl methacrylate and diethylaminoethyl methacrylate copolymer, ammonio methacrylate copolymer type B, and ammonio methacrylate copolymer type A.

In one embodiment, said polymeric stabilizing agent is a poly(methacrylate), and said poly(methacrylate) is an anionic poly(methacrylate) selected from methacrylic acid-methylmethacrylate-copolymers, preferably having a ratio of free carboxyl groups to ester groups in a range of from 1:1 to 1:5, more preferably from 1:1 to 1:2.

In one embodiment, said hydrophobic active pharmaceutical ingredient (API) is a drug that is insoluble in water and aqueous solutions and that is, preferably, selected from macrolides, non-steroidal anti-inflammatory drugs, hormones, antibodies, steroids, anti-cancer agents, opioid drugs, and mixtures thereof, wherein more preferably said macrolide is selected from macrolide immune suppressants, macrolide antibiotics, and macrolide antifungals; wherein more preferably, said hydrophobic active pharmaceutical ingredient (API) is selected from tacrolimus, sirolimus, everolimus, pimecrolimus, erythromycin, clarithromycin, azithromycin, roxithromycin, josamycin, spiramycin, telithromycin, tylosin, fidaxomicin, nystatin, natamycin, amphotericin B; in particular, acetylsalicylic acid, ibuprofen, dexibuprofen, flurbiprofen, naproxen, ketoprofen, tiaprofenic acid, diclofenac, indomethacin, acemetacin, flufenamic acid, mefenamic acid, oxicams, e.g. piroxicam, tenoxicam, meloxicam, lornoxicam, nabumeton, rofecoxib, parecoxib, etoricoxib, celecoxib, alpha-atrial natriuretic peptide, arginine vasopressin, atropine, augrnerosen, atorvastatin, bevacizumab, calcitonins, chorionic gonadotropins, corticotropin, desmopressin, epibatidine, cetuxirnab, exenatide, trastuzumab, adalimumab, human insulin, ketoconazole, lanreotide, lutropin alpha, metoprolol, minoxidil, nesiritide, octreotide, paclitaxel, paracetamol, pegaptanib, recombinant follicle stimulating hormone, recombinant growth factors, infliximab, rituximab, sermorelin, somatotropin, a taxane derivative, taxol, teriparatide acetate, thyrotropin, triclosan, urofollitropin, omalizumab, actinomycin D, albendazole, aldosterone, alprazolam, amiodarone, amitriptyline, amprenavir, asimadoline, atorvastatin, bunitrolol, buspirone, camptothecin, carbamazepine, carvedilol, celiprolol, cyclosporine A, cimetidine, clotrimazole, colchicine, cortisone, daunorubicin, debrisoquine, dexamethasone, diazepam, digitoxin, digoxin, diltiazem, docetaxel, domperidone, doxorubicin, efavirenz, epirubicin, erythromycin, ergotamine, estradiol, estradiol glucuronide, erlotinib, etoposide, phenytoin, fentanyl, felodipine, phenothiazines, fexofenadine, fluoroquinolones, fluorouracil, gentamicin, griseofulvin, hydrocortisone, imatinib, indinavir, itraconazole, ivermectin, ketoconazole, kaempferol, levofloxacin, lidocaine, loperamide, losartan, lovastatin, mebendazole, methylprednisolone, methotrexate, mibefradil, midazolam, nisoldipine, morphine, nelfinavir, nicardipine, nitrendipine, nifedipine, ondansetron, paclitaxel, pentazocine, praziquantel, prednisolone, prednisone, quercetin, quinidine, ranitidine, rifabutin, rifampicin, ritonavir, saquinavir, sulfame-thizole, tamoxifen, talinolol, teniposide, terfenadine, tetracycline, topotecan, triamcinolone, valspodar, verapamil, vinblastine, vincristine, vindesine, zopiclone, and mixtures thereof.

In one embodiment, the hydrophobic active pharmaceutical ingredient (API) is a macrolide, and the macrolide is selected from macrolide immune suppressants, macrolide antibiotics, and macrolide antifungals, wherein the macrolide immune suppressant is selected from tacrolimus, sirolimus, everolimus and pimecrolimus; the macrolide antibiotic is selected from erythromycin, clarithromycin, azithromycin, roxithromycin, josamycin, spiramycin, telithromycin, tylosin, and fidaxomicin; and the macrolide antifungal is selected from polyenes, in particular nystatin, natamycin, and amphotericin B.

In one embodiment, said suitable non-aqueous solvent is selected from acetone, dimethyl sulfoxide, ethanol, methanol, 1-propanol, 2-propanol, tetrahydrofuran, acetic acid, acetonitrile, anisole, 1-butanol, 2-butanol, t-butyl alcohol, 2-methyl-1-propanol, 3-methyl-1-butanol, 1-pentanol, butyl acetate, chloroform, cyclohexane, 1,1,-diethoxypropane, 1,1-dimethoxymethane, 1,2-dimethoxyethane, 1,4-dioxane, 2,2-dimethoxypropane, dichloromethane, diethyl ether, di-iso-propyl ether, dimethylformamide, 2-ethoxyethanol, ethyl acetate, ethyl formate, ethylene glycol (1,2-ethanediol), formic acid, heptane, hexane, isobutyl acetate, isopropyl acetate, 2-methoxyethanol, 1-methyl-2-pyrrolidone, methyl acetate, methyl t-butyl ether, methyl butyl ketone, methyl cyclohexane, methyl ethyl ketone (MEK), methyl isobutyl ketone, methyl isopropyl ketone, methyl tetrahydrofuran, n-methyl pyrrolidone, pentane, petroleum ether, propyl acetate, pyridine, sulfolane, 2,2,4-trimethylpentan (i-octane), toluol, trichloroacetic acid, trichloroethylene, trifluoracetic acid, xylol, and any combination of the foregoing, wherein, preferably, said suitable non-aqueous solvent is miscible with water and, more preferably is selected from acetone, DMSO, ethanol, methanol, isopropanol, 1-propanol, acetonitrile. 1,4 dioxane, tetrahydrofuran, and mixtures of the foregoing, wherein, even more preferably, said suitable non-aqueous solvent is selected from acetone, DMSO, ethanol and isopropanol.

In one embodiment, said suitable acid is selected from inorganic and organic acids, such as hydrochloric acid, nitric acid, sulfuric acid, acetic acid, oleic acid, propionic acid, pentanoic acid, caprylic acid, succinic acid, benzoic acid, fumaric acid, citric acid, glutamic acid, aspartic acid, citric acid, malic acid; butyric acid, and aminopolycarboxylic acids, such as iminodiacetic acid (IDA), nitrilotriacetic acid (NTA), ethylenediaminetetraacetic acid (EDTA), diethylenetriaminepentaacetic acid (DTPA), Ethylene-bis(oxyethylene-nitrilo)-tetraacetic acid) (EGTA), (1,2-bis(o-aminophenoxy)ethane-N,N,N',N'-tetraacetic acid) (BAPTA), 2,2',2'',2'''-(1,4,7,10-Tetraazacyclododecane-1,4,7,10-tetrayl)tetraacetic acid (DOTA), ethylenediamine-N,N'-bis(2-hydroxyphenylacetic acid) (EDDHA), and Ethylenediamine-N,N'-disuccinic acid (EDDS), and mixtures or combinations of any of the foregoing; and said suitable base is selected from NaOH, KOH, triethylamine, L-lysine, L-histidine, ammonium hydroxide, and mixtures or combinations of any of the foregoing.

In one embodiment, said mixing step occurs by a suitable mixing technology allowing for the intimate and immediate mixing of said first and second compositions, wherein, preferably, said mixing technology is selected from split and recombine mixing (SAR), mixing by impinging-jet mixers, e.g. microjet reactor mixing (MJR), stirring in a vessel, e.g. a batch reactor, mixing by T- and/or Y shaped fluidic mixers, mixing by static mixers, in-line rotor stator mixing, dynamic in-line mixing, and ultrasonic mixing.

In one embodiment, said defined size range is from 10 nm to 500 nm, preferably from 20 nm to more preferably from 20 nm to 250 nm, even more preferably from 50 nm to 200 nm.

In a further aspect, the present invention relates to a plurality of nanoparticles, e.g. a dispersion of nanoparticles, produced by the method according to any of the foregoing embodiments, wherein said nanoparticles comprise a hydrophobic active ingredient (API) and at least one stabilizing agent, having a defined size range from 10 nm to 500 nm, preferably from 20 nm to 350 nm, more preferably from 20 nm to 250 nm, even more preferably from 50 nm to 200 nm, and having one or several of the following structural features:

- Said hydrophobic active pharmaceutical ingredient (API) is present in said nanoparticles in amorphous form and does not show any XRPD signals attributable to crystalline form(s) of said API;
- said nanoparticles exhibit mucoadhesive properties, as measured by an increase in zeta-potential that is observed if said plurality of nanoparticles, e.g. of said dispersion of nanoparticles, are mixed with a 0.5 % (w/w) aqueous suspension of mucin, wherein a mixture of said plurality of nanoparticles, e.g. of said dispersion of nanoparticles, and of said aqueous suspension of mucin has a zeta potential that is increased in comparison to an aqueous suspension of mucin alone without any nanoparticles; and wherein, preferably, said increase in zeta potential is observed with increasing amounts of said plurality of nanoparticles, e.g. of said dispersion of nanoparticles, being mixed with said aqueous suspension of mucin, such that a first mixture of a first amount of said plurality of nanoparticles with a defined amount of a 0.5 % (w/w) aqueous suspension of mucin has a higher zeta potential than a second mixture of a second amount of said plurality of nanoparticles with the same defined amount of a 0.5 % (w/w) aqueous suspension of mucin, if said first amount of said nanoparticles is greater than said second amount of said nanoparticles;
- said hydrophobic active pharmaceutical ingredient (API) is present in said nanoparticles in a solid or immobilized state, as measured by $^1$H-NMR spectroscopy, and does not show any $^1$H-NMR signals attributable to a liquid or dissolved or mobile state of said API.

In one preferred embodiment, the nanoparticles in a dispersion prepared according to the present invention, are characterized by the active pharmaceutical ingredient (API) present in such nanoparticles being in a solid or immobilized state, as measured by $^1$H-NMR spectroscopy, wherein a $^1$H-NMR-spectrum of such dispersion of nanoparticles lacks any characteristic signals of the active pharmaceutical ingredient (API). Hence, the active pharmaceutical ingredient is not present in such nanoparticles in a mobile or dissolved or liquid state.

In yet a further aspect, the present invention also relates to a pharmaceutical composition comprising a plurality of nanoparticles, e.g. a dispersion of nanoparticles, as produced in accordance with the present invention.

In yet a further aspect, the present invention relates to a plurality of nanoparticles, e.g. a dispersion of nanoparticles, as produced in accordance with the present invention, for use in the treatment of a disease or for use as a medicament or medicine.

In one embodiment of this aspect, these nanoparticles are applied or administered to the oral mucosa or other mucosal tissue(s).

In yet a further aspect, the present invention relates to a pharmaceutical composition comprising a plurality of nanoparticles, e.g. a dispersion of nanoparticles, as produced in accordance with the present invention for use in the treatment of a disease or for use as a medicament or medicine.

In one embodiment of this aspect, the pharmaceutical composition is applied or administered to the oral mucosa or other mucosal tissue(s).

In yet a further aspect, the present invention also relates to a use of a plurality of nanoparticles, e.g. a dispersion of nanoparticles, as produced in accordance with the present invention, for the preparation of a medicament or medicine for the treatment of a disease.

In one embodiment of this aspect, these nanoparticles are applied or administered to the oral mucosa or other mucosal tissue(s).

In yet a further aspect, the present invention also relates to a method of treatment of a disease, wherein said method comprises administering to a patient in need thereof, a plurality of nanoparticles, e.g. a dispersion of nanoparticles, as produced in accordance with the present invention, or wherein said method comprises administering to a patient in need thereof, a pharmaceutical composition comprising a plurality of nanoparticles, e.g. a dispersion of nanoparticles, as produced in accordance with the present invention.

In one embodiment of this aspect, these nanoparticles are applied or administered to the oral mucosa or other mucosal tissue(s).

In any of the aforementioned aspects, depending on the type of active pharmaceutical ingredient (API) within such nanoparticles, respective diseases may be treated by such medicament or medicine. Exemplary diseases include, but are not limited to immunological diseases or disorders, inflammatory diseases, cancerous diseases, and infectious diseases.

The term "immunological disease or disorder", as used herein, relates to any immunological disease or disorder known to the person skilled in the art, particularly to diseases, disorders, and/or conditions that can be prevented or treated with a macrolide, such as tacrolimus or sirolimus. For example, an immunological disease or disorder may be selected from transplant rejections, preferably solid organ transplant rejections, such as liver, kidney or heart allograft rejections; autoimmune diseases, such as systemic lupus erythematosus, psoriasis, vitiligo, or lichen ruber planus; infections, such as bacterial infections, e.g. Helicobacter pylori infections or lyme disease; tumors, such as angiofibroma, lymphoma, e.g. T-cell lymphoma, kidney cancer, neuroendocrine tumors, or breast cancer; and inflammatory diseases or conditions, such as atopic eczema, uveitis, dermatitis, Kimura's disease, or inflammations associated with coronary heart disease. For example, an immunological disease or disorder to be prevented or treated with a macrolide, such as tacrolimus or sirolimus, may be selected from transplant rejections, preferably solid organ transplant rejections, such as liver, kidney or heart allograft rejections; autoimmune diseases, such as psoriasis; infections; tumors; and inflammatory diseases, disorders, or conditions, such as atopic eczema, uveitis, or Kimura's disease. In one embodiment, the immunological disease or disorder is selected from transplant rejections, preferably solid organ transplant rejections, such as liver, kidney or heart allograft rejections; autoimmune diseases, such as systemic lupus erythematosus, psoriasis, vitiligo, or lichen ruber planus; infections, such as bacterial infections, e.g. Helicobacter pylori infections or lyme disease; tumors, such as angiofibroma, lymphoma, e.g. T-cell lymphoma, kidney cancer, neuroendocrine tumors, or breast cancer; and inflammatory diseases or conditions, such as atopic eczema, uveitis, dermatitis, Kimura's disease, or inflammations associated with coronary heart disease. In a preferred embodiment, the immunological disease or disorder is a transplant rejection. In one embodiment, the immunological disease or disorder is a transplant rejection, preferably a solid organ transplant rejection, such as a liver, kidney or heart allograft rejection. In one embodiment, the terms "immunological disorder" and "immunological condition" are used interchangeably. In a preferred embodiment, the macrolide is for use in preventing or treating a transplant rejection.

It should be noted that the inventors surprisingly found that nanoparticles produced in accordance with the present invention allow for a release of the API in a controlled manner by appropriate selection of acid or base, its respective strength, and/or the ionization degree of the polymeric stabilizer. In the nanoparticles according to the present invention, the hydrophobic active pharmaceutical ingredient (API) is present therein in amorphous form and does not show any XRPD signals attributable to crystalline form(s) of said API. Moreover, it also does not show any $^1$H-NMR signals attributable to a mobile or liquid state. Moreover, the nanoparticles according to the present invention, for example when provided as a dispersion of a plurality of nanoparticles, can be dried and processed into a solid dosage form whilst maintaining the amorphous state of the API. Nanoparticles according to the present invention display mucoadhesive properties as measured by an increase in zeta potential which is observed for a mixture of a dispersion of nanoparticles and an aqueous suspension of mucin versus an aqueous suspension of mucin alone without any nanoparticles present. Because of their mucoadhesive properties, the nanoparticles according to the present invention are particularly useful for dosage forms that are to be used for buccal or sublingual applications, such as a mucoadhesive film that is applied to the oral mucosa. Moreover, they are also useful for other dosage forms that are to be used for application to any other mucosal tissue(s).

The term "active pharmaceutical ingredient (API)", as used herein, refers to an agent that provides pharmacological activity in the treatment, medication, cure, prevention, diagnosis of a disease. Sometimes, as used herein, such term "active pharmaceutical ingredient (API)" is used synonymously with the term "drug" or "medicine" or "pharmaceutically active agent".

The term "hydrophobic", as used herein in the context of an active pharmaceutical ingredient (API) refers to a quality of such API that renders such API "practically insoluble in water" and, instead "soluble or freely soluble in an organic solvent or a hydrophobic solvent".

The term "insoluble in water" is meant to refer to the solubility of a solute, in particular of an active pharmaceutical ingredient, in water being preferably < 0.01 g/l more preferably being < 0.001 g/l. It is preferred that the definition of insolubility in water is understood in accordance with the corresponding definitions of the US Pharmacopoeia, according to which an API is

- sparingly soluble in water if it has a solubility in the range of from 10-33 mg/ml;
- slightly soluble in water if it has a solubility in the range of from 1-10 mg/ml;
- very slightly soluble in water if it has a solubility in the range of from 0.1-1 mg/ml; and;
- practically insoluble in water if it has a solubility in the range of from <0.1 mg/ml.

Hence, an API that is "insoluble in water" may be "sparingly soluble", "slightly soluble", "very slightly soluble" or "practically insoluble" in water, as defined herein.

The term "soluble or freely soluble in an organic solvent or hydrophobic solvent" is meant to refer to the solubility of a solute, in particular of an active pharmaceutical ingredient in an organic solvent or hydrophobic solvent in ≥ 10 g/l, preferably ≥ 100 g/l. In accordance with preferred embodiments of the present invention, a hydrophobic active pharmaceutical ingredient (API) is practically insoluble in water or insoluble in water, and is soluble or freely soluble in one or several non-aqueous solvents or organic solvents or hydrophobic solvents.

In a preferred embodiment, a suitable non-aqueous solvent according to the present invention is selected from acetone, dimethyl sulfoxide, ethanol, methanol, tetrahydrofuran, 1-propanol, 2-propanol, acetic acid, acetonitrile, anisole, 1-butanol, 2-butanol, butyl acetate, chloroform, cyclohexane, 1,1,-diethoxypropane, 1,1-dimethoxymethane, 1,2-dimethoxyethane, 1,4-dioxane, 2,2-dimethoxypropane, dichloromethane, diethyl ether, di-iso-propyl ether, dimethylformamide, 2-ethoxyethanol, ethyl acetate, ethyl formate, ethylene glycol (1,2-ethanediol), formic acid, heptane, hexane, isobutyl acetate, isopropyl acetate, 2-methoxyethanol, 2-methyl-1-propanol, 3-methyl-1-butanol, 1-methyl-2-pyrrolidone, methyl acetate, methyl t-butyl ether, methyl butyl ketone, methyl cyclohexane, methyl ethyl ketone (MEK), methyl isobutyl ketone, methyl isopropyl ketone, methyl tetrahydrofuran, n-methyl pyrrolidone, 1-pentanol, , pentane, petroleum ether, propyl acetate, pyridine, sulfolane, t-butyl alcohol, 2,2,4-trimethylpentan (i-octane), toluol, trichloroacetic acid, trichloroethylene, trifluroacetic acid, xylol, or any combination thereof. In one embodiment, the suitable non-aqueous solvent is selected from acetone, dimethyl sulfoxide, ethanol, methanol, tetrahydrofuran, 1-propanol, 2-propanol, acetic acid, acetonitrile, anisole, 1-butanol, 2-butanol, butyl acetate, chloroform, cyclohexane, 1,1,-diethoxypropane, 1,1-dimethoxymethane, 1,2-dimethoxyethane, 1,4-dioxane, 2,2-dimethoxypropane, dichloromethane, diethyl ether, di-iso-propyl ether, dimethylformamide, 2-ethoxyethanol, ethyl acetate, ethyl formate, ethylene glycol (1,2-ethanediol), formic acid, heptane, hexane, isobutyl acetate, isopropyl acetate, 2-methoxyethanol, 2-methyl-1-propanol, 3-methyl-1-butanol, 1-methyl-2-pyrrolidone, methyl acetate, methyl t-butyl ether, methyl butyl ketone, methyl cyclohexane, methyl ethyl ketone (MEK), methyl isobutyl ketone, methyl isopropyl ketone, methyl tetrahydrofuran, n-methyl pyrrolidone, 1-pentanol, pentane, petroleum ether, propyl acetate, pyridine, sulfolane, t-butyl alcohol, 2,2,4-trimethylpentan (i-octane), toluol, trichloroacetic acid, trichloroethylene, trifluroacetic acid, xylol, and any combination thereof, preferably acetone, dimethyl sulfoxide, or ethanol, more preferably acetone or dimethyl sulfoxide.

In a preferred embodiment, the non-aqueous solvent is selected from DMSO, acetone, acetic acid, methanol, ethanol, iso-propanol, dimethylformamide, chloroform, and ethyl acetate, preferably acetone, DMSO or ethanol, more preferably acetone.

The term "hydrophilic solvent", as used herein, is meant to refer to a solvent which is or comprises water or an aqueous solution, such that the major portion of such hydrophilic solvent is water and displays all the characteristics typical of water or an aqueous solution, such as water being the solvent for all solutes in such solution, and the capability of such solvent to form a hydration shell.

The term "polymeric stabilizing agent", as used herein, is meant to refer to a polymer that acts as a stabilizer for the nanoparticles in accordance with the present invention. In preferred embodiments of the present invention said polymeric stabilizing agent is a polymer that has ionizable groups that are ionizable by the addition of a suitable acid or suitable base, and/or having ionic groups. In one particularly preferred embodiment, said polymeric stabilizing agent is selected from polycationic polymers and polyanionic polymers, preferably of a polymethacrylate-based-copolymer-type. In one embodiment, said polymeric stabilizing agent having ionizable or ionic groups, is a poly(methacrylate), preferably selected from neutral protonizable poly(methacrylates) and cationic poly(methacrylates), and anionic poly(methacrylates). In a particularly preferred embodiment, said poly(methacrylate) is selected from copolymers of dialkylaminoethyl methacrylates and methacrylic acid ester(s), and copolymers of trialkylamonioethylmethacrylates and methacrylic acid ester(s), in particular copolymers of dimethylaminoethylmethacrylates and methacrylic acid ester(s) and copolymers of trimethylamonioethylmethacrylates and methacrylic acid esters. In a particularly preferred embodiment, said poly(methacrylate) is a cationic copolymer based on dimethylaminoethylmethacrylate, butylmethacrylate, and methylmethacrylate with a ratio of approximately 2:1:1. Commercially, such cationic copolymer is available as Eudragit E100. (It is sometimes herein also referred to as poly(butyl methacrylate-co-(2-dimethylaminoethyl) methacrylate-co-methyl methacrylate).

In one embodiment, said polymeric stabilizing agent is selected from poly(butyl methacrylate-co-(2-dimethylaminoethyl) methacrylate-co-methyl methacrylate), methyl methacrylate and diethylaminoethyl methacrylate copolymer, ammonio methacrylate copolymer type B, and ammonio methacrylate copolymer type A. The latter two copolymers are commercially available as Eudragit RS and Eudragit RL, respectively.

A methyl methacrylate and diethylaminoethyl methacrylate copolymer is commercially available under the designation Kollicoat® Smartseal 100P.

In another preferred embodiment, said polymeric stabilizing agent, preferably said poly(methacrylate) is a methacrylic acid-based anionic copolymer, based on methacrylic acid and methyl methacrylate, preferably with a ratio of free carboxyl groups to ester groups being in a range of from 1:1 to 1:5, more preferably in a range of from 1:1 to 1:2, even more preferably being either approximately 1:1 or approximately 1:2. In the former case, such poly(methacrylate) is commercially available as Eudragit L100, whereas in the latter case, such poly(methacrylate) is commercially available as Eudragit S100.

The term "nanoparticle", as used herein, relates to a particle of an active pharmaceutical ingredient (API), the average dimension(s) of which is (are) in the nanometer range, in particular such that any longest extension of such nanoparticle is < 1 μm. In a preferred embodiment, a nanoparticle in accordance with the present invention has a defined size that is in the range of from 10 nm to 500 nm, preferably from 20 nm to 350 nm, more preferably from 20 nm to 250 nm, even more preferably from 50 nm to 200 nm.

Sometimes herein reference is made to a "first amount", a "second amount", and/or a "third amount", for example in the context of the method of preparing a dispersion of nanoparticles according to the present invention. More specifically, in such method, there is a first composition comprising a hydrophobic active pharmaceutical ingredient (API) and a polymeric stabilizing agent both of which are dissolved in a "first amount" of a suitable non-aqueous solvent. Moreover, such first composition comprises a "second amount" of a suitable hydrophilic solvent. Moreover, there may also be a "third amount" of suitable acid or base contained within said "second amount". In all these occurrences of different amounts, the usage of the ordinal adjectives "first", "second", and "third" simply serves to distinguish the different amounts (of solvent(s) or acid(s) or base(s)) from each other, and any such (first, second or third) amount(s) can also simply be referred to as "amount(s)" (or "volume(s)"), without specifying them to be "first", "second" or "third". In particular, usage of the ordinal adjectives "first", "second", and "third" does not imply any order or quality attributed to the different amounts thus referred to, other than that these amounts are not the same entities and can be distinguished from each other either by their size/volume, their content or both.

The present inventors have surprisingly found that advantageously, the polymeric stabilizing agent having ionic groups or groups that are ionizable, may be provided in a single first space in a first composition as part of such first composition together with a hydrophobic active pharmaceutical ingredient (API) that is dissolved in first amount of a suitable non-aqueous solvent, wherein, however, the polymeric stabilizing agent forming part of such first composition is provided therein as being dissolved also within said suitable non-aquoeus solvent. The non-aqueous solvent dissolving both the hydrophobic active pharmaceutical ingredient and the polymeric stabilizing agent, is provided in a first amount within said first composition, and the hydrophilic solvent is provided in a second amount within the first composition, and the volume ratio of such first amount to said second amount is typically in the range of from 20:1 to 2:1, preferably in a volume ratio in the range of from 10:1 to 4:1. In other words, the non-aquoeus character of said first composition prevails, and the non-aqueous solvent is always provided therein as the major part thereof, such that the overall character of such first composition is always non-aqueous and thus results in said first composition comprising the hydrophobic active pharmaceutical ingredient in dissolved form. It should be noted, however, that in some other embodiments, the polymeric stabilizing agent may also be provided within said first composition as part of, and being dissolved within, said hydrophilic solvent, i.e. within said second amount, instead of being provided as part of, and being dissolved within, said non-aqueous solvent, i.e. within said first amount. In yet other embodiments, the polymeric stabilizing agent may be provided within said first composition as part of, and being dissolved within, both said hydrophilic solvent, i.e. within said second amount, and as part of, and being dissolved within, said non-aqueous solvent, i.e. within said first amount.

It should also be noted that in some embodiments, said first composition may additionally comprise further solutes, such as stabilizing agents and/or surfactants. Likewise, in some embodiments, said second composition may additionally comprise further solutes, such as stabilizing agents and/or surfactants.

The present inventors have surprisingly found that in providing the hydrophobic active pharmaceutical ingredient together with said polymeric stabilizing agent in a single (first) composition, before the generation of nanoparticles, an aggregation of such nanoparticles later formed may be prevented. Without wishing to be bound by any theory, the present inventors believe that the advantage of providing the polymeric stabilizing agent together with the hydrophobic active pharmaceutical ingredient in said first composition allows for the formation of suitable interfaces between the hydrophobic active pharmaceutical ingredient and the polymeric stabilizing agent as well as the hydrophobic solvent, such that later on in step c), when said first composition is mixed with a second composition that (only) comprises a suitable hydrophilic solvent (such mixing resulting in the generation of nanoparticles), aqueous dispersions of nanoparticles of the hydrophobic active pharmaceutical ingredient (API) stabilized by the polymeric stabilizing agent may be formed that are stable and non-aggregate. In certain embodiments, the stability of the resultant aqueous dispersion of nanoparticles of API stabilized by said polymeric stabilizing agent may be even increased by the addition of a third amount of a suitable acid or suitable base as part of the first composition. To the best knowledge of the inventors, this has not been described anywhere before.

In yet a further aspect, the present invention relates to a method of producing nanoparticles of a defined size, said nanoparticles comprising an active pharmaceutical ingredient (API) and, optionally, at least one polymer, wherein said pharmaceutically active agent is soluble or freely soluble in an organic solvent and has a solubility in water of < 33mg/ml, preferably < 10 mg/ml, more preferably < 1 mg/ml, wherein, more preferably, said pharmaceutically active agent has a solubility in water of < 0.1 mg/ml, said method comprising:

a) providing, in any order but separate from each other, an organic solvent and one of: water and an aqueous solution;
b) providing, in any order but separate from each other, a pharmaceutically active agent and, optionally, at least one polymer;
c) dissolving, in any order:

- said pharmaceutically active agent in said organic solvent, and
- said at least one polymer, if present, in said water or in said aqueous solution or in said organic solvent;

d) providing a mixing device having a first region, a second region and a third region, said first, second and third regions being arranged in said mixing device and configured so as to allow the flow of one or several streams of liquid from said first to said second to said third region;
e) generating in said first region of said mixing device, in any order but separate from each other, a stream of said organic solvent of a defined first flow rate and a stream of said water or

aqueous solution of a defined second flow rate, and allowing in said second region of said mixing device said streams to run parallel and adjacent to each other and to thus form an interface between said streams, enabling a mixing between said organic solvent and said water or aqueous solution by diffusion across said interface, said mixing resulting in a precipitation of nanoparticles in said second and/or third region of the mixing device, said nanoparticles comprising said pharmaceutically active agent and, optionally, said at least one polymer.

In one embodiment, in step e), said streams are allowed to run in laminar flow parallel and adjacent to each other.

In one embodiment of said method,

- the order of steps a) and b) is ab or ba, or steps a) and b) are concomitant or overlapping with each other; with the proviso that both steps a) and b) are performed before step c); and
- step d) is performed any time with respect to any of steps a), b) and c), i. e. step d) is performed before, during or after any of steps a), b) and c); with the proviso that step d) is performed before step e).

In one embodiment, during step e) said stream of organic solvent and said stream of water or aqueous solution generated in said first region, are subsequently split into two, three, four, five, six, seven, eight, nine, ten or more substreams each, preferably a plurality of $2^n$ substreams, where n = an integer from 4 to 20, and said two, three, four, five, six, seven, eight, nine, ten or more substreams of said organic solvent, preferably said plurality of $2^n$ substreams of said organic solvent, are allowed, in said second region, to run parallel and adjacent to said each other, respectively, thus forming a plurality of interfaces between said substreams, enabling a mixing between said organic solvent and said water or aqueous solution by diffusion across said plurality of interfaces; wherein, preferably, said splitting into substreams is achieved by said mixing device being a split- and-recombine-mixer.

In one embodiment, said mixing device is a split- and-recombine-mixer.

In one embodiment, in said mixing device, at least said first region and said second region, preferably also said third region, are made of a material that is inert against said organic solvent; wherein, preferably, said inert material is a metal, more preferably, selected from stainless steel, nickel, aluminum, titanium, molybdenum, chromium, and alloys of any of the foregoing; wherein, even more preferably, said inert material is selected from stainless steel, aluminum, nickel, nickel alloys, in particular alloys of nickel and molybdenum, and alloys of nickel, molybdenum and chromium.

In one embodiment, said active pharmaceutical ingredient (API)is selected from macrolides, non-steroidal anti-inflammatory drugs, hormones, antibodies, steroids, anti-cancer agents, opioid drugs, and mixtures thereof, wherein, preferably, said pharmaceutically active agent is selected from tacrolimus, sirolimus, everolimus, pimecrolimus, erythromycin, clarithromycin, azithromycin, roxithromycin, josamycin, spiramycin, telithromycin, tylosin, fidaxomicin, nystatin, natamycin, amphotericin B; in particular, acetylsalicylic acid, ibuprofen, dexibuprofen, flurbiprofen, naproxen, ketoprofen, tiaprofenic acid, diclofenac, indomethacin, acemetacin, flufenamic acid, mefenamic acid, oxicams, e.g. piroxicam, tenoxicam, meloxicam, lornoxicam, nabumeton, rofecoxib, parecoxib, etoricoxib, celecoxib, alpha-atrial natriuretic peptide, arginine vasopressin, atropine, augrnerosen, atorvastatin, bevacizumab, calcitonins, chorionic gonadotropins, corticotropin, desmopressin, epibatidine, cetuxirnab, exenatide, trastuzumab, adalimumab, human insulin, ketoconazole, lanreotide, lutropin alpha, metoprolol, minoxidil, nesiritide, octreotide, paclitaxel, paracetamol, pegaptanib, recombinant follicle stimulating hormone, recombinant growth factors, infliximab, rituximab, sermorelin, somatotropin, a taxane derivative, taxol, teriparatide acetate, thyrotropin, triclosan, urofollitropin, omalizumab, actinomycin D, albendazole, aldosterone, alprazolam, amiodarone, amitriptyline, amprenavir, asimadoline, atorvastatin, bunitrolol, buspirone, camptothecin, carbamazepine, carvedilol, celiprolol, cyclosporine A, cimetidine, clotrimazole, colchicine, cortisone, daunorubicin, debrisoquine, dexamethasone, diazepam, digitoxin, digoxin, diltiazem, docetaxel, domperidone, doxorubicin, efavirenz, epirubicin, erythromycin, ergotamine, estradiol, estradiol glucuronide, erlotinib, etoposide, phenytoin, fentanyl, felodipine, phenothiazines, fexofenadine, fluoroquinolones, fluorouracil, gentamicin, griseofulvin, hydrocortisone, imatinib, indinavir, itraconazole, ivermectin, ketoconazole, kaempferol, levofloxacin, lidocaine, loperamide, losartan, lovastatin, mebendazole, methylprednisolone, methotrexate, mibefradil, midazolam, nisoldipine, morphine, nelfinavir, nicardipine, nitrendipine, nifedipine, ondansetron, paclitaxel, pentazocine, praziquantel, prednisolone, prednisone, quercetin, quinidine, ranitidine, rifabutin, rifampicin, ritonavir, saquinavir, sulfame-thizole, tamoxifen, talinolol, teniposide, terfenadine, tetracycline, topotecan, triamcinolone, valspodar, verapamil, vinblastine, vincristine, vindesine, zopiclone, and mixtures thereof.

In a preferred embodiment, said active pharmaceutical ingredient (API) is selected from a wide range of known drug classes, including, but not limited to, painkillers, anti-inflammatory substances, anthelmintics, antiarrhythmic substances, antibiotics (including penicillins), anticoagulants, antidepressants, antidiabetic substances, anti-epileptics, antihistamines, antihypertensive substances, antimuscarinic substances, antimycobacterial substances, antineoplastic substances, immunosuppressants, antithyroid substances, antiviral substances, anoxiolytic sedatives (hypnotics and neuroleptics), astringents, beta-adrenoreceptor antagonists, blood products and blood substitute products, inotropic substances for the heart, contrast agents, corticosteroids, substances to suppress coughing, (expectorants and mucolytics), diagnostic substances, diagnostic imaging substances, diuretics, dopaminergic substances (substances to combat Parkinson's disease), hemostatics, immunological substances, substances to regulate fat, muscle relaxants, parasympathomimetics, parathyroidal calcitonin and biphosphonates, prostaglandins, radiopharmaceuticals, sex hormones (including steroids), antiallergics, stimulants and anorectics, sympathomimetics, thyroidal substances, vasodilators and xanthines.

In one embodiment, said organic solvent is selected from ethanol, acetone, methanol, tetrahydrofuran, 2-isopropanol, acetic acid, acetonitrile, anisole, 1-butanol, 2-butanol, butyl acetate, chloroform, cyclohexane, 1,1,-diethoxypropane, 1,1-dimethoxymethane, 1,2-dimethoxyethane, 1,4-dioxane, 2,2-dimethoxypropane, dichloromethane, diethyl ether, di-iso-propyl ether, dimethyl sulfoxide, dimethylformamide, 2-ethoxyethanol, ethyl acetate, ethyl formate, ethylene glycol (1,2-ethanediol), formic acid, heptane, hexane, isobutyl acetate, isopropyl acetate, 2-methoxyethanol, 2-methyl-1-propanol, 3-methyl-1-butanol, 1-methyl-2-pyrrolidone, methyl acetate, methyl t-butyl ether, methyl butyl ketone, methyl cyclohexane, methyl ethyl ketone (MEK), methyl isobutyl ketone, methyl isopropyl ketone, methyl tetrahydrofuran, n-methyl pyrrolidone, 1-pentanol, 1-propanol, 2-propanol, pentane, petroleum ether, propyl acetate, pyridine, sulfolane, t-butyl alcohol, 2,2,4-trimethylpentan (i-octane), toluene, trichloroacetic acid, trichloroethylene, trifluoracetic acid, xylol and mixtures thereof; wherein preferably said organic solvent is selected from acetone, ethanol,methanol, tetrahydrofuran, and 2-isopropanol.

In one embodiment, in step b) in addition to the to the pharmaceutically active agent, also at least one polymer is provided, wherein preferably, said polymer is an amphiphilic polymer.

In the embodiment wherein in step b) also at least one polymer is provided, said polymer, in particular said amphiphilic polymer, is selected from polyvinyl caprolactam-polyvinyl acetate-polyethylene glycol graft copolymers; castor oils, in particular non-hydrogenated castor oils, hydrogenated castor oils, pegylated castor oils, and polyoxyl castor oils; poly(methacrylates) and D-α-tocopherol-polyethylenglycol-succinate (TPGS), and wherein, preferably, the poly(methacrylates) are selected from neutral protonizable poly(methacrylates), cationic poly(methacrylates) and anionic poly(methacrylates), more preferably selected from copolymers of dialkylaminoethyl methacrylates and methacrylic acid ester(s), and copolymers of trialkylammonioethyl methacrylates and methacrylic acid ester(s), in particular copolymers of dimethylaminoethyl methacrylates and methacrylic acid esters, and copolymers of trimethylammonioethyl methacrylates and methacrylic acid esters.

In one embodiment, in said mixing device, a ratio of said defined second flow rate to said defined first flow rate is in the range of from 1:1 to 100.

In one embodiment, the nanoparticles produced by the method according to this aspect have an average size in the range of from 5 nm to 700 nm, preferably from 10 nm to 700 nm, more preferably from 20 nm to 500 nm, even more more preferably, 20 nm to 100 nm; said average size of said nanoparticles being preferably measured or determined by dynamic light scattering as described herein. (.

In one embodiment, the nanoparticles produced have a polydispersity index <1, preferably < 0.4, more preferably <0.2, even more preferably <0.15, even more preferably <0.1.

In one embodiment, the total flow rate, i. e. the sum of said defined first and second flow rates, is in a range of from 1 ml/min to 300 ml/min, preferably from 1 ml/min to 100 ml/min, more preferanly from 1 ml/min to 50 ml/min, even more preferably from 1 ml/min to 30 ml/min..

In embodiments which are useful for commercial production, the total flow rate is in a range of from 1 ml/min to 300 ml/min. In embodiments useful for research and development, the total flow rate is in a range of from 1 ml/min to 50 ml/min.

In a further aspect, the present invention also relates to a nanoparticle or a plurality of nanoparticles, produced by the method for producing nanoparticles of a defined size, as defined further above, wherein preferably said plurality of nanoparticles has a polydispersity index (PDI) of ≤ 0.4, preferably ≤ 0.2, more preferably ≤ 0.1.

In yet another aspect, the present invention also relates to a nanoparticle or a plurality of nanoparticles, as defined and produced by the method for producing nanoparticles of a defined size, as defined further above, for use in a method of treatment of a disease. Exemplary diseases are as described herein.

Furthermore, the present invention is described by the following figures which are given to illustrate the invention, not to limit it.

In these figures,

Figure 1 shows Zeta potential of dispersion NUC05_036 after dilution of 1:10 with water;

Figure 2 shows prepared dispersions of samples NUC05_b001-b068 ("RAP" = rapamycin);

Figure 3 shows images of the diluted samples (dilution factor 100, medium demineralized water) from DLS measurement ("RAP" = rapamycin);

Figure 4 shows light microscopic images of samples NUC05_b073, b081 and b089 (undiluted);

Figure 5 shows light microscopic images of samples NUC05_b074, b082 and b090 (undiluted);

Figure 6 shows various samples prepared via a batch.stirring process using different acids, as summarized in tables 6 and 7;

Figure 7 shows the data from table 10 as resulting pH-value vs. degree of protonation ("protonation grade"). The dispersions were produced in accordance with table 9;

Figure 8 shows the data from table 10 as particle size vs. degree of protonation ("protonation grade"). The dispersions were produced in accordance with table 9;

Figure 9 shows the data from table 10 as particle size vs. pH. The dispersions were produced in accordance with table 9;

Figure 10 shows the Zeta potential of Eudragit E100 (triangle, open), chitosan (circle) and NUc05_b143 (triangle, filled) as a function of mucin ratio using a pH 4 buffer;

Figure 11 shows Zeta potential of Eudragit E100 (triangle, open), chitosan (circle) and NUc05_b143 (triangle, filled) as a function of mucin ratio using a pH 7 buffer;

Figure 12 shows time-dependent hydrodynamic diameter (Z-average) of samples $NUC0_3\_043$ ("NUC03_043_t0/t1/t2/t3/t4") and trial 17 ("Versuch17_t0/1/2/3/4");

Figure 13 shows the release of two dispersions: Sirolimus solubilized in E100 ("Trial 17") and Sirolimus solubilized in Soluplus (control, "NUC03_043") with logarithmic time axis. Shown is the mean value from each of 3 independent experiments and the standard deviation of the mean values;

Figure 14 shows (A) 2nd derivative absorbance spectra of Sirolimus in ASB at various concentrations. (B) resulting standard curve used for quantification of Sirolimus in receiver chamber in flux measurements;

Figure 15 shows averaged acceptor chamber profiles of Formulations 1a, 1b, 1c and the control sample over 120 minutes;

Figure 16 shows correlation of protonation grade (A) ("Protonierungsgrad") and particle size (B) with flux of prototype 1a, 1b and 1c. Flux of the control sample was 0.036 $\mu$g/min/cm$^2$ and is indicated as red line;

Figure 17 shows an optical image of the prepared samples (dilution factor 100, medium water) as a function of time;

Figure 18 shows electron micrographs of sample NUC05_024 (100% protonation);

Figure 19 shows electron micrographs of sample NUC05_025 (60% protonation);

Figure 20 shows electron micrographs of sample NUC05_026 (25% protonation). Average diameter of about 70 nm;

Figure 21 shows diffractograms of samples nucRAP001 nucRAP004 in comparison to XRPD of Rapamacyin calculated from single crystal data. The comparison reveals that the signals of the reference of the API could be observed in sample nucRAP001 and nucRAP002. The samples nucRAP003, nucRAP004 and nucRAP005 show an amorphous halo with only one signal at 25.48° 2Theta;

Figure 22 shows an image of different formulation compositions (Ibuprofen/Eudragit E 1/1.5; degree of protonation vs. FRR);

Figure 23 shows an image of the different formulation compositions (Eudragit E/Ibuprofen Ratio vs. FRR);

Figure 24 shows another image of the different formulation compositions (Eudragit E/Ibuprofen Ratio vs. FRR);

Figure 25 shows an image of the different formulation compositions (Eudragit E/Itraconazole 1.5/1; degree of protonation vs. FRR);

Figure 26 shows an image of the different formulation compositions (Eudragit E/prednisolone 1.5/1; protonation level vs. FRR);

Figure 27 shows the preparation of placebo samples and verum samples of sirolimus for $^1$H-NMR spectroscopy;

Figure 28 shows $^1$H-NMR spectra of samples 1 and 3 (sirolimus ("SIR") dissolved in acetone, and sirolimus ("SIR") + E100 dissolved in acetone;

Figure 29 shows $^1$H-NMR spectra of placebo samples 6-8;

Figure 30 shows $^1$H-NMR spectra of verum samples 9 - 11;

Figure 31 shows a comparison of $^1$H-NMR spectra of placebo sample 8 (Eudragit E100+ acid in $D_2O$/Acetone, 100% protonation) with "verum"-sample 11 (SIR/E100+ acid in $D_2O$/Acetone 100% protonation); and

Figure 32 shows a scheme of the experimental preparation of sirolimus nanoparticles stabilized by Eudragit E100, with two different approaches according to which a surfactant was included either during precipitation (**FLM04_025**) or after precipitation (**FLM02_b230**).

Furthermore, reference is now made to the following examples which are given to illustrate, not to limit the present invention.

**Examples**

**1 Solubility of sirolimus in organic solvents**

One task is the production of a highly concentrated dispersion. The prerequisite for this is the use of a solvent for the production of the dispersion in which the active substance has a high solubility.

For this purpose, the saturation solubility of sirolimus in suitable organic solvents was determined. Crystalline active ingredient was added to a defined amount of solvent at room temperature while stirring until an insoluble sediment was formed. After filtration, the concentration of sirolimus in the supernatant was quantified by HPLC.

*Table 1: Saturation solubility of sirolimus in selected organic solvents*

| Type of solvent | | Solubility / mg/ml | Boiling point/ °C | I Class |
|---|---|---|---|---|
| **Polar protic** | Water | Approx. 0.001 | 100 | |
| | Methanol | 246 | 65 | 2 |
| | Ethanol | 179 | 78 | 3 |
| | iso-Propanol | 25,0 | 82,5 | 3 |
| **Polar aprotic** | Acetone | 236 | 56 | 3 |

Although sirolimus is practically insoluble in water, the active substance was very soluble in polar, protic solvents. However, the active ingredient is also very soluble (≥200 mg/ml) in the polar, aprotic solvent tested.

Acetone was chosen for all further experiments. Non-toxicity (ICH Class 3) and lowest boiling point - and thus the possibility of complete removal by drying at low temperatures - distinguish this solvent.

Using a solution of sirolimus in acetone at 230 mg/ml, a dispersion of more than 20 mg/ml sirolimus (FRR 10) would be possible.

**2 Production of the dispersions**

Two process solutions are prepared for the production of the nanosuspension.

Process solution A (solvent) consists of a mixture of API and the stabiliser (e.g. Eudragit E100), which are first dissolved in an appropriate solvent. The choice of solvent depends on the specific solubility. Organic solvents such as THF, acetone, chlorinated hydrocarbons or water can be used. In the second step, a defined amount of an acid is added. The acid can be diluted in water or added as a pure substance (liquid, solid). Next, a defined amount of water is added to produce the desired water content, preferably 20 % (v/v) relative to the process solution. The process solution A is ready for further production after a standing time of preferably 1-2 hours.

Process solution B (non-solvent) consists of an aqueous solution that may contain additional stabilisers.

The API/Solvent solution (process solution A) is combined with a non-solvent (process solution B) in which the API has little or low solubility and in which the stabiliser has sufficient solubility at acidic, neutral or basic pH. The combining of the two phases can be done by batch stirring processes or continuous mixing processes.

By reacting the API-containing solvent phase A with the non-solvent phase B in the presence of a stabiliser, stable API dispersions can be obtained.

**3 Particle size measurement**

Particle size was determined using dynamic light scattering (DLS) at a scattering angle of 150° using a DynaPro Plate Reader (Wyatt) after dilution of the sample with deionized water (dilution factors see respective chapter). Water was used as non-solvent in the precipitation of the drug product intermediate. Therefore, minimal influence of nanoparticles on dilution is expected.

The autocorrelation function of the scattered light was analysed applying the cumulant algorithm to the autocorrelation function of the measured intensity according to ISO 13321 and ISO 22412. A mono-exponential function is fitted to the autocorrelation function resulting in an intensity-based mean value for the size (z-average) and the dimensionless width parameter PDI (Polydispersity Index) which describes the width of the assumed Gaussian distribution. The following terms are derived from the PDI:

- Polydispersity Index (PDI) = Relative variance = $(StdDev/mean)^2$
- PDI width = StdDev = $(PDI)^{1/2}$ * mean

- %Polydispersity (%PD) = Coefficient of variation = $(PDI)^{1/2} \times 100$

The PDI is scaled such that values smaller than 0.05 are rarely seen other than with highly monodisperse standards. Values greater than 0.7 indicate that the sample has a very broad size distribution and is probably not suitable for the dynamic light scattering technique.

The settings listed below were used for DLS measurements.

| ID | Name | Value |
|---|---|---|
| Sample | Material | Protein |
| | RI (Refractive index) | 1.584 |
| | Absorption | 0.010 |
| | Dispersant | water |
| | Temperature | 25°C |
| | Viscosity | 0.8872 |
| | RI (Refractive index) | 1.330 |
| | Temperature | 25°C |
| | Equilibration time | 60 sec |
| | Cell type | Disposable 96 Wellplate |
| Measurement | Measurement angle | 150° |
| | Measurement duration | 10 runs. 5 seconds each run |
| | Measurements per Well within a scan | 1 |
| | Number of scans: | 1 |
| | Attenuation | Automatic selection |

| ID | Name | Value | |
|---|---|---|---|
| Data processing | Analysis model | Property | Value |
| | | Cutoff | |
| | | Correlation function low cutoff (µs) | 0.80 |
| | | Correlation function high cutoff (µs) | 1.00e+06 |
| | | Peak Radius Cutoffs | |
| | | Peak Radius Low Cutoff (nm) | 0.50 |
| | | Peak Radius High Cutoff (nm) | 10000.00 |
| | | Cumulants Type | Wyatt (recommended) |
| | | Regularization Type | DYNALS (recommended) |
| | | Measurement Time Limit Factor | 5.000 |
| | | Calculate D10/D50/D90 | No |
| | | Calculate Polydispersity | Yes |
| | | Collection Type | Experiment Designer |
| | | Minimum Peak Intensity (%) | 1.000 |

*Remark:* The cumulant fitting method always provides one single size value for every sample but is less vulnerable to noise than other algorithms. However, it is unsuitable for heterogeneous polydisperse samples with PDI>0.4.

**4 Zeta potential of dispersion**

The interface of the particles of a colloidal dispersion has a significant influence on its stability. If the particles repel each other through their surface properties, the substance system remains stable against coagulation and subsequent phase separation. If the particles are large enough and the density difference to the medium is sufficient, sedimentation or flotation can occur.

Electrostatic charged and steric shells prevent the particles from coming so close to each other that the short-range van der Waals attraction could lead to flocculation or coalescence.

*Steric stabilisation* occurs when macromolecules are attached to the particle surface by adsorption or covalent bonds. If the solvent is good in relation to the macromolecules of the shell, the shells of the particles cannot push significantly into each other and the particles remain far enough apart for the dispersion to be stable.

For *electrostatic stabilisation,* charges must be present on the surface of the particles. The surface charges are compensated by the counterions. The counterions form a diffuse ion layer around the particles. The repulsion between the diffuse ion layers stabilises the dispersion. A zeta potential greater than $\pm 30$ mV proves efficient electrostatic stabilisation.

*Carrying out the measurement*

The zeta potential of the E100-based dispersion NUC05_036 was determined by electrophoretic light scattering at a scattering angle of 173 ° using a Zetasizer Nano ZS (Malvern) after dilution of the sample with water (10 $\mu$l dispersion plus 1 ml water).

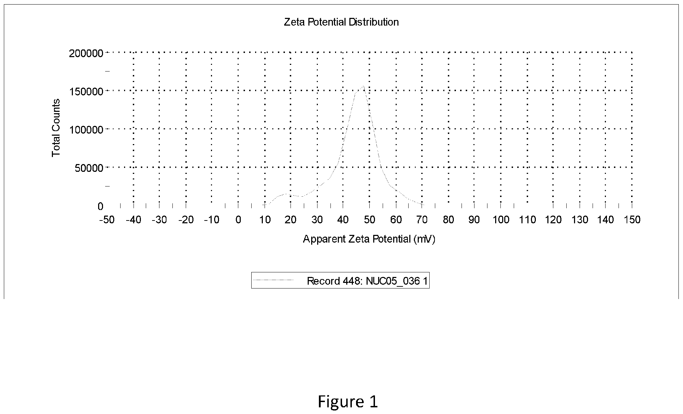

The measurement resulted in a potential of 44 mV. An exemplary measurement is shown in figure 1

*Conclusion*

The result suggests that the solubilisates based on the charged polymer methacrylate are stabilised primarily by electrostatic forces rather than steric interactions.

**5 Preparation of the formulation via batch - stirring process** *Production of the process solutions*

Process solution A (solvent): A solution consisting of 167 mg/mL sirolimus and 250 mg/mL Eudragit E100 in acetone is prepared. To 8 mL of this solution, add 2 mL of acid solution (concentration of acid see Table 2) and let the mixture stand for 2 hours. The process solution can then be used.

Process solution B (non-solvent): Demineralised water was used as process solution B.

*Processing in the batch stirring process*

To produce a solvent/non-solvent ratio of 1/4, 1 mL of process solution A is added to 4 mL of process solution B with rapid stirring (750 rpm). The solution is stirred for another 30 minutes and is then ready for characterisation.

*Characterisation - Particle size*

For characterisation of the prepared samples with regard to particle size, the hydrodynamic diameter was determined by means of dynamic light scattering (DLS) on a Wyatt Plate Reader III. The degree of dilution was chosen depending on the solid concentration and was between 1:10 and 1:500 in water and/or pH buffers. The homogeneity of the prepared samples was examined optically and by light microscopy. Here, the samples were qualitatively classified into the corresponding categories.

**5.1 Variation of formulation parameters (Part 1): Solids content, degree of protonation, amount of water, API/ stabiliser ratios, FRR**

The variation of the formulation parameters was carried out with the active ingredient sirolimus. The formulation parameters solids content, degree of protonation, amount of water, API/stabiliser ratios, FRR were investigated.

The total solids content has no significant influence on the particle size.

The amount of water has a significant effect on particle size at a FRR of 4. As the amount of water in the solvent solution increases, the total amount of non-solvent (water) in the final formulation increases, resulting in an increase in particle size.

The degree of protonation of the stabilising polymer (e.g. Eudragit E100) plays the central role in adjusting the particle size. As the degree of protonation increases, the particle size increases.

The stabiliser/API ratio also influences particle size and pH. The higher the stabiliser/API ratio at constant total substance content, the larger the particle size.

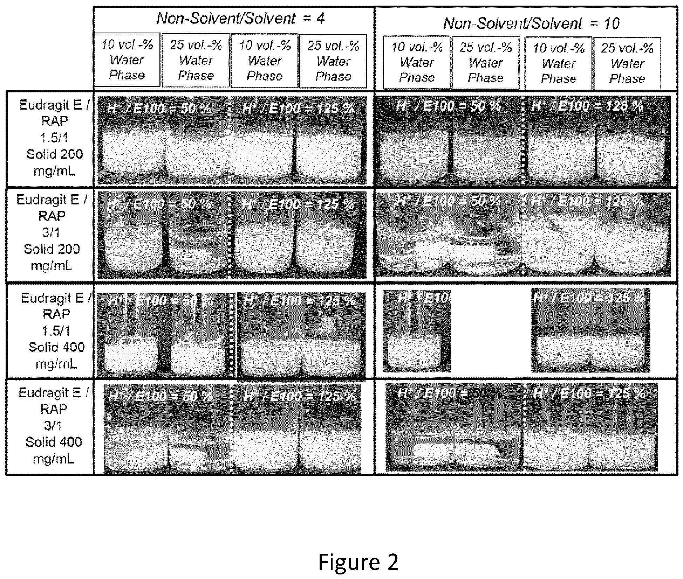

Results are shown in figure 2 and table 3.

*Table 2: Manufacturing conditions of the samples prepared via batch stirring process, 1st part.*

| Formulation code | Protonation | [SIR] | [E100] | Water Phase | FRR |
|---|---|---|---|---|---|
| NUC05_ | % | mg/ml | mg/ml | v/v % | |
| b001 | 50 | 80 | 120 | 10 | 4 |

(continued)

| Formulation code | Protonation | [SIR] | [E100] | Water Phase | FRR |
|---|---|---|---|---|---|
| NUC05_ | % | mg/ml | mg/ml | v/v % | |
| b002 | 50 | 80 | 120 | 25 | 4 |
| b003 | 125 | 80 | 120 | 10 | 4 |
| b004 | 125 | 80 | 120 | 25 | 4 |
| b009 | 50 | 80 | 120 | 10 | 10 |
| b010 | 50 | 80 | 120 | 25 | 10 |
| b011 | 125 | 80 | 120 | 10 | 10 |
| b012 | 125 | 80 | 120 | 25 | 10 |
| b021 | 50 | 50 | 150 | 10 | 4 |
| b022 | 50 | 50 | 150 | 25 | 4 |
| b023 | 125 | 50 | 150 | 10 | 4 |
| b024 | 125 | 50 | 150 | 25 | 4 |
| b028 | 50 | 50 | 150 | 10 | 10 |
| b029 | 50 | 50 | 150 | 25 | 10 |
| b030 | 125 | 50 | 150 | 10 | 10 |
| b031 | 125 | 50 | 150 | 25 | 10 |
| b041 | 50 | 100 | 300 | 10 | 4 |
| b042 | 50 | 100 | 300 | 25 | 4 |
| b043 | 125 | 100 | 300 | 10 | 4 |
| b044 | 125 | 100 | 300 | 25 | 4 |
| b049 | 50 | 100 | 300 | 10 | 10 |
| b050 | 50 | 100 | 300 | 25 | 10 |
| b051 | 125 | 100 | 300 | 10 | 10 |
| b052 | 125 | 100 | 300 | 25 | 10 |
| b057 | 50 | 160 | 240 | 10 | 4 |
| b058 | 50 | 160 | 240 | 25 | 4 |
| b059 | 125 | 160 | 240 | 10 | 4 |
| b060 | 125 | 160 | 240 | 25 | 4 |
| b065 | 50 | 160 | 240 | 10 | 10 |
| b066 | 50 | 160 | 240 | 25 | 10 |
| b067 | 125 | 160 | 240 | 10 | 10 |
| b068 | 125 | 160 | 240 | 25 | 10 |

*Table 3: Formulation composition of samples prepared by batch stirring process, 1st part.*

| Formulation code | Protonation | Acetone | [SIR] | [E100] | E100/ SIR | pH | Zeta potential | size | PDI |
|---|---|---|---|---|---|---|---|---|---|
| NUC05_ | % | v/v | mg/ml | mg/ml | | | mV | nm | |
| b001 | 50 | 0.18 | 12.0 | 18.0 | 1.5 | 6.2 | - | 143 | 0.146 |
| b002 | 50 | 0.15 | 12.0 | 18.0 | 1.5 | 6.2 | - | 98 | 0.165 |
| b003 | 125 | 0.18 | 12.0 | 18.0 | 1.5 | 6.0 | 62 | 526 | 0.135 |

(continued)

| Formulation code | Protonation | Acetone | [SIR] | [E100] | E100/ SIR | pH | Zeta potential | size | PDI |
|---|---|---|---|---|---|---|---|---|---|
| NUC05_ | % | v/v | mg/ml | mg/ml | | | mV | nm | |
| b004 | 125 | 0.15 | 12.0 | 18.0 | 1.5 | 6.6 | 60 | 301 | 0.114 |
| b009 | 50 | 0.082 | 5.5 | 8.2 | 1.5 | 6.0 | 36 | 119 | 0.154 |
| b010 | 50 | 0.068 | 5.5 | 8.2 | 1.5 | 5.9 | 38 | 91 | 0.158 |
| b011 | 125 | 0.082 | 5.5 | 8.2 | 1.5 | 6.3 | 59 | 246 | 0.144 |
| b012 | 125 | 0.068 | 5.5 | 8.2 | 1.5 | 5.0 | 57 | 240 | 0.036 |
| b021 | 50 | 0.18 | 7.5 | 22.5 | 3 | 6.2 | 58 | 207 | 0.180 |
| b022 | 50 | 0.15 | 7.5 | 22.5 | 3 | 7.0 | 55 | 49 | 0.501 |
| b023 | 125 | 0.18 | 7.5 | 22.5 | 3 | 5.0 | 65 | 740 | 0.213 |
| b024 | 125 | 0.15 | 7.5 | 22.5 | 3 | 5.1 | 64 | 370 | 0.107 |
| b028 | 50 | 0.082 | 3.4 | 10.2 | 3 | 5.9 | - | 63 | (*) |
| b029 | 50 | 0.068 | 3.4 | 10.2 | 3 | 5.9 | 72 | 33 | 0.447 |
| b030 | 125 | 0.082 | 3.4 | 10.2 | 3 | 4.8 | 38 | 174 | 0.246 |
| b031 | 125 | 0.068 | 3.4 | 10.2 | 3 | 5.0 | - | - | (*) |
| b041 | 50 | 0.18 | 15.0 | 45.0 | 1.5 | 6.3 | 44 | 96 | 0.391 |
| b042 | 50 | 0.15 | 15.0 | 45.0 | 1.5 | 6.3 | 41 | 51 | (*) |
| b043 | 125 | 0.18 | 15.0 | 45.0 | 1.5 | 4.9 | 50 | 477 | 0.027 |
| b044 | 125 | 0.15 | 15.0 | 45.0 | 1.5 | 4.9 | 50 | 250 | 0.111 |
| b049 | 50 | 0.082 | 6.8 | 20.5 | 1.5 | 5.9 | 55 | 78 | (*) |
| b050 | 50 | 0.068 | 6.8 | 20.5 | 1.5 | 6.0 | 38 | 59 | 0.534 |
| b051 | 125 | 0.082 | 6.8 | 20.5 | 1.5 | 4.8 | 52 | 231 | 0.137 |
| b052 | 125 | 0.068 | 6.8 | 20.5 | 1.5 | 5.1 | 53 | 217 | 0.004 |
| b057 | 50 | 0.18 | 24.0 | 36.0 | 3 | 6.0 | 52 | 169 | 0.208 |
| b058 | 50 | 0.15 | 24.0 | 36.0 | 3 | 6.5 | 54 | 150 | 0.544 |
| b059 | 125 | 0.18 | 24.0 | 36.0 | 3 | 4.9 | 66 | 409 | 0.080 |
| b060 | 125 | 0.15 | 24.0 | 36.0 | 3 | 5.1 | 62 | 279 | 0.103 |
| b065 | 50 | 0.082 | 10.9 | 16.4 | 3 | 6.3 | - | 189 | 0.094 |
| b066 | 50 | 0.068 | 10.9 | 16.4 | 3 | - | - | - | - |
| b067 | 125 | 0.082 | 10.9 | 16.4 | 3 | 4.8 | 65 | 228 | 0.129 |
| b068 | 125 | 0.068 | 10.9 | 16.4 | 3 | 5.0 | 62 | 246 | 0.139 |
| (*) multimodal distribution | | | | | | | | | |

### 5.2 Variation of formulation parameters (part 2): API/stabiliser ratios, degree of protonation, FRR

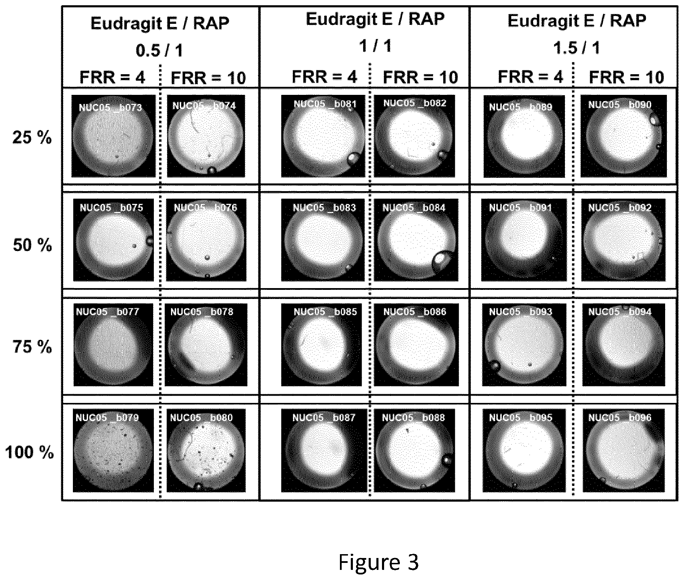

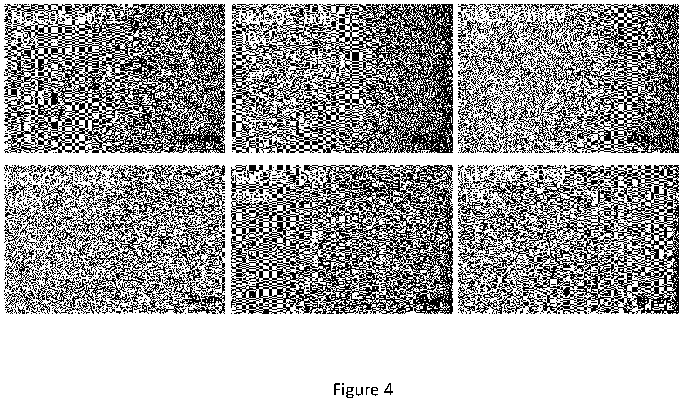

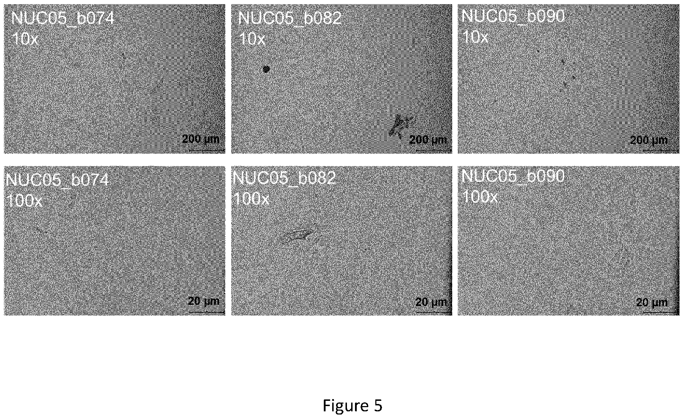

Here the API concentration was kept constant and the amount of stabiliser was varied, resulting in different stabiliser/sirolimus ratios. The experiments were carried out by varying the flow rate ratio (FRR) and the degree of protonation. Acetic acid was used as the acid. The amount of water in the prehydrolysis solution was 20 % (V/V). Distilled water was used as dilution medium for the determination of the hydrodynamic diameter. The production parameters and corresponding results are shown in the tables (see Table 4 and Table 5) and figures 3-5.

*Table 4: Manufacturing conditions of the samples prepared via batch stirring process, 2nd part.*

| Formulation code | Protonation | [SIR] | [E100] | FRR |
|---|---|---|---|---|
| NUC05_ | % | mg/ml | mg/ml | |
| b073 | 25 | 167 | 83 | 4 |
| b074 | 25 | 167 | 83 | 10 |
| b075 | 50 | 167 | 83 | 4 |
| b076 | 50 | 167 | 83 | 10 |
| b077 | 75 | 167 | 83 | 4 |
| b078 | 75 | 167 | 83 | 10 |
| b079 | 100 | 167 | 83 | 4 |
| b080 | 100 | 167 | 83 | 10 |
| b081 | 25 | 167 | 167 | 4 |
| b082 | 25 | 167 | 167 | 10 |
| b083 | 50 | 167 | 167 | 4 |
| b084 | 50 | 167 | 167 | 10 |
| b085 | 75 | 167 | 167 | 4 |
| b086 | 75 | 167 | 167 | 10 |
| b087 | 100 | 167 | 167 | 4 |
| b088 | 100 | 167 | 167 | 10 |
| b089 | 25 | 167 | 250 | 4 |
| b090 | 25 | 167 | 250 | 10 |
| b091 | 50 | 167 | 250 | 4 |
| b092 | 50 | 167 | 250 | 10 |
| b093 | 75 | 167 | 250 | 4 |
| b094 | 75 | 167 | 250 | 10 |
| b095 | 100 | 167 | 250 | 4 |
| b096 | 100 | 167 | 250 | 10 |

*Table 5. Formulation composition of samples prepared via batch stirring process, 2nd part.*

| Formulation code | Protonation | Acetone | [SIR] | [E100] | E100/ SIR | Optical homogeneity | pH | Zeta potential | size | PDI |
|---|---|---|---|---|---|---|---|---|---|---|
| NUC05_ | % | v/v | mg/ml | mg/ml | | | | mV | nm | |
| b073 | 25 | 0.16 | 26.7 | 13.3 | 0.5 | low | 6.0 | 58 | 195 | 0.178 |
| b074 | 25 | 0.073 | 12.1 | 6.0 | 0.5 | low | 5.7 | 62 | 181 | 0.234 |
| b075 | 50 | 0.16 | 26.7 | 13.3 | 0.5 | medium | 6.0 | 59 | 196 | 0.116 |
| b076 | 50 | 0.073 | 12.1 | 6.0 | 0.5 | medium | 5.7 | 61 | 188 | 0.125 |
| b077 | 75 | 0.16 | 26.7 | 13.3 | 0.5 | low | 5.8 | 61 | 289 | 0.158 |
| b078 | 75 | 0.073 | 12.1 | 6.0 | 0.5 | low | 5.5 | 79 | 254 | 0.219 |
| b079 | 100 | 0.16 | 26.7 | 13.3 | 0.5 | low | 5.4 | 51 | 337 | 0.153 |
| b080 | 100 | 0.073 | 12.1 | 6.0 | 0.5 | low | 5.2 | 67 | 267 | 0.186 |
| b081 | 25 | 0.16 | 26.7 | 26.7 | 1.0 | medium | 6.3 | 29 | 154 | 0.323 |

(continued)

| Formulation code | Protonation | Acetone | [SIR] | [E100] | E100/SIR | Optical homogeneity | pH | Zeta potential | size | PDI |
|---|---|---|---|---|---|---|---|---|---|---|
| NUC05_ | % | v/v | mg/ml | mg/ml | | | | mV | nm | |
| b082 | 25 | 0.073 | 12.1 | 12.1 | 1.0 | medium | 6.0 | 59 | 143 | 0.343 |
| b083 | 50 | 0.16 | 26.7 | 26.7 | 1.0 | high | 6.2 | 49 | 193 | 0.095 |
| b084 | 50 | 0.073 | 12.1 | 12.1 | 1.0 | high | 5.9 | 60 | 156 | 0.171 |
| b085 | 75 | 0.16 | 26.7 | 26.7 | 1.0 | high | 6.0 | 50 | 184 | 0.104 |
| b086 | 75 | 0.073 | 12.1 | 12.1 | 1.0 | high | 5.7 | 66 | 194 | 0.082 |
| b087 | 100 | 0.16 | 26.7 | 26.7 | 1.0 | high | 5.3 | 54 | 252 | 0.072 |
| b088 | 100 | 0.073 | 12.1 | 12.1 | 1.0 | high | 5.5 | 59 | 180 | 0.177 |
| b089 | 25 | 0.16 | 26.7 | 40.0 | 1.5 | medium | 6.4 | - | 136 | 0.432 |
| b090 | 25 | 0.073 | 12.1 | 18.2 | 1.5 | medium | 6.1 | - | 143 | (*) |
| b091 | 50 | 0.16 | 26.7 | 40.0 | 1.5 | high | 6.3 | 49 | 140 | 0.232 |
| b092 | 50 | 0.073 | 12.1 | 18.2 | 1.5 | high | 6.0 | 48 | 138 | 0.304 |
| b093 | 75 | 0.16 | 26.7 | 40.0 | 1.5 | high | 5.8 | 50 | 250 | 0.097 |
| b094 | 75 | 0.073 | 12.1 | 18.2 | 1.5 | high | 5.8 | 47 | 204 | 0.143 |
| b095 | 100 | 0.16 | 26.7 | 40.0 | 1.5 | high | 5.3 | 51 | 200 | 0.078 |
| b096 | 100 | 0.073 | 12.1 | 18.2 | 1.5 | high | 5.1 | 57 | 250 | 0.156 |
| (*) multimodal distribution | | | | | | | | | | |

An increase in the stabiliser/API ratio at a protonation level of 25% and an FRR of 4 (cf. NUC05_b073, b081, b089) led to a decrease in hydrodynamic particle size and an increase in polydispersity indices. Homogeneity was significantly improved by a decrease in aggregates (light microscopy images) with increasing stabiliser concentration. This was accompanied by an increase in the pH of the final suspension and a decrease in the zeta potential. A similar observation was made for an FRR of 10 under otherwise identical conditions (cf. NUC05_b074, b082, b090), but here a better homogeneity was determined overall.

An increase in the degree of protonation leads to lower pH values of the final suspension and larger hydrodynamic radii, especially with an Eudragit E/SIR ratio of > =1. This observation is independent of the FRR used. Increasing protonation also leads to increasing homogeneity.

**5.3 Variation of the formulation parameters: Acid**

Different acids were investigated for the pre-protonation of the API solvent mixture. Watersoluble acids were added in the water phase, water-insoluble acids were weighed directly and added to the solvent - solvent phase.

With the alkanoic acids, particles could be successfully produced up to the C5 acid (valeric acid). Different particle sizes resulted depending on the acids used. The use of different acids and the resulting counterions during protonation result in different complex properties and thus different product properties.

The use of non-polar acids (>C8 acids) resulted in inhomogeneous mixtures. A particle size analysis was therefore not possible.

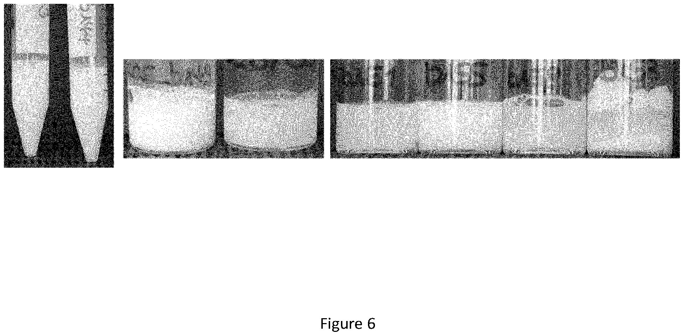

The manufacturing conditions and results are shown in Table 6 and Table 7 and figure 6. An overview of the used acids is shown in table 8:

*Table 6: Preparation conditions of the samples prepared via batch stirring process using different acids.*

| Formulation code | Protonation | Acid | [SIR] | [E100] | E100/SIR | FRR |
|---|---|---|---|---|---|---|
| NUC05_ | % | | mg/ml | mg/ml | | |
| b143 | 60 | Hydrochloric Acid | 167 | 250 | 1.5 | 4 |

(continued)

| Formulation code | Protonation | Acid | [SIR] | [E100] | E100/SIR | FRR |
|---|---|---|---|---|---|---|
| NUC05_ | % | | mg/ml | mg/ml | | |
| b144 | 60 | Acetic Acid | 167 | 250 | 1.5 | 4 |
| b145 | 60 | Oleic Acid | 167 | 250 | 1.5 | 4 |
| b146 | 60 | Octanoic Acid | 167 | 250 | 1.5 | 4 |
| b148 | 60 | Stearic Acid | 167 | 250 | 1.5 | 4 |
| b149 | 60 | Propionic acid | 167 | 250 | 1.5 | 4 |
| b150 | 60 | Valeric acid | 167 | 250 | 1.5 | 4 |
| b151 | 60 | Valeric acid | 167 | 250 | 1.5 | 4 |
| b152 | 60 | Citric acid | 167 | 250 | - | 4 |
| b153 | 30 | Citric acid | 167 | 250 | 1.5 | 4 |
| b154 | 60 | Malic acid | 167 | 250 | - | 4 |
| b155 | 30 | Malic acid | 167 | 250 | 1.5 | 4 |
| b164 | 20 | Citric acid | 167 | 250 | 1.5 | 4 |
| b158 | 60 | Benzoic acid | 167 | 250 | 1.5 | 4 |

Table 7: Formulation composition of samples prepared via batch stirring process using different acids.

| Formulation code | Protonation | Acid | [SIR] | [E100] | Optical evaluation | pH | size | PDI |
|---|---|---|---|---|---|---|---|---|
| NUC05_ | % | | mg/ml | mg/ml | | | nm | |
| b143 | 60 | Hydrochloric Acid | 26.7 | 40.0 | homogeneous | 6.1 | 235 | 0.231 |
| b144 | 60 | Acetic Acid | 26.7 | 40.0 | homogeneous | 6.0 | 205 | 0.179 |
| b145 | 60 | Oleic Acid | 26.7 | 40.0 | aggregated | 6-7 | n/a | n/a |
| b146 | 60 | Octanoic Acid | 26.7 | 40.0 | aggregated | 6-7 | n/a | n/a |
| b148 | 60 | Stearic Acid | 26.7 | 40.0 | aggregated | 6-7 | n/a | n/a |
| b149 | 60 | Propionic acid | 26.7 | 40.0 | homogeneous | 5.9 | 226 | 0.139 |
| b150 | 60 | Valeric acid | 26.7 | 40.0 | homogeneous | 6.3 | 98 | 0.279 |
| b151 | 60 | Valeric acid | 26.7 | 40.0 | homogeneous | 6.3 | 102 | 0.274 |
| b152 | 60 | Citric acid | - | - | n/a | n/a | n/a | n/a |
| b153 | 30 | Citric acid | 26.7 | 40.0 | flocculation | n/a | n/a | n/a |
| b154 | 60 | Malic acid | - | - | n/a | n/a | n/a | n/a |
| b155 | 30 | Malic acid | 26.7 | 40.0 | homogeneous | 6.4 | 165 | 0.162 |
| b164 | 20 | Citric acid | 26.7 | 40.0 | flocculation | 6 | n/a | n/a |
| b158 | 60 | Benzoic acid | 26.7 | 40.0 | homogeneous | 6.4 | 73 | 0.232 |

Table 8: Overview of the acids used for preprotonation and properties of the acids.

| Acid | Acidic function | Anion (Chain length) | pKa | logK (ALOGPS) | Water solubility |
|---|---|---|---|---|---|
| | No. | | | mg/ml | mg/ml |
| Hydrochloric Acid | 1 | Cl | -7 | 0.61 | n/a |
| Acetic Acid | 1 | C2 | 4.54 | -0.12 | 323 |
| Oleic Acid | 1 | C18 (unsat.) | 4.99 | 7.68 | 0.0001 |
| Octanoic Acid | 1 | C8 | 5.19 | 2.92 | 0.907 |

(continued)

| Acid | Acidic function | Anion (Chain length) | pKa | logK (ALOGPS] | Water solubility |
|---|---|---|---|---|---|
| | No. | | | mg/ml | mg/ml |
| Stearic Acid | 1 | C18 (sat.) | 4.95 | 8.02 | 0.00007 |
| Propionic acid | 1 | C3 | 4.75 | 0.68 | 352 |
| Valeric acid | 1 | C5 | 5.01 | 1.34 | 40 |
| Citric acid | 3 | | 3.1 / 4.8 / 6.4 | -1.3 | 106 |
| Malic acid | 2 | | 3.2 | -0.87 | 218 |
| Benzoic acid | 1 | C6H5 (arom.) | 4.08 | 1.72 | 7 |

*Observation*

- The use of different acids for pre-protonation leads to particles of different sizes.
- Surprisingly, the hydrophobic part of the acid is a decisive criterion for the processability and has an influence on the physical properties. Larger hydrophobic acid residues, which act as counterions after protonation, lead to an increase in hydrophobicity and to the precipitation of undefined aggregates (see experiments NUC05_b145-148) and not to the desired particle formation.
- The use of multidentate acids (citric acid, malic acid) leads to gelation or precipitation of the pre-protonated solvent solution when the same molarity of the substances is used (cf. NUC05_b144, b152, b154), presumably due to complexation / cross-linking of the polymer chains.
- The use of bidentate acids (malic acid) leads to homogeneous dispersions when the same molarity is used in relation to the acid groups present (cf. NUC05_b144, b143, b155).
- The use of tridentate acids (citric acid) leads to flocculation when the same molarity is used in relation to the acid groups present (Cf. NUC05_b143, b144, b164).

*Conclusions*

- The formulations can be made with different acids
- The type of acid influences the physical properties (particle size, homogeneity)
- The type of acid influences bioavailability / release

**6 Preparation of the formulation via continuous advection chamber**

*Production of the process solutions*

Process solution A (solvent): A solution consisting of 167 mg/mL sirolimus and 250 mg/mL Eudragit E100 in acetone is prepared. To 8 mL of this solution, 2 mL of acid solution are added and the mixture is left to stand for 2 hours. The process solution can then be used.

Process solution B (non-solvent): Demineralised water was used as process solution B.

*Processing*

The continuous production of nanosuspension containing sirolimus via an advection chamber was carried out with an asymmetric split-and-recombine mixer (Ehrfeld company). The process solutions were delivered by syringe pumps and the solvent/non-solvent ratio was adjusted via flow rate ratios (FRR). Ethanol was used for pre-rinsing and as a cleaning solution for the process lines. The exact process parameters are summarised in Table 9. ("FRR" = flow rate ratio; "TFR" = total flow rate; "SIR" = sirolimus). After an initial flushing phase and stabilisation of the process parameters, a volume of approx. 10 mL was collected.

To compare the tests using an advection chamber, samples with the same formulation parameters were prepared using a batch stirring process.

*Table 9: Preparation conditions of the samples prepared using a continuous advection chamber and batch stirring process.*

| Formulation code | Protonation | [SIR] | [E100] | FRR | TFR |
|---|---|---|---|---|---|
| NUC05_ | % | mg/ml | mg/ml | | mL/min |
| 040 | 25 | 167 | 250 | 4 | 30.0 |
| 036 | 60 | 167 | 250 | 4 | 30.0 |
| 037 | 75 | 167 | 250 | 4 | 30.0 |
| 038 | 90 | 167 | 250 | 4 | 30.0 |
| 039 | 100 | 167 | 250 | 4 | 30.0 |
| b163 | 25 | 167 | 250 | 4 | Batch stirring process |
| b159 | 60 | 167 | 250 | 4 | Batch stirring process |
| b160 | 75 | 167 | 250 | 4 | Batch stirring process |
| b161 | 90 | 167 | 250 | 4 | Batch stirring process |
| b162 | 100 | 167 | 250 | 4 | Batch stirring process |

*Table 10: Formulation composition of samples prepared using a continuous advection chamber and batch stirring process.*

| Formulation code | Protonation | Acetone | [SIR] | [E100] | E100 /SIR | Optical evaluation | pH | size | PDI |
|---|---|---|---|---|---|---|---|---|---|
| NUC05_ | % | v/v | mg/ml | mg/ml | | | | nm | |
| 040 | 25 | 0.16 | 26.7 | 40.0 | 1.5 | Highly viscous | 6.6 | - | (*) |
| 036 | 60 | 0.16 | 26.7 | 40.0 | 1.5 | homogenous | 6.3 | 147 | 0.294 |
| 037 | 75 | 0.16 | 26.7 | 40.0 | 1.5 | homogenous | 6.2 | 168 | 0.201 |
| 038 | 90 | 0.16 | 26.7 | 40.0 | 1.5 | homogenous | 5.9 | 203 | 0.204 |
| 039 | 100 | 0.16 | 26.7 | 40.0 | 1.5 | homogenous | 4.1 | 245 | 0.222 |
| b163 | 25 | 0.16 | 26.7 | 40.0 | 1.5 | Highly viscous | 6.5 | - | (*) |
| b159 | 60 | 0.16 | 26.7 | 40.0 | 1.5 | homogenous | 6.4 | 204 | 0.192 |
| b160 | 75 | 0.16 | 26.7 | 40.0 | 1.5 | homogenous | 6.0 | 275 | 0.160 |
| b161 | 90 | 0.16 | 26.7 | 40.0 | 1.5 | homogenous | 5.8 | 304 | 0.106 |
| b162 | 100 | 0.16 | 26.7 | 40.0 | 1.5 | homogenous | 4.2 | 315 | 0.229 |
| (*) multimodal distribution | | | | | | | | | |

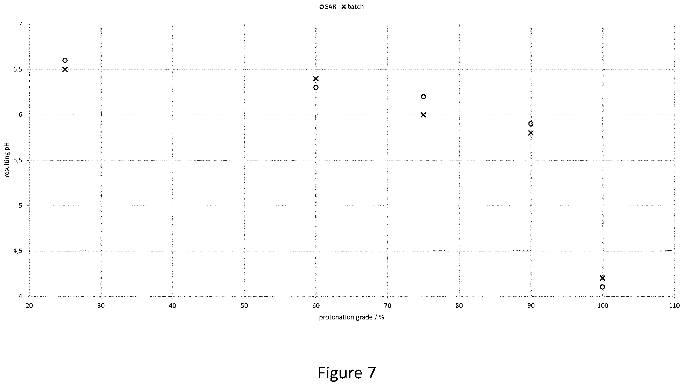

Figure 7 shows the data from table 10 as resulting pH-value vs. degree of protonation ("protonation grade"). The dispersions were produced in accordance with table 9.

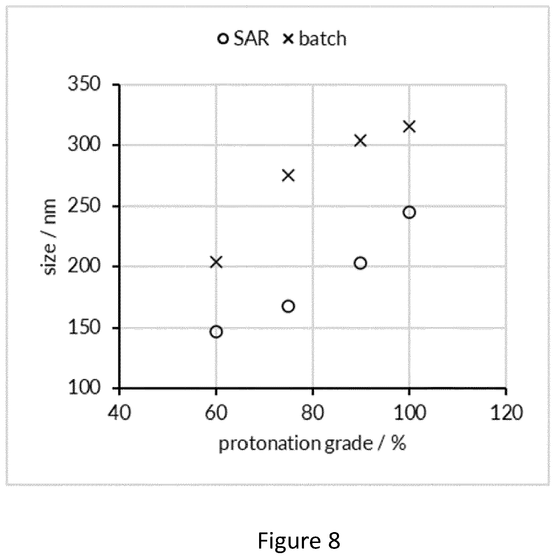

Figure 8 shows the data from table 10 as particle size vs. degree of protonation ("protonation grade"). The dispersions were produced in accordance with table 9.

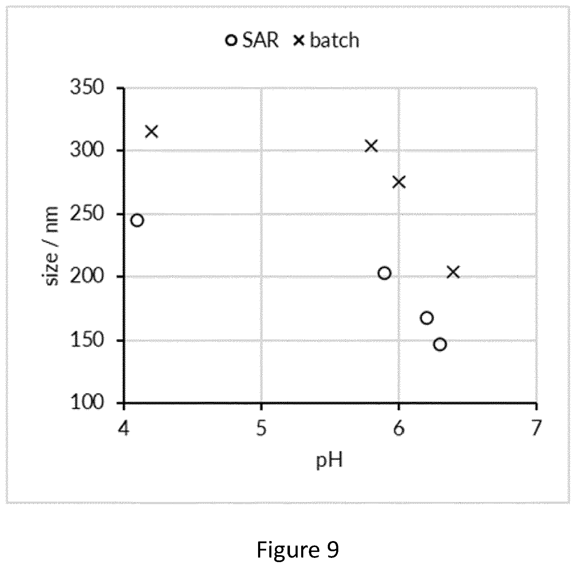

Figure 9 shows the data from table 10 as particle size vs. pH. The dispersions were produced in accordance with table 9.

***Observation***

- Particles of different sizes and homogeneities are generated depending on the protonation stage.

- An increase in the degree of protonation leads to a higher homogeneity, a decreasing pH value and an increase in particle size.
- Compared to production via the batch stirring process, smaller particle sizes are produced using an advection chamber.

*Conclusions*

- The formulation can be produced continuously with an advection chamber.
- The use of the advection chamber leads to smaller particle sizes compared to production in the batch stirring process.

**7 Encapsulation efficiency**

A prerequisite for the successful use of the solubilised active ingredient in any pharmaceutic dosage form is that it is encapsulated as completely as possible.

*Table 11: Preparation of the dispersions*

| Formulation code | Protonation | [SIR] | [E100] | FRR | TFR |
|---|---|---|---|---|---|
| | % | mg/ml | mg/ml | | ml/min |
| NUC05_028 | 60 | 167 | 250 | 4 | 30 |

*Table 12:Composition of the dispersions and their characterisation*

| Formulation code | Composition | | | | Characterisation | | | |
|---|---|---|---|---|---|---|---|---|
| | Acetone | [SIR] | [E100] | E100 /SIR | pH | Zeta potential | Diameter | PDI |
| | v/v | mg/ml | mg/ml | | | mV | nm | |
| NUC05_028 | 16 | 26.7 | 40.0 | 1.5 | 6.3 | 35 | 142 | 0.268 |

The prepared dispersions were therefore subjected to size exclusion chromatography (SEC) and the content of the filtrate quantified by HPLC as described above. Particles above a certain size are not retained by the column material and are found in the filtrate, while free, non-encapsulated active substance is retained by the column material.

A *HiTrap desalting* column from Cytiva with the following specifications was used:

- Resin: Sephadex G-25 Superfine, cross-linked dextran
- Bed volume: 5 ml
- Bed height: 25 mm
- Column inner diameter: 16 mm
- Recommended sample volume: < 1.5 ml
- Exclusion limit: 5000 Da
- pH stability: 2 to 13

Proceed as follows:

- Eluent and conditioning solution: 25 mM NaCl pH 3.3 (adjusted with glacial acetic acid)
- Conditioning: 25 mL
- Loading: 1.5 mL sample, discard eluate
- Elution: 1.5 mL 25 mM NaCl, collect eluate
- Dilute eluate with acetonitrile

*Table 13: Determined assay pre- and post SEC and encapsulation efficiency*

| Formulation code | Assay [mg/mL] | Encapsulation Efficiency [%] |
|---|---|---|
| NUC05_028 pre SEC | 22.3 | |
| NUC05_028 post SEC | 20.5 | 92 |

### Conclusions

- The active substance content of the dispersion was smaller than the theoretically calculated (22.3 mg/ml versus 26.7 mg/ml).
- In Nuc05_028, the content of the filtrate decreased by 8% due to SEC. The active substance was present in the dispersion almost completely (92%) in particles that were not retained by column material.

### 8 Interaction of dispersions with mucin

The interaction of dispersions with mucin was evaluated by means of electrostatic investigations. For this purpose, the zeta potential of mucin/dispersion mixtures was determined. The zeta potential determined as a function of the mixing ratios serves as a measure of the interactions between mucin and particle dispersion.

Chitosan was used as a positively charged mucoadhesive reference substance. The polymer used, Eudragit E100, was used alone as reference substance as well. A formulation containing sirolimus (code NUC05_b143) was used as an example with regard to its mucin adhesion (for formulation parameters see Table 7).

For the test, 0.5 % (w/w) mucin suspension was prepared and different amounts of the test substances were added (final composition 0.05-0.80 % (w/w). The mucin mixtures were then diluted in a dilution factor of 5 with a pH buffer (Acetate buffer, pH =4, 50 mM; Phosphate buffer, pH =7, 50 mM).

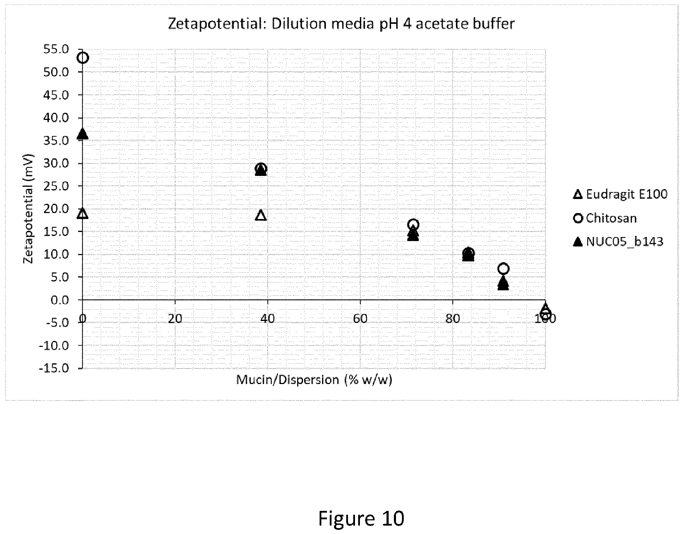

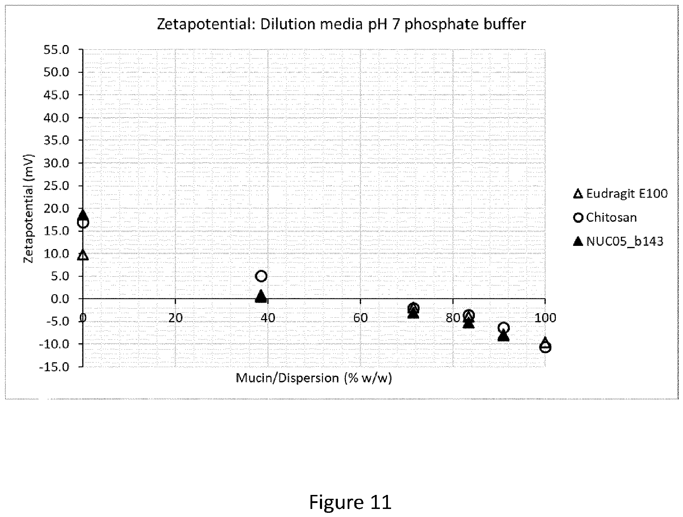

The increase of the zeta potential with increasing amount of mucoadhesive substance can be observed for the tested references as well as for the prepared dispersion. The results are shown in figures 10 and 11.

### 9 Release of the dispersion

A prerequisite for the successful use of the solubilised active substance in any pharmaceutical dosage form is release. Release means that the active ingredient can leave the solubilisate unchanged. This creates the prerequisite for the active ingredient to be absorbed by the body over time.

To characterise the diffusion of the active ingredient from the nanoparticles, a two-compartment model was used as the experimental setup.

### Campaign 1

The dispersion was prepared with a microfluidic device. A dispersion in which the active ingredient was micellarly solubilised in the polymer Soluplus served as a control. To prepare the Soluplus dispersion, sirolimus was dissolved in acetone at a concentration of 150 mg/mL and continuously processed at a volume ratio of 1:7 with a 150 mg/mL Soluplus solution. The total flow rate was 17.5 mL/min. Approximately 10 mL of an opalescent dispersion was obtained. To prepare the Sirolimus dispersion containing Eudragit, Sirolimus was dissolved in acetone at a concentration of 167 mg/mL and Eudragit E100 at a concentration of 250 mg/mL. Subsequently, the reaction was carried out with a HCl solution to obtain a degree of protonation of 60% and a water content of 20% (v/v). This solution was continuously mixed with water in a volume ratio of 1:5.3. This produces an opalescent dispersion. The total flow rate at which the dispersion was collected was 31.4 mL/min. 10 mL were prepared.

The particle size of the dispersions was determined after preparation and after dilution with water of 1:100 using dynamic light scattering (DLS) at a scattering angle of 150°.

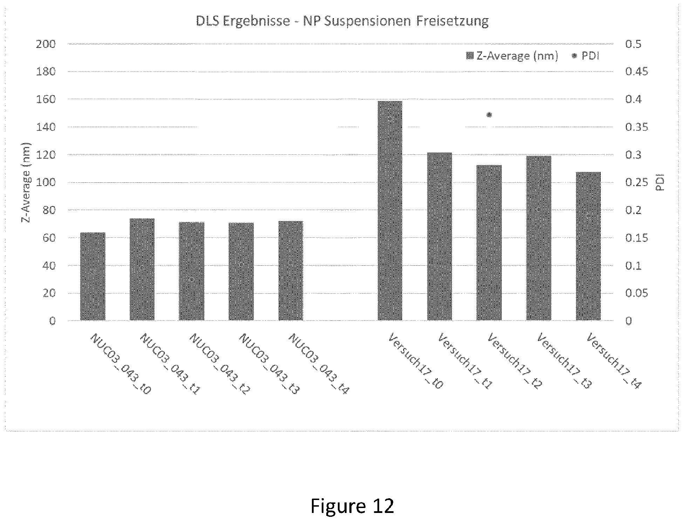

The zeta potential of the dispersions was determined after preparation and after dilution with water of 1:10 using a Zetasizer Nano ZS (Malvern) after dilution of the sample with water (10 $\mu$l dispersion plus 1 ml water). Results are shown in table 15 and figure 12.

*Table 14: Manufacturing process*

| Formulation code | Protonation | [SIR] | [E100] | [Soluplus] in Non-solvent | FRR | TFR |
|---|---|---|---|---|---|---|
| | % | mg/ml | mg/ml | mg/ml | | ml/min |
| NUC03_043 | - | 150 | - | 150 | 7 | 17.5 |
| Trial 17 | 60 | 167 | 250 | - | 5.3 | 31.4 |

*Table 15: Composition of the dispersion and its characterisation*

| Formulation code | Composition | | | | Characterisation | | |
|---|---|---|---|---|---|---|---|
| | Acetone | [SIR] | [Polymer] | Polymer/SIR | Zeta potential | size | PDI |
| | v/v | mg/ml | mg/ml | | mV | nm | |
| NUC03_043 | 12 | 19 | 131 | 7 | 1 | 64 | 0.089 |
| Trial 17 | 16 | 26 | 40 | 1.5 | n/a | 159 | 0.365 |

The selection of the dialysis membrane depends on the particle size of the nanosuspension. The chosen material should be compatible with acetone. Regenerated cellulose with a MWCO of 300 kDa was selected.

With this pore size, the nanoparticles are retained behind the membrane due to their size, while the active ingredient (914 Da) and the free, stabilising polymers Soluplus (~118 kDa) and Eudragit E100 (47 kDa) can pass through the membrane.

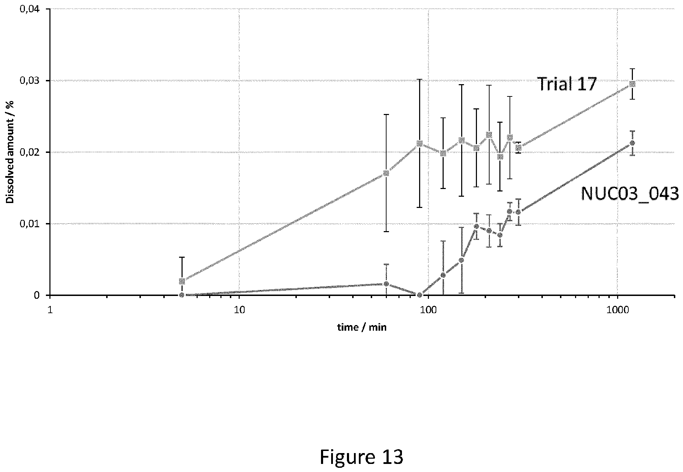

To carry out the release, a defined volume of the dispersion was filled into 9 cm of the dialysis tube (300 kDa) and sealed. The release vessel was filled with medium and the sample packed in the tube was added. The medium was stirred continuously during the experiment. Each release was performed three times. Results are shown in figure 13.

Figure 13 shows the release of two dispersions: Sirolimus solubilized in E100 ("Trial 17") and Sirolimus solubilized in Soluplus (control, "NUC03_043") with logarithmic time axis. Shown is the mean value from each of 3 independent experiments and the standard deviation of the mean values.

Quantification of the samples was done by HPLC under the conditions given below.

*Table 16: Release conditions*

| | |
|---|---|
| Apparatus | USP II, Mini Vessel |
| Membrane | Biotech Cellulose Ester (CE) Membrane, MWCO 300 kDa Spectrum ™, inner diameter 20 mm<br>Membrane area: approx. 12 cm$^2$ |
| Sample volume | 6 ml dispersion |
| Medium | Artificial saliva (pH 7.4) |
| Media volume | 200 ml |
| Rotation | 200 rpm |
| Times | 5, 60, 90, 120, 150, 180, 210, 240, 270, 300, 1200 min |
| Withdrawal volume | 500 $\mu$l |
| Temperature | 37°C |

*Table 17: Composition of the artificial saliva (Simulated Saliva 2, SS2)*

| Component | Concentration / g/l |
|---|---|
| KCl | 0.720 |
| NaCl | 0.600 |
| CaCl$_2$ | 0.220 |
| KH$_2$PO$_4$ | 0.680 |
| Na$_2$HPO$_4$ * 12 H$_2$O | 0.866 |

(continued)

| Component | Concentration / g/l |
|---|---|
| KHCO$_3$ | 1.500 |
| KSCN | 0.060 |
| Citric acid | 0.030 |

Simulated Saliva according to Marques, M. R. C., Loebenberg, R. & Almukainzi, M. Simulated Biological Fluids with Possible Application in Dissolution Testing. Dissolution Technol. 18, 15-28 (2011).

*Table 18: Chromatographic conditions for quantification of the active substance*

| Pillar: | 150 x 4.6 mm, 3 µm, L1 | | |
|---|---|---|---|
| River: | 0.5 mL/min | | |
| Mobile Phase A: | 0.01 % H3PO4 (100 µL H3PO4 in 1000 mL AVP) | | |
| Mobile Phase B: | ACN | | |
| Gradient | Time | A [%] | B [%] |
| | 0 | 50 | 50 |
| | 15 | 25 | 75 |
| | 15,1 | 50 | 50 |
| | 20 | 50 | 50 |
| Injection volume: | 5 µL | | |
| Wavelength: | 277 nm | | |
| Diluent | ACN | | |
| Linearity range tested | 20 - 80 µg/mL | | |
| LOQ estimated | 2 µg/mL | | |

The chemical stability of the dispersion during release was assessed using the chromatograms obtained during quantification. No new signals could be observed in the chromatogram. The active substance is stable under the selected release conditions.

*Table 19: Data of the formulation NUC03_043*

| time / min | V1 | V2 | V3 | mean | Stddev | CV |
|---|---|---|---|---|---|---|
| 0 | 0 | 0 | 0 | 0 | 0 | #DIV/0! |
| 5 | 0 | 0 | 0 | 0 | 0 | #DIV/0! |
| 60 | 0 | 0.00468 | 0 | 0.00156 | 0.002702 | 173% |
| 90 | 0 | 0 | 0 | 0 | 0 | #DIV/0! |
| 120 | 0 | 0.00828 | 0 | 0.00276 | 0.00478 | 173% |
| 150 | 0 | 0.00908 | 0.00552 | 0.00487 | 0.00458 | 94% |
| 180 | 0.00778 | 0.01140 | 0.00961 | 0.00960 | 0.00181 | 19% |
| 210 | 0.00767 | 0.01161 | 0.00765 | 0.00898 | 0.00228 | 25% |
| 240 | 0.00913 | 0.00944 | 0.00655 | 0.00837 | 0.00159 | 19% |
| 270 | 0.01070 | 0.01309 | 0.01120 | 0.01166 | 0.00126 | 11% |
| 300 | 0.01035 | 0.01368 | 0.01069 | 0.01157 | 0.00183 | 16% |
| 1200 | 0.01928 | 0.02229 | 0.02210 | 0.02122 | 0.00169 | 8% |

*Table 20: Data of the formulation trial 17*

| time / min | V1 | V2 | V3 | mean | Stddev | CV |
|---|---|---|---|---|---|---|
| 0 | 0 | 0 | 0 | 0 | 0 | #DIV/0! |
| 5 | 0.00581 | 0 | 0 | 0.00194 | 0.00335 | 173% |
| 60 | 0.01907 | 0.02404 | 0.00800 | 0.01704 | 0.00821 | 48% |
| 90 | 0.02611 | 0.02660 | 0.01085 | 0.02119 | 0.00896 | 42% |
| 120 | 0.02092 | 0.02410 | 0.01445 | 0.01982 | 0.00492 | 25% |
| 150 | 0.02936 | 0.02168 | 0.01374 | 0.02159 | 0.00781 | 36% |
| 180 | 0.02224 | 0.02497 | 0.01447 | 0.02056 | 0.00545 | 26% |
| 210 | 0.02903 | 0.02290 | 0.01526 | 0.02240 | 0.00690 | 31% |
| 240 | 0.02145 | 0.02270 | 0.01384 | 0.01933 | 0.00480 | 25% |
| 270 | 0.02742 | 0.02264 | 0.01595 | 0.02200 | 0.00576 | 26% |
| 300 | 0.02144 | 0.01997 | 0.02038 | 0.02060 | 0.00076 | 4% |
| 1200 | 0.03197 | 0.02822 | 0.02832 | 0.02950 | 0.00214 | 7% |

**Conclusions**

- The active ingredient solubilised in polymeric methacrylate releases sirolimus faster than that solubilised in Soluplus, although the particle size of this formulation is larger than that of the Soluplus formulation.
- The active substance is chemically stable during the experiment.

**Campaign 2**

The dispersion was prepared with a microfluidic device.

To prepare the Sirolimus dispersion containing Eudragit, Sirolimus was dissolved in acetone at a concentration of 167 mg/mL and Eudragit at a concentration of 250 mg/mL. Subsequently, the reaction was carried out with a HCl solution to obtain a degree of protonation of 60%, respectively 75% and 90% and a water content of 20% (v/v). This solution was continuously mixed with water in a volume ratio of 1:4. This produces an opalescent dispersion. The total flow rate at which the dispersion was collected was 30 mL/min. 10 mL were prepared.

The control was a physical mixture of the same chemical composition as NUC05_036, in which the active ingredient was dispersed (stirred) in the mixture as an unmodified crystalline powder.

The particle size of the dispersions was determined after preparation and after dilution with water of 1:100 using dynamic light scattering (DLS) at a scattering angle of 150°. The zeta potential of the dispersions was determined after preparation and after dilution with water of 1:10 with a Zetasizer Nano ZS (Malvern) after dilution of the sample with water (10 µl dispersion plus 1 ml water).

*Table 21: Manufacturing process*

| Formulation code | Prototype | Protonation | [SIR] | [E100] | FRR | TFR |
|---|---|---|---|---|---|---|
| | | % | mg/ml | mg/ml | | ml/min |
| NUC05_036 | 1a | 60 | 167 | 250 | 4 | 30 |
| NUC05_037 | 1b | 75 | 167 | 250 | 4 | 30 |
| NUC05_038 | 1C | 90 | 167 | 250 | 4 | 30 |
| 211118_NUC05_036 -control | Control | | | | 4 | 30 |

*Table 22: Composition of the dispersion and its characterisation*

| Formulation code | Composition | | | | Characterisation | | |
|---|---|---|---|---|---|---|---|
| | Acetone | [SIR] | [E 100] | E100/SIR | pH | size | PDI |
| | v/v | mg/ml | mg/ml | | | nm | |
| Prototype 1a | 16 | 26.7 | 40 | 1,5 | 6.3 | 147 | 0.294 |
| Prototype 1b | 16 | 26.7 | 40 | 1,5 | 6.2 | 168 | 0.201 |
| Prototype 1c | 16 | 26.7 | 40 | 1,5 | 5.9 | 203 | 0.222 |
| Control | 16 | 26.7 | 40 | 1,5 | | N/D | N/D |

### Quantification of Sirolimus by UV-absorbance

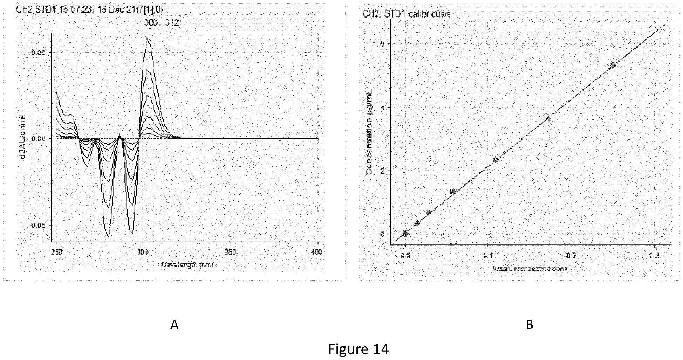

The standard solutions of Sirolimus were prepared using serial addition method. Briefly, a stock solution of SIR was prepared by dissolving powder of SIR in DMSO mixture at concentration $\sim$ 2 mg/mL. Calculated aliquots of the stock solution were added to corresponding medium and spectral data were obtained at 4 concentration points ranging from 0.3 to 5.1 $\mu$g/mL. No precipitation occurred during the standards preparation. Area under the second derivative curve (Figure 14A) in wavelength region 300 - 312 nm was selected to build the standard curves (Figure 14A). Linearity of the standard curves was characterised by $R^2$ > 0.999. Results are shown in figure 14 A and B.

### Flux measurements

Flux measurements were performed using $\mu$FLUX™ apparatus (Pion Inc., Billerica, MA, USA). In this device, four side-by-side donor-receiver pairs are placed in a temperature controlled unit connected to a circulating water bath. The donor chambers were separated from the receiver chambers by a vertically positioned filter supported membrane. Prior to the assay the membrane was formed by placing 25 $\mu$l of 20% lecithin in dodecane lipid solution (GIT Lipid, Pion Inc., Billerica MA) on the filter support material (PVDF, polyvinylidene fluoride, 1.54 cm$^2$ open area, 0.45 $\mu$m pore size, 120 $\mu$m thickness, 70% nominal porosity). Subsequently, the donor chambers were filled with 20 ml of simulated saliva medium and loaded with the different prototype formulations (600 $\mu$l each). The formulations were added at a concentration of 26.6 mg/ml to the donor compartments to obtain a final concentration of 0.78 mg/ml of Sirolimus. The receiver compartments contained 20 ml of acceptor sink buffer (ASB, Pion Inc., Billerica, MA, USA). ASB is a 20 mM HEPES buffer solution adjusted to pH 7.4 containing surfactant (1% SDS ) that allows to maintain sink conditions during the experiments. Crossbar magnetic stirrers, rotating at 150 rpm, provided agitation in the chambers. Media in donor and receiver compartments were maintained at 37 $\pm$ 2 °C. Each flux experiment was done in duplicate.

Absorbance spectra in all four receiver chambers were monitored between 250 and 400 nm (2 nm spectral resolution) in situ every 30 seconds using fibre optic UV/VIS probes equipped with 20 mm pathlength tips and connected to the Rainbow® instrument (Pion Inc., Billerica, MA, USA).

The concentration of SIR in the receiver chambers was calculated from the UV absorbance using standard curves described above.

From the obtained concentration-time profiles in the receiver compartments, flux (J) was calculated. The latter is a measurement of the mass transfer through the membrane, and it is defined as total amount of material (or mass) crossing one unit area of the membrane per unit time, as provided by:

$$J = \frac{dm}{A \cdot dt} = \frac{V}{A} \cdot \frac{dc}{dt}$$

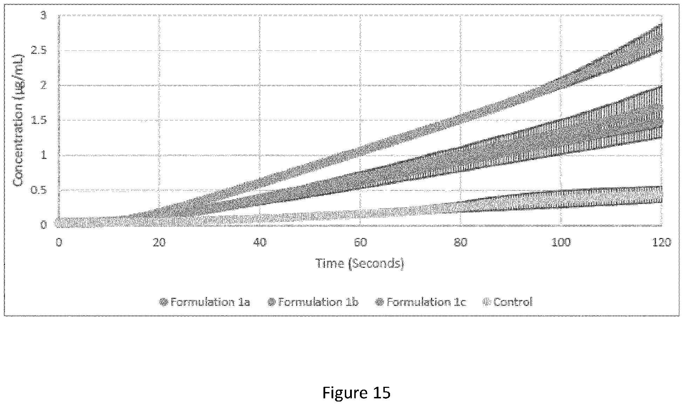

where A is the area of the membrane (1.54 cm$^2$), V is the volume of the receiver compartment (20 ml) and dc/dt is the slope of the concentration-time profile of SIR in the receiver compartment (Figure 15).

*Table 23 Results from flux measurements (average of 2 samples)*

| Formulation code | Flux (J) (30-60 min) | Flux relative to control |
|---|---|---|
| | $\mu$g/min/cm$^2$ | |
| NUC05_036 | 0.171 | 4.8 |
| NUC05_037 | 0.190 | 5.3 |

(continued)

| Formulation code | Flux (J) (30-60 min) | Flux relative to control |
|---|---|---|
| | $\mu$g/min/cm$^2$ | |
| NUC05_038 | 0.292 | 8.1 |
| Control | 0.036 | 1 |

## Conclusions

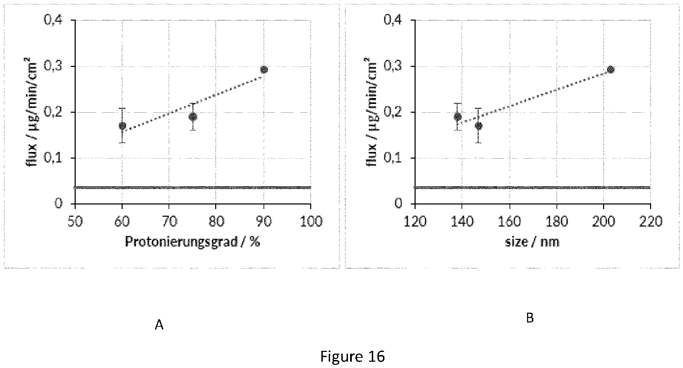

- The active ingredient solubilised and nanoised in the polymer methacrylate releases sirolimus at least 4.8 times faster than a physical mixture of the same composition in which the active ingredient is not solubilised (control).
- *Surprisingly,* the release of the formulations increases with particle size. That is, the formulation with the largest particle size releases the fastest (Figure 16B). Likewise the release of the formulation increases with degree of protonation ("Protonierungsgrad") (figure 16A).

## 10 Chemical stability of the dispersion

A prerequisite for the successful use of the solubilised active ingredient in any pharmaceutical dosage form is its chemical stability. The dispersion produces an intermediate that is further processed into a final dosage form. For the duration of processing, the dispersion must not show any detectable degradation.

The dispersion prepared in chapter 9 was therefore stored at room temperature and the content quantified by HPLC after different storage times as described above.

*Table 24 Degradation of two dispersions of solubilised sirolimus as a* function *of storage time*

| Formulation code | Time / d | CalcAmount [mg/mL] | Recovery [%] |
|---|---|---|---|
| NUC03_043 | 0 | 18,62 | 100.00 |
| | 2 | 18,22 | 97.85 |
| | 3 | 18,50 | 99.36 |
| Trial 17 | 0 | 23,01 | 100.00 |
| | 2 | 23,91 | 103.91 |
| | 3 | 23,40 | 101.70 |

## Conclusions

- The active substance content of the dispersions was 2 % (NUC03_043) and 11 % (trial 17) lower than the theoretically calculated content.
- After three days of storage at room temperature, no detectable amounts of degradation products formed.
- Measurable degradation sets in at higher temperature and/or storage time.
- The dispersion is sufficiently stable to be used as an intermediate product in pharmaceutical production.

## 11 Transmission electron microscopy (TEM) of the dispersions

Three samples with different degrees of protonation were prepared and the samples were characterised by electron microscopy.

The mixing of the liquid streams was carried out using a microfluidic device. For this purpose, sirolimus was dissolved in acetone at a concentration of 167 mg/ml and mixed with a prehydrated solution of Eudragit E100. This solution was continuously mixed with water in a volume ratio of 1:5.3. This produces an opalescent dispersion. The total flow rate at which the dispersion was collected was 33 ml/min. 10 ml were prepared.

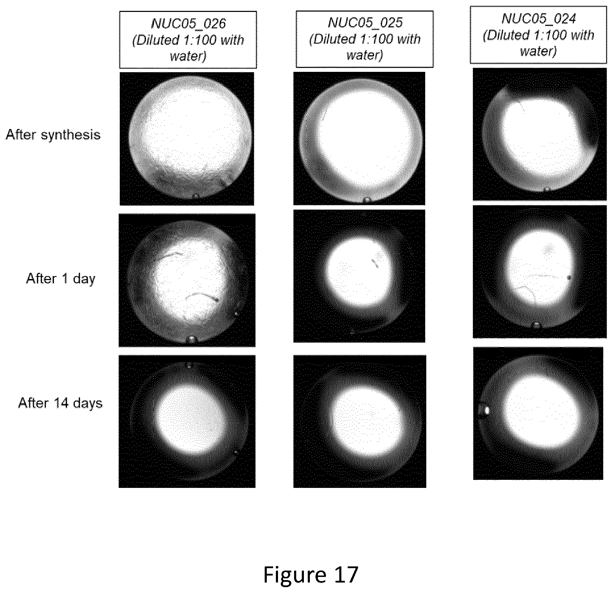

The particle size of the dispersions was determined after preparation and after dilution with water of 1:100 using dynamic light scattering (DLS) at a scattering angle of 150°. Images of dilutions are shown in figure 17.

The zeta potential of the dispersions was determined after preparation and after dilution with water of 1:10 using a Zetasizer Nano ZS (Malvern) after dilution of the sample with water (10 μl dispersion plus 1 ml water).

Table 25: Variation of the degree of protonation: manufacturing process

| Formulation code | Protonation | [SIR] | [E100] | E100/ SIR | FRR | TFR |
|---|---|---|---|---|---|---|
| NUC05_ | % | mg/ml | mg/ml | | | ml/min |
| 024 | 100 | 167 | 250 | 1.5 | 5.3 | 33.0 |
| 025 | 60 | 167 | 250 | 1.5 | 5.3 | 33.0 |
| 026 | 25 | 167 | 250 | 1.5 | 5.3 | 33.0 |

Table 26: Variation of the degree of protonation: dispersion and its characterisation

| Formulation code | Protonation | Acetone | [SIR] | [E100] | pH | Zeta potential | size (2 h) | PDI (2 h) | size (14 d) | PDI (14 d) |
|---|---|---|---|---|---|---|---|---|---|---|
| NUC05_ | % | v/v | mg/ml | mg/ml | | mV | nm | | nm | |
| 024 | 100 | 0.16 | 21.3 | 30.3 | 6.1 | 59 | 207 | 0.297 | 169 | 0.376 |
| 025 | 60 | 0.16 | 21.3 | 30.3 | 6.1 | 50 | 141 | 0.227 | 89 | 0.307 |
| 026 | 25 | 0.16 | 21.3 | 30.3 | 6.4 | 49 | n/a | (*) | 54 | 0.126 |
| (*) multimodal distribution | | | | | | | | | | |

### TEM Instrument parameters

TEM micrographs were recorded on a Tecnai F20 transmission electron microscope operated at an acceleration voltage of 200 kV. Electrons are generated by a Schottky field emission cathode. The information limit of the electron optics is specified at 0.19 nm. Micrographs were recorded using a direct electron detection camera (K2 summit, Gatan) with a size of 3710 pixels × 3838 pixels or with a CCD camera (ultrascan 1000, Gatan) with a size of 2048 pixels × 2048 pixels. The cameras have been calibrated using a cross grating replica standard with 2160 lines per mm. All micrographs used for the data analysis were acquired with a pixel size of 0.17 nm.

### TEM Sample preparation

Cryo-TEM samples were prepared according to a standard protocol using a Vitrobot© (FEI, Netherlands) plunge freeze device. TEM grids used for this method are Quantifoil© copper grids. To hydrophilize these grids, they are flow discharged prior to application. Subsequently, 3 μl of sample dispersion is applied to the grid and excess solution is blotted off with a filter paper. Directly after the blotting, the specimen is plunged into liquid ethane. Finally, the frozen specimen is stored in liquid nitrogen until TEM inspection.

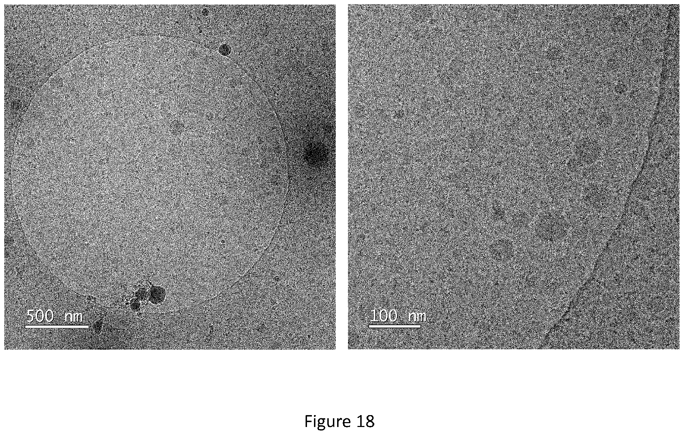

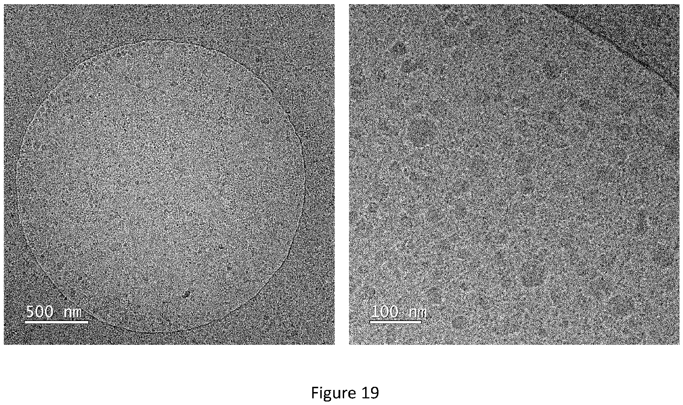

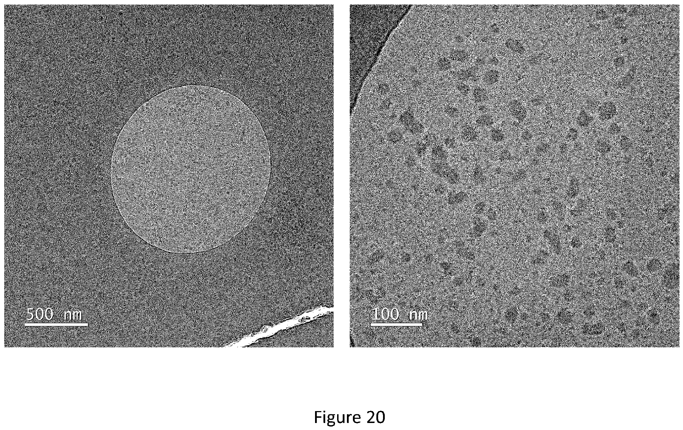

Drop cast samples are prepared by applying approx 3 μl of sample dispersion to a carbon coated TEM grid (custom made). For negative staining with uranyl acetate the same amount of 4% aq. solution of uranyl acetate is additionally applied to the grid and left on the grid for 30 seconds. Then, excess solution is blotted off with a filter paper and the sample is allowed to dry at ambient conditions. After completely dry, the sample is ready for TEM inspection. TEM images are shown in Figures 18 - 20.

### Conclusions

- Primarily roundish particles are visible, the diameter of which becomes smaller as the degree of protonation decreases.
- The particle sizes determined with dynamic light scattering are confirmed.
- No reflexes could be found that can be assigned to crystalline sirolimus. The active substance is amorphously solubilised in the matrix.

## 12 X-ray diffraction of the dispersion

As one part of pharmaceutical development, a nano formulation was produced from the active ingredient. To answer the question of whether Sirolimus with a particle size of 100 to 150 nm is crystalline or amorphous in the nano formulation, the active ingredient dispersion was analysed by X ray powder diffractometry using a capillary sample holder In addition, taking into account identical measurement conditions, the corresponding placebo formulation (Eudragit E 100 in aqueous acetone solution) is investigated in order to be able to identify possible Sirolimus Bragg signals in the subsequent phase analysis.

Furthermore, a powdered active ingredient samples of Sirolimus was examined by means of XRPD as reference. A single crystal structure known from the literature (Swindells, The X ray crystal structure of Sirolimus, Canadian Journal of Chemistry 56 (1978) 2491) can be used as a reference for the phase analysis to be carried out.

*Table 27: Manufacturing process*

| Formulation code | Protonation | [SIR] | [E100] | FRR | TFR |
|---|---|---|---|---|---|
| NUC05_ | % | mg/ml | mg/ml | | ml/min |
| 025 | 60 | 167 | 250 | 5.2 | 33.0 |

*Table 28: Dispersion and its characterisation*

| Formulation code | Protonation | Acetone | [SIR] | [E100] | E100 /SIR | pH | Zeta potential | size | PDI |
|---|---|---|---|---|---|---|---|---|---|
| NUC05_ | % | v/v | mg/ml | mg/ml | | | mV | nm | |
| 025 | 60 | 0.16 | 21.5 | 32.3 | 1.5 | 6.1 | 50 | 141 | 0.227 |

### *XRPD method description*

For Powder X Ray Diffraction (XRPD) the samples were placed into standard glass capillaries of 10 mm (liquid sample) and 0 7 mm diameter (powder). The measurements were performed at room temperature with a D 8 Bruker Advance Diffractometer (Cu-K$\alpha$ = 1.54059 Å, Johansson primary beam monochromator, position sensitive detector) in transmission mode with rotation of the sample. Data were collected in a two-theta range of 1.5 - 50°. The tube voltage and current were 40 kV and 40 mA, respectively.

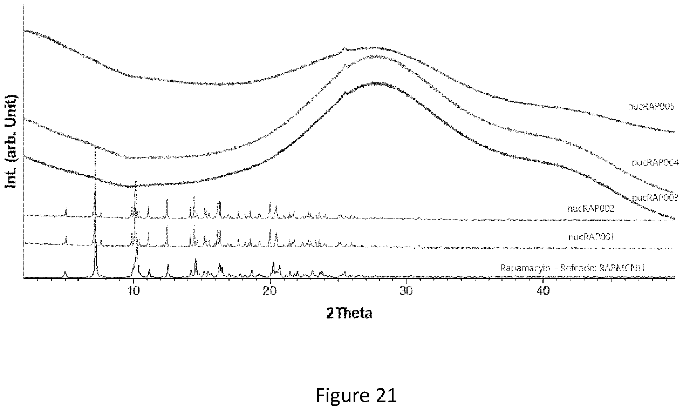

Therefore, a so-called phase analysis of the samples are performed by using X ray powder diffraction. The measured diffraction patterns are compared to an X ray powder diffractogram calculated from single crystal data. The samples nucRAP 001 & 002 show X ray diffraction patterns that are consistent with the reference pattern of rapamacyin. Results are shown in Figure 21.

In general, the amorphous part of a sample is identified by a so-called characteristic halo in an X ray powder pattern. The crystalline part of a sample is shown by X ray signals. In both samples nucRAP 003 004 an amorphous halo was detected On the one hand it can be assumed that the API exists in the amorphous state in sample nucRAP 003 on the other hand it might be the case that the low concentration of the sample is insufficient for the used method In both samples nucRAP 003 and nucRAP 004 an amorphous halo with only one signal at 25.48° 2Theta could be observed. The signal at 25.48°might therefore originate from a compound of the placebo mixture or can be attributed to an artifact

### *Conclusion*

• No reflexes could be found that can be assigned to crystalline sirolimus. The active substance is amorphously solubilised in the matrix.

## 13 Proof of suitability as a template formulation by embedding alternative active ingredients

The use of the formulation as a template formulation was investigated with different active ingredients. Prednisolone, itraconazole, ibuprofen and tacrolimus were investigated as active ingredients.

Different formulation parameters were varied for the respective active ingredients in the batch stirring process and/or in the continuous mixing process.

| Drug | Molar mass | Water solubility | Melting point | Log P | Hansen solubility parameter (HSP) | | | |
|---|---|---|---|---|---|---|---|---|
| | | | | | polar | dispersive | H-bonding | Total |
| | g/mol | mg/ml | °C | | | | | |
| Sirolimus | 914 | 0.0026 | 180 | 4.56 | | | | |
| Tacrolimus | 804 | | 126 | 4.5 | 3.1 | 18.8 | 5 | 19.7 |
| Itraconazole | 705 | 0.0096 | 166 | 5.66 | 5.5 | 19.4 | 10.3 | 22.6 |
| Ibuprofen | 206 | 0.021 | 75 | 3.97 | 6.91 | 16.60 | 9.97 | 20.6 |
| Prednisolone | 360 | 0.223 | 235 | 1.62 | 5.59 | 20.63 | 16.65 | 27.1 |

**Polymers**

| | | | | | | | | |
|---|---|---|---|---|---|---|---|---|
| Eudragit E100 | | | | | | | | 18.74 |
| Soluplus | | | | | 0.3 | 17.4 | 8.6 | 19.4 |

### 13.1 Ibuprofen

### 13.1.1 Variation of degree of protonation and non-solvent/solvent ratio in batch process

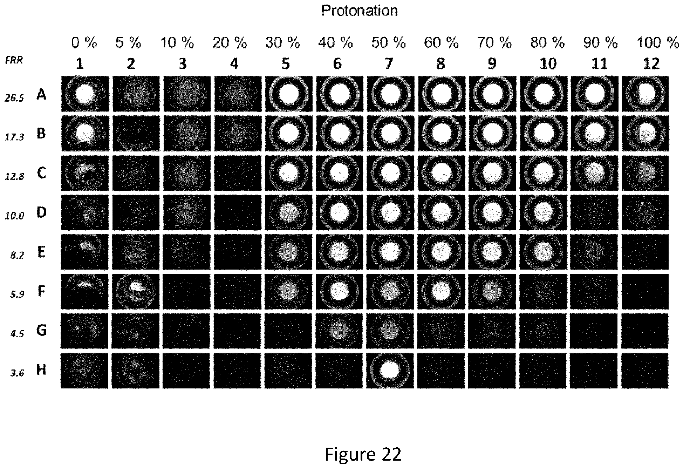

Evaluation of the variation in degree of protonation and non-solvent/solvent ratio at constant API/Eudragit E ratio (1/1.8). (see figure 22)

*Implementation*

- in 96 well plate
- Batch process: Water in 96-well plate; addition of process solution A to B in the appropriate ratio; shaking of the plate, characterisation
- Process solution A (solvent): A solution consisting of 167 mg/mL ibuprofen and 300 mg/mL Eudragit E100 in acetone is prepared. Appropriately concentrated acid solutions are added to this solution to achieve the desired degree of protonation (0-100 %) and amount of water (20 % (v/v)). The mixture was left to stand for 2 hours. The Proess solution was then used.
- Process solution B (non-solvent): Demineralised water was used as process solution B.

*Table 29: Preparation conditions of the ibuprofen/Eudragit-E mixtures: FRR in corresponding row.*

| Row | Volume Solvent | Volume Non-Solvent | FRR | [Ibuprofen] final | [E100] final |
|---|---|---|---|---|---|
| | μL | μL | | mg/ml | mg/ml |
| A | 10 | 265 | 26.5 | 4.86 | 8.73 |
| B | 15 | 260 | 17.3 | 7.29 | 13.09 |
| C | 20 | 255 | 12.8 | 9.72 | 17.45 |
| D | 25 | 250 | 10.0 | 12.15 | 21.82 |
| E | 30 | 245 | 8.2 | 14.57 | 26.18 |
| F | 40 | 235 | 5.9 | 19.43 | 34.91 |
| G | 50 | 225 | 4.5 | 24.29 | 43.64 |
| H | 60 | 215 | 3.6 | 29.15 | 52.36 |

*Table 30: Preparation conditions of the ibuprofen/Eudragit-E mixtures: Degree of protonation in corresponding column.*

| Column | Protonation |
|---|---|
|  | % |
| 1 | 0 |
| 2 | 5 |
| 3 | 10 |
| 4 | 20 |
| 5 | 30 |
| 6 | 40 |
| 7 | 50 |
| 8 | 60 |
| 9 | 70 |
| 10 | 80 |
| 11 | 90 |
| 12 | 100 |

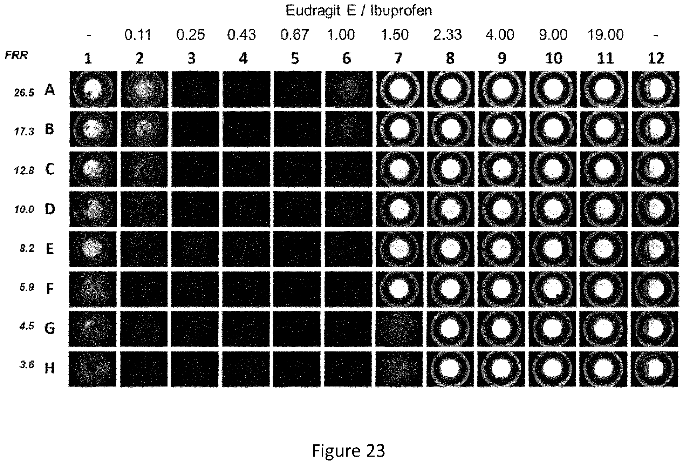

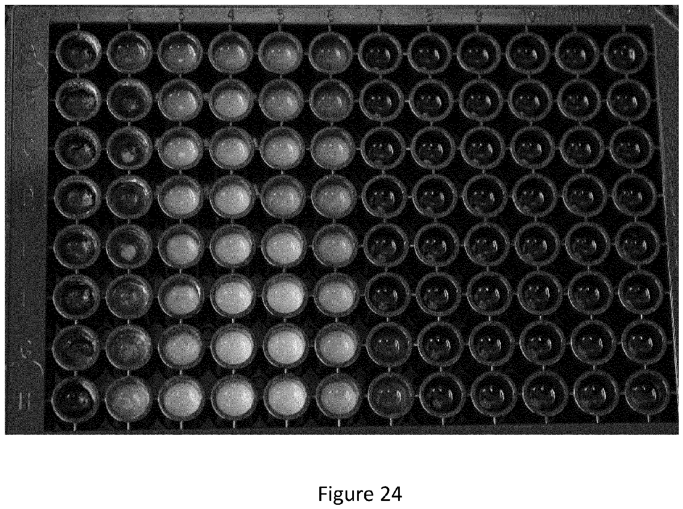

For results, see also figures 23 and 24.

***Results.***

***Observation***

- Homogeneity / precipitation of solids as a function of the degree of protonation
- Degree of protonation significantly responsible for homogeneity; 50 % protonation optimal;
- The lower the non-solvent/solvent ratio, the higher the tendency to crystallise.
- DLS: particle size measurement of the undiluted, clear solutions measurable, however no statement possible due to different viscosities

***Conclusion***

• Formulation can be used to prepare ibuprofen dispersions

**13.1.2 Variation stabiliser/API and non-solvent/solvent ratio in batch process**

Evaluation of the variation in Eudragit E/Ibuprofen and non-solvent/solvent ratio at a constant protonation level of 60 %.

***Implementation***

- in 96 well plate:
- Batch process: Water in 96-well plate; addition of process solution A to B in appropriate ratios; shaking of the plate, characterisation
- Process solution A (solvent): Appropriate concentrated solution consisting of ibuprofen and Eudragit E100 in ethanol is prepared. Appropriate concentrated acid solutions are added to this solution to achieve the desired degree of protonation (0-100 %) and amount of water (20 % (v/v)). The mixture was left to stand for 2 hours. The process solution was then used.
- Process solution B (non-solvent): Demineralised water was used as process solution B.

*Table 31: Manufacturing conditions of the ibuprofen/Eudragit-E mixtures: FRR in corresponding row.*

| Row | Volume Solvent | Volume Non-Solvent | FRR | [Solvent] | [Total Solid] |
|-----|----------------|--------------------|----|-----------|---------------|
|     | μL | μL |  | v/v% | mg/mL |
| A | 10 | 265 | 26.5 | 3.64 | 5.82 |
| B | 15 | 260 | 17.3 | 5.45 | 8.73 |
| C | 20 | 255 | 12.8 | 7.27 | 11.64 |
| D | 25 | 250 | 10.0 | 9.09 | 14.55 |
| E | 30 | 245 | 8.2 | 10.91 | 17.45 |
| F | 40 | 235 | 5.9 | 14.55 | 23.27 |
| G | 50 | 225 | 4.5 | 18.18 | 29.09 |
| H | 60 | 215 | 3.6 | 21.82 | 34.91 |

*Table 32. Final ibuprofen concentrations in wells. Data in mg/mL.*

|   | 1 | 2 | 3 | 4 | 5 | 6 | 7 | 8 | 9 | 10 | 11 | 12 |
|---|---|---|---|---|---|---|---|---|---|----|----|----|
| A | 5.8 | 5.2 | 4.7 | 4.1 | 3.5 | 2.9 | 2.3 | 1.7 | 1.2 | 0.6 | 0.3 | 0.0 |
| B | 8.7 | 7.9 | 7.0 | 6.1 | 5.2 | 4.4 | 3.5 | 2.6 | 1.7 | 0.9 | 0.4 | 0.0 |
| C | 11.6 | 10.5 | 9.3 | 8.1 | 7.0 | 5.8 | 4.7 | 3.5 | 2.3 | 1.2 | 0.6 | 0.0 |
| D | 14.5 | 13.1 | 11.6 | 10.2 | 8.7 | 7.3 | 5.8 | 4.4 | 2.9 | 1.5 | 0.7 | 0.0 |
| E | 17.5 | 15.7 | 14.0 | 12.2 | 10.5 | 8.7 | 7.0 | 5.2 | 3.5 | 1.7 | 0.9 | 0.0 |
| F | 23.3 | 20.9 | 18.6 | 16.3 | 14.0 | 11.6 | 9.3 | 7.0 | 4.7 | 2.3 | 1.2 | 0.0 |
| G | 29.1 | 26.2 | 23.3 | 20.4 | 17.5 | 14.5 | 11.6 | 8.7 | 5.8 | 2.9 | 1.5 | 0.0 |
| H | 34.9 | 31.4 | 27.9 | 24.4 | 20.9 | 17.5 | 14.0 | 10.5 | 7.0 | 3.5 | 1.7 | 0.0 |

*Table 33. Final Eudragit E concentrations in wells. Data in mg/mL.*

|   | 1 | 2 | 3 | 4 | 5 | 6 | 7 | 8 | 9 | 10 | 11 | 12 |
|---|---|---|---|---|---|---|---|---|---|----|----|----|
| A | 0.0 | 0.6 | 1.2 | 1.7 | 2.3 | 2.9 | 3.5 | 4.1 | 4.7 | 5.2 | 5.5 | 5.8 |
| B | 0.0 | 0.9 | 1.7 | 2.6 | 3.5 | 4.4 | 5.2 | 6.1 | 7.0 | 7.9 | 8.3 | 8.7 |
| C | 0.0 | 1.2 | 2.3 | 3.5 | 4.7 | 5.8 | 7.0 | 8.1 | 9.3 | 10.5 | 11.1 | 11.6 |
| D | 0.0 | 1.5 | 2.9 | 4.4 | 5.8 | 7.3 | 8.7 | 10.2 | 11.6 | 13.1 | 13.8 | 14.5 |
| E | 0.0 | 1.7 | 3.5 | 5.2 | 7.0 | 8.7 | 10.5 | 12.2 | 14.0 | 15.7 | 16.6 | 17.5 |
| F | 0.0 | 2.3 | 4.7 | 7.0 | 9.3 | 11.6 | 14.0 | 16.3 | 18.6 | 20.9 | 22.1 | 23.3 |
| G | 0.0 | 2.9 | 5.8 | 8.7 | 11.6 | 14.5 | 17.5 | 20.4 | 23.3 | 26.2 | 27.6 | 29.1 |
| H | 0.0 | 3.5 | 7.0 | 10.5 | 14.0 | 17.5 | 20.9 | 24.4 | 27.9 | 31.4 | 33.2 | 34.9 |

*Table 34. Final Eudragit E/Ibuprofen ratio in wells.*

|   | 1 | 2 | 3 | 4 | 5 | 6 | 7 | 8 | 9 | 10 | 11 | 12 |
|---|---|---|---|---|---|---|---|---|---|----|----|----|
| A | - | 0.1 | 0.3 | 0.4 | 0.7 | 1.0 | 1.5 | 2.3 | 4.0 | 9.0 | 19.0 | - |
| B | - | 0.1 | 0.3 | 0.4 | 0.7 | 1.0 | 1.5 | 2.3 | 4.0 | 9.0 | 19.0 | - |
| C | - | 0.1 | 0.3 | 0.4 | 0.7 | 1.0 | 1.5 | 2.3 | 4.0 | 9.0 | 19.0 | - |
| D | - | 0.1 | 0.3 | 0.4 | 0.7 | 1.0 | 1.5 | 2.3 | 4.0 | 9.0 | 19.0 | - |
| E | - | 0.1 | 0.3 | 0.4 | 0.7 | 1.0 | 1.5 | 2.3 | 4.0 | 9.0 | 19.0 | - |
| F | - | 0.1 | 0.3 | 0.4 | 0.7 | 1.0 | 1.5 | 2.3 | 4.0 | 9.0 | 19.0 | - |

(continued)

|   | 1 | 2 | 3 | 4 | 5 | 6 | 7 | 8 | 9 | 10 | 11 | 12 |
|---|---|---|---|---|---|---|---|---|---|----|----|----|
| G | - | 0.1 | 0.3 | 0.4 | 0.7 | 1.0 | 1.5 | 2.3 | 4.0 | 9.0 | 19.0 | - |
| H | - | 0.1 | 0.3 | 0.4 | 0.7 | 1.0 | 1.5 | 2.3 | 4.0 | 9.0 | 19.0 | - |

*Results*

*Observation*

- Homogeneity / precipitation of solids as a function of the Eudragit E / ibuprofen ratio;
- Eudragit E / ibuprofen ratio significantly responsible for particle formation
- The higher the Eudragit E / ibuprofen ratio, the lower the particle formation rate;
- Low influence of non-solvent/solvent ratio on particle formation
- DLS: Particle size measurement of the undiluted, clear solutions measurable, however no statement possible due to different viscosities;

*Conclusion*

- Eudragit E / Ibuprofen ratio significantly responsible for particle formation
- Formulation can be used to prepare ibuprofen dispersions

**13.2 Itraconazole**

Evaluation of the variation in degree of protonation and non-solvent/solvent ratio at constant API/Eudragit E ratio (1/1.5).

*Implementation*

- in 96 well plate:
- Batch process: Water in 96-well plate; addition of process solution A to B in appropriate ratios; shaking of the plate, characterisation
- Process solution A (solvent): A solution consisting of 2 mg/mL itraconazole and 3 mg/mL Eudragit E100 in acetone is prepared. Appropriately concentrated acid solutions are added to this solution to achieve the desired (0-100 %) and amount of water (20 % (v/v)). The mixture was left to stand for 2 hours. Subsequently, the process solution was used.
- Process solution B (non-solvent): Demineralised water was used as process solution B.

*Table 35. Preparation conditions of the itraconazole/Eudragit-E mixtures: FRR in appropriate series.*

| Row | Volume Solvent | Volume Non-Solvent | FRR | [Solvent] | [Itraconazole] final | [E100] final |
|-----|----------------|--------------------|-----|-----------|----------------------|--------------|
|     | μL | μL |  | v/v% | mg/ml | mg/ml |
| A | 10 | 265 | 26.5 | 3.64 | 0.058 | 0.087 |
| B | 15 | 260 | 17.3 | 5.45 | 0.087 | 0.131 |
| C | 20 | 255 | 12.8 | 7.27 | 0.116 | 0.175 |
| D | 25 | 250 | 10.0 | 9.09 | 0.145 | 0.218 |
| E | 30 | 245 | 8.2 | 10.91 | 0.175 | 0.262 |
| F | 40 | 235 | 5.9 | 14.55 | 0.233 | 0.349 |

(continued)

| Row | Volume Solvent | Volume Non-Solvent | FRR | [Solvent] | [Itraconazole] final | [E100] final |
|---|---|---|---|---|---|---|
| G | 50 | 225 | 4.5 | 18.18 | 0.291 | 0.436 |
| H | 60 | 215 | 3.6 | 21.82 | 0.349 | 0.524 |

*Table 36. Preparation conditions of the itraconazole/Eudragit-E mixtures: Degree of protonation in corresponding column.*

| Column | Protonation |
|---|---|
|  | % |
| 1 | 0 |
| 2 | 5 |
| 3 | 10 |
| 4 | 20 |
| 5 | 30 |
| 6 | 40 |
| 7 | 50 |
| 8 | 60 |
| 9 | 70 |
| 10 | 80 |
| 11 | 90 |
| 12 | 100 |

### Results

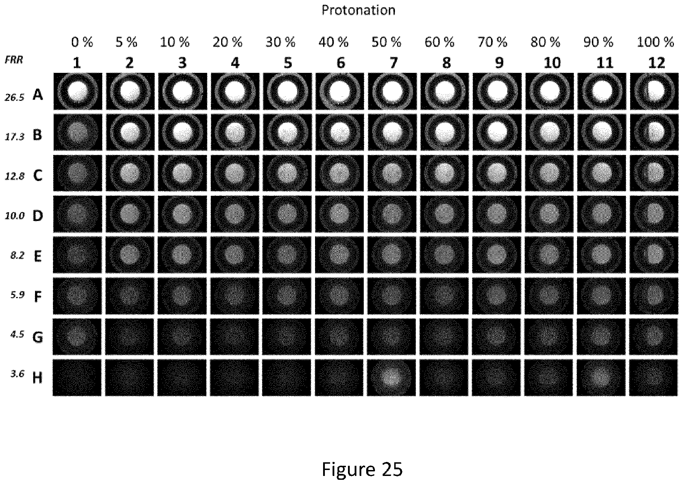

see also figure 25

*Table 37. Determined particle sizes of Eudragit E/Itraconazole formulations derived from DLS characterization (Z-average diameter, dilution factor 20 in water).*

|  | 1 | 2 | 3 | 4 | 5 | 6 | 7 | 8 | 9 | 10 | 11 | 12 |
|---|---|---|---|---|---|---|---|---|---|---|---|---|
| A | 216 | 262 | 267 | 297 | 290 | 283 | 269 | 291 | 273 | 266 | 261 | 254 |
| B | 238 | 276 | 259 | 322 | 292 | 297 | 277 | 265 | 306 | 273 | 298 | 257 |
| C | 148 | 263 | 279 | 294 | 290 | 316 | 342 | 289 | 300 | 300 | 287 | 311 |
| D | 242 | 269 | 298 | 304 | 404 | 325 | 295 | 300 | 318 | 336 | 285 | 295 |
| E | 260 | 324 | 293 | 353 | 340 | 381 | 349 | 332 | 355 | 304 | 300 | 292 |
| F | 314 | 248 | 362 | 361 | 405 | 378 | 354 | 367 | 343 | 320 | 311 | 309 |
| G | 342 | 310 | 364 | 365 | 356 | 382 | 302 | 316 | 321 | 321 | 339 | 295 |
| H | 191 | 427 | 396 | 379 | 392 | 346 | 328 | 326 | 340 | 328 | 271 | 319 |

### Observation

- Particle formation as a function of the degree of protonation and non-solvent/solvent ratio
- The lower the non-solvent/solvent ratio, the higher the particle formation

*Conclusion*

• Formulation can be used to prepare itraconazole dispersions

**13.3 Prednisolone**

Evaluation of the variation in degree of protonation and non-solvent/solvent ratio at constant API/Eudragit E ratio (1/1.5).

*Implementation*

- in 96 well plate:
- Batch process: Water in 96-well plate; addition of process solution A to B in the appropriate ratio; shaking of the plate, characterisation
- Process solution A (solvent): A solution consisting of 10 mg/mL prednisolone and 15 mg/mL Eudragit E100 in acetone is prepared. Appropriately concentrated acid solutions are added to this solution to achieve the desired degree of protonation (0-100 %) and amount of water (20 % (v/v)). The mixture was left to stand for 2 hours. The Proess solution was then used.
- Process solution B (non-solvent): Demineralised water was used as process solution B.

Table 38. Manufacturing conditions of the prednisolone/Eudragit-E mixtures: FRR in corresponding row.

| Row | Volume Solvent | Volume Non-Solvent | FRR | [Prednisolone] final | [E100] final |
|---|---|---|---|---|---|
| | μL | μL | | mg/ml | mg/ml |
| A | 10 | 265 | 26.5 | 0.291 | 0.291 |
| B | 15 | 260 | 17.3 | 0.436 | 0.436 |
| C | 20 | 255 | 12.8 | 0.582 | 0.582 |
| D | 25 | 250 | 10.0 | 0.727 | 0.727 |
| E | 30 | 245 | 8.2 | 0.873 | 0.873 |
| F | 40 | 235 | 5.9 | 1.164 | 1.164 |
| G | 50 | 225 | 4.5 | 1.455 | 1.455 |
| H | 60 | 215 | 3.6 | 1.745 | 1.745 |

Table 39 Preparation conditions of the prednisolone/Eudragit-E mixtures: Degree of protonation in corresponding column.

| Column | Protonation |
|---|---|
| | % |
| 1 | 0 |
| 2 | 5 |
| 3 | 10 |
| 4 | 20 |
| 5 | 30 |
| 6 | 40 |
| 7 | 50 |
| 8 | 60 |
| 9 | 70 |

(continued)

| Column | Protonation |
|--------|-------------|
|        | % |
| 10 | 80 |
| 11 | 90 |
| 12 | 100 |

*Results*

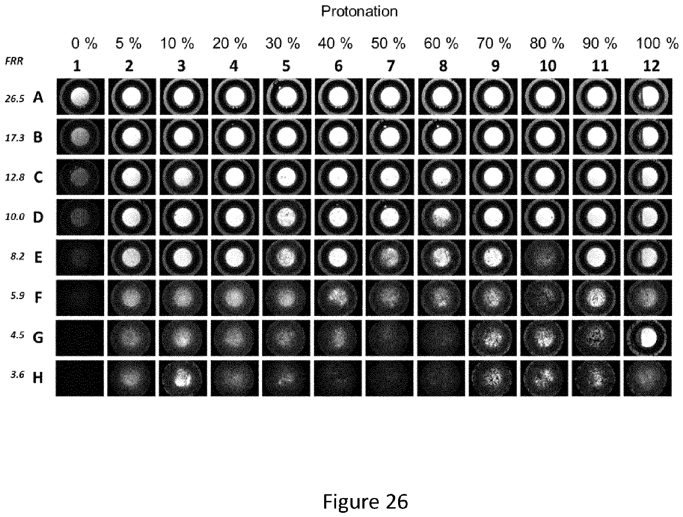

see also figure 26

*Observation*

- Homogeneity / precipitation of solids as a function of the degree of protonation
- FRR of greater than 10 generally provides homogeneous solutions: Prednisolone solubilised;
- The lower the non-solvent/solvent ratio, the higher the tendency to crystallise.

*Conclusion*

Formulation can be used to prepare dispersions of prednisolone nanoparticles.

**13.4 Preparation of tacrolimus nanoparticles stabilized by an anionic poly(methacrylate)**

The methodology according to the present invention can also be used to prepare nanoparticles of a hydrophobic active pharmaceutical ingredient (API) which are stabilized by an anionic polymeric stabilizing agent.

This is exemplified in the following using tacrolimus as the hydrophobic active pharmaceutical ingredient (API) and Eudragit L100, e.g. Eudragit L100-55, as anionic polymeric stabilizing agent.

2.0 g Eudragit L100-55 and 400 mg Tacrolimus ("Tac") are dissolved in 6 mL ethanol under stirring until completely dissolved. Then, ethanol is added to obtain a total volume of 10 mL resulting in a concentration of 200 mg/mL Eudragit L100-55 and 40 mg/mL Tacrolimus. The stock solution is then further pre-neutralized by addition of an appropriate amount of a 3.41M NaOH solution in water to achieve a targeted deprotonation level of 20 %. The solution is then dried under vacuum or inert gas until all solvent and water is evaporated. The solid residue is rehydrated with 3.41 M NaOH solution to achieve a targeted deprotonation level of 100 %. Then, 40 mL of water are added under stirring. Stirring is maintained for 60 minutes resulting in a stable Tac/Eudragit L100-55 dispersion with a particle size of 391 nm (Z-average, dilution factor 100 in water).

**14 Examination of aggregate state of active pharmaceutical ingredient stabilized by poly(/methacrylate)**

NMR spectroscopy can be used to distinguish between the two states of aggregation, liquid and solid. The question of the investigations was in which state sirolimus ("SIR") is present in Eudragit E100 particles, also depending on the degree of protonation of the polymer.

Liquids usually show very narrow lines in the 1H-NMR, which corresponds to long transverse relaxation times in the range of several 100 ms - s. In the solid state, especially in densely packed or crystalline solids, the transverse relaxation becomes much shorter compared to the value in the liquid, corresponding to a much larger line width. If this value is mainly determined by the technique in the case of low molecular weight liquids, intrinsic factors are decisive in the solid state. In the case of 1H-NMR, this is mainly the 1H-1H homonuclear dipolar interaction; in the case of disperse mixtures of substances, the influence of the interfaces on the line width caused by differences in susceptibility may also have to be taken into account. Therefore, when analysing the samples, special attention was paid to the line width in addition to the signals and their chemical shift. All samples were measured with identical NMR parameters, only the number of experiments per spectrum was varied between 4 and 16 after optimisation of the acquisition. The essential NMR parameters are summarised in the table below.

*Table:40 Parameters of NMR-measurement*

| Device | 300 MHz Bruker SB Avance Nano |
|---|---|
| Probe head | BBFO, 5 mm |
| Pulss sequence | • Single pulse-FID (zg)<br>• Hahn echo |
| Number of Scans (n) | 4 and 16, resp. |
| spectral band width | 10 ppm |
| Number of digital points | 66560 |
| Repetition time | 10 s |
| Enhancement | 0.25 dB |
| Data processing | • Line Broadening<br>• Fourier-Transformation<br>• Phase correction individually<br>• Chemical shift referenced to acetone for all samples 0 Hz |

Eleven samples with different protonation were measured in D20 in 5 mm NMR tubes.

| No. | designation | ID | [SIR] / mg/ml | [E100] / mg/ml | [Acetone] / v/v |
|---|---|---|---|---|---|
| 1 | Sirolimus in Acetone | - | 167 | - | 100 |
| 2 | Eudragit E100 in Acetone | - | - | 250 | 100 |
| 3 | Sirolimus + E100 in Acetone | - | 167 | 250 | 100 |
| 4 | Placebo Solvent: Eudragit E100 + acid in Acetone (60 % protonation) | FLM02_b614 | | 200 | 80 |
| 5 | Solvent: Sirolimus/E100 + acid in Acetone (60 % protonation) | FLM02_b610 | 133.6 | 200 | 80 |
| 6 | Placebo: Eudragit E100+acid in $D_2O$/Acetone (60 % protonation) | FLM02_b611 | - | 40 | 16 |
| 7 | Placebo: Eudragit E100+ acid in $D_2O$/Acetone (25 % protonation) | FLM02_b612 | - | 40 | 16 |
| 8 | Placebo: Eudragit E100+ acid in $D_2O$/Acetone (100 % protonation) | FLM02_b613 | - | 40 | 16 |

| No. | designation | ID | [SIR] / mg/ml | [E100] / mg/ml | [Acetone] / v/v |
|---|---|---|---|---|---|
| 9 | Verum: SIR/E100+ acid in $D_2O$/Acetone (60 % protonation) | FLM02_b615 | 26.7 | 40 | 16 |
| 10 | Verum: SIR/E100+ acid in $D_2O$/Acetone (25 % protonation) | FLM02_b616 | 26.7 | 40 | 16 |
| 11 | Verum: SIR/E100+ acid in $D_2O$/Acetone (100 % protonation) | FLM02_b617 | 26.7 | 40 | 16 |

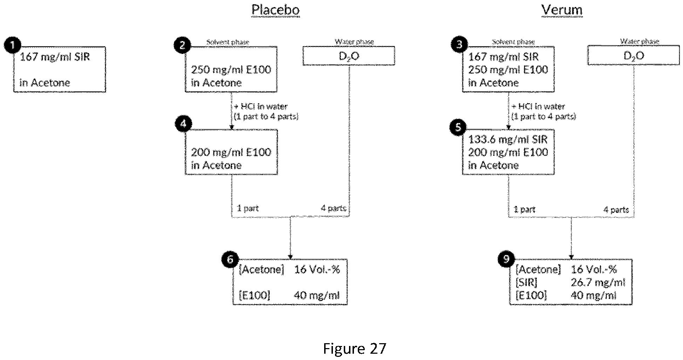

Sample were prepared in accordance with Figure 27.

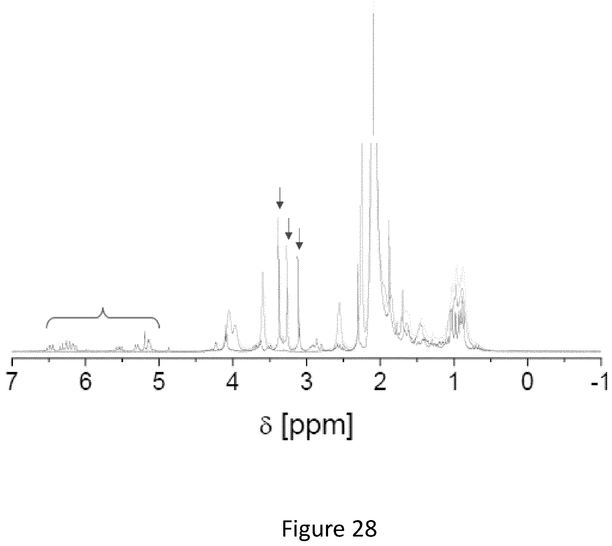

The different samples were subjected to $^1$H-NMR, and the spectra were analysed and compared. Spectra of samples 1 and 3 (sirolimus ("SIR") dissolved in acetone, and sirolimus ("SIR") + E100 dissolved in acetone can be inspected in Figure 28. The signals highlighted by arrows in the region 3 - 3.5 ppm and by the bracket in the region 5 - 7 ppm indicate the presence of sirolimus and are characteristic (fingerprint) signals of sirolimus.

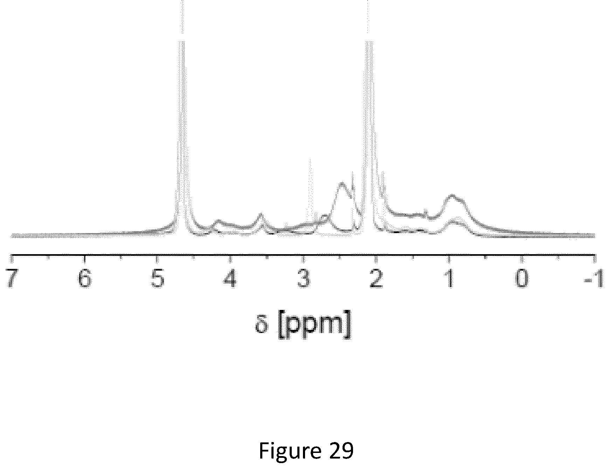

Spectra of placebo samples 6 - 8 are shown in Figure 29 and (obviously) only show the respective signals of Eudragit which are relatively broad.

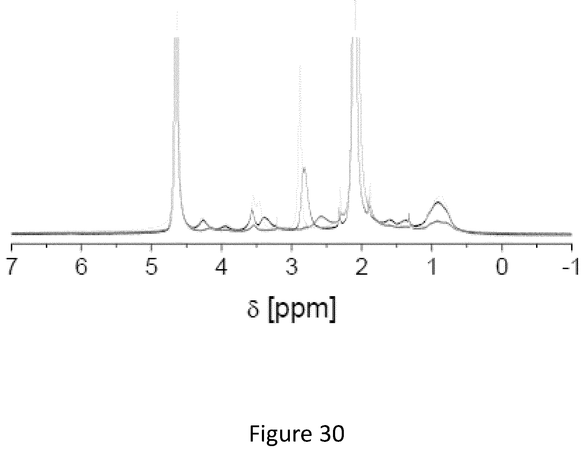

Spectra of Verum-samples 9 - 11 are shown in Figure 30. The characteristic signals of sirolimus in the regions 3 - 3.5 ppm and $\delta$ 5 - 7 ppm are hardly visible.

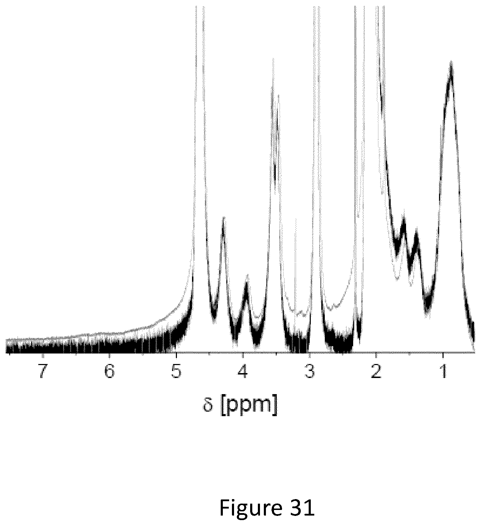

A comparison of placebo sample 8 (Eudragit E100+ acid in $D_2O$/Acetone, 100% protonation) with "verum"-sample 11 (SIR/E100+ acid in $D_2O$/Acetone 100% protonation) is shown in figure 31 and makes it clear that peaks characteristic of sirolimus (in the regions 3 - 3.5 ppm and 5 - 7 ppm) can hardly be detected, and instead for such "Verum"-sample 11, there is considerable line broadening. Such line broadening is due to a change of the aggregate state of sirolimus in the E100-stabilized nanoparticles from liquid to solid. Similar result have been obtained for 60% and 25% protonation.

*Conclusion*

Regardless of the degree of protonation of the Verum samples, no indications could be found that can be assigned to freely mobile ("liquid") or dissolved sirolimus. The active substance is immobilised in the matrix of the polymeric stabilizing agent and can be considered to be in a solid state.

**15 Evaluation of pharmacokinetics of tacrolimus or sirolimus nanodispersions in rats**

Rats were selected as an animal model for evaluation of the pharmacokinetic of tacrolimus or sirolimus nanosuspensions. Application was done sublingual in order to benefit from the reduced thickness of this tissue compared to the buccal area.

**15.1 Sirolimus encapsulated in E100 in rats (2022NCM001)**

The pharmacokinetic profile of a nanoparticular formulation of Sirolimus after sublingual administration in five rats was evaluated.

The preparation of formulation took place in accordance with the following tables:

*Table: Preparation of formulation*

| Formulation code | Protonation | [SIR] | [E100] | FRR | TFR |
|---|---|---|---|---|---|
| | % | mg/ml | mg/ml | | ml/min |
| FLM04-017 | 60 | 167 | 250 | 4 | 30 |

*Table: Composition of dispersion and characterisation*

| Formulation code | Composition | | | | Characterization | | | |
|---|---|---|---|---|---|---|---|---|
| | Acetone | [SIR] | [E100] | E100/ SIR | pH | size | PDI | zeta |
| | v/v | mg/ml | mg/ml | | | nm | | mV |
| FLM04_017 | 16 | 26.7 | 40 | 1.5 | 6.33 | 154 | 0.389 | + 50 |

Naive, adult male Sprague Dawley rats of around 300 g body weight were housed in a separate temperature-controlled room (20-24°C) and maintained in a 12h light/12h dark cycle. Food and water were available ad libitum throughout the duration of the study.

Formulation was freshly prepared on the day of application. It was administered sublingually to each animal at a dose of 5 mg per kg body weight.

For sublingual administration, rats were shortly anaesthetized with isoflurane. Prior to application, the sublingual area was blotted dry with cotton swabs. The appropriate volume of the formulation was applied under the tongue with a pipette. The animals were kept in a sitting position for 5 minutes. After 5 minutes the area under the tongue was blotted dry again to avoid oral administration of the remaining suspension. Then the animals were placed into the cage until recovery.

Blood samples were collected by puncture of the lateral tail vein at the following time points: predose, 15 min, 30 min, 1 h, 2 h, 4 h, 8 h and 24 h post dose. At each of the designated time points 20 $\mu$l blood was collected from the tail vein into K3-EDTA capillaries (Minivette POCT, SARSTEDT). The blood samples were transferred into polypropylene tubes and frozen on dry ice within 1-2 min. of sampling and stored at -20°C until LC-MS analysis.

*Liquid chromatography - mass spectrometry (LC-MS)*

*Preparation of working solutions for calibration standards and quality controls*

Individual stock solutions were used for preparation of calibration standards and quality control samples (QCs). Stock solutions of Sirolimus in DMSO (1 mg/mL) were diluted in DMSO to obtain a start solution and further working solutions.

*Preparation of blood samples*

All samples were kept on ice until the addition of the precipitation reagent. Calibration standards and quality controls (QCs) were prepared by spiking 20 $\mu$L blank blood with 2.4 $\mu$L of the corresponding working solution, then vortexed twice for 5 s with a 5-s break in between. Calibration standards and quality controls were prepared in duplicates.

A volume of 20 $\mu$L of each unknown sample, zero, blank and wash blank sample was spiked with 2.4 $\mu$L DMSO. The mixtures were vortexed twice for 5 s with a 5-s break in between. After 1 min of equilibration, a volume of 40 $\mu$L internal standard Sirolimus (1200 ng/ml) diluted in HFBA 0.2% in ACN was added to each calibration standard, QC sample, zero sample and unknown sample, while a volume of 40 $\mu$L plain ACN was added to all blanks and wash blanks. Samples were vortexed for 10 s and then centrifuged for 10 minutes at 8000 rpm and at room temperature.

Finally, an aliquot of 50 $\mu$L of the supernatant was diluted with 50 $\mu$L water in a fresh tube, vortex mixed for 10 seconds, and an aliquot was transferred to LC-MS autosampler vials and subsequently subjected to LC-MS with an injection volume of 15 $\mu$l.

*LC-MS settings*

The HPLC system consisted of a Dionex UltiMate 3000 RS pump and Dionex UltiMate 3000 RS column compartment and an Accela Open Autosampler (Thermo Fisher Scientific, USA). Mass spectrometry was performed on a Q Exactive (Orbitrap) accurate mass spectrometer equipped with a heated electrospray (H-ESI) interface (Thermo Fisher Scientific, USA) connected to a PC running the standard software Chromeleon 7.2.

Analytes were separated on a Poroshell HPH-C18, 2.7u, 100×3 mm analytical column (Agilent Technologies) with a C6-Phenyl, 4×2.0 mm ID precolumn. The HPLC was performed in the gradient mode using Methanol as organic phase (A) and Ammonium acetate 10mM in water as aqueous phase (B) as given in the table below. The pump flow rate was set to 700 $\mu$l/min.

*Table: HPLC gradient*

| Mobile phase | 0 min | 0.1 min | 0.2 min | 2.4 min | 2.5 min | 4.5 min |
|---|---|---|---|---|---|---|
| A (%) | 25 | 25 | 97 | 97 | 25 | 25 |
| B (%) | 75 | 75 | 3 | 3 | 75 | 75 |

An optimized MS tune file was used and as a lock mass for internal mass calibration the [M+H] + ion of the diisooctyl phthalate (m/z 391.28429), which is ubiquitously present in the solvent system was taken. The MS was operated in the PRM positive mode using collision energy of 25 and 35 for Sirolimus and Tacrolimus (ISTD), respectively; the accurate mass of the monitoring ions $\pm$10 mDa were used for test item and internal standard peak integration. Further analyzer settings were as follows: max. injection time 120 ms, sheath gas 40, aux gas 10, sweep gas 2, spray voltage 4 kV, capillary temperature 350°C, heater 350°C.

The table below gives an overview on the MS and chromatography parameters used for the test item and the internal standard.

*Table41: Overview on the MS conditions and retention time*

| Items | Molecular weight (g/mol) | Precursor Mass m/z | Scan range (m/z) | Quantitation Ion m/z | Retention time (min) |
|---|---|---|---|---|---|
| Sirolimus | 914.17 | 936.54 | 65.00-975.00 | 409.234 | 2.18 |
| Tacrolimus (ISTD) | 804.02 | 826.47 | 57.33-860.00 | 616.309 | 2.16 |

*Results*

Sirolimus was detected in the blood of all rats after sublingual administration; the maximum concentration was reached after 0.5 hours (t_max), as can be seem from the following table:

*Table: Results of 2022NCM001 (mean of 5 animals)*

| Time / h | mean blood concentration / ng/ml |
|---|---|
| 0 | 1.1 |
| 0.25 | 8.6 |
| 0.5 | 24.3 |
| 1 | 11.7 |
| 2 | 14.1 |
| 4 | 11.6 |
| 8 | 7.4 |
| 24 | 4.5 |
| **Pharmacokinetic analysis (m ean)** | |
| AUC(o-last) (ng*h/mL) | 183.1 |

*Conclusions*

The results of this study demonstrate that sublingual absorption of Sirolimus from a nanoparticular dispersion based on Eudragit E100 is possible in rats after only (!) 5 min application time.

**15.2 Sirolimus encapsulated in E100 in rats (2022NCM002)**

The pharmacokinetic profile of two different nanoparticular formulations of Sirolimus after sublingual administration in rats was evaluated. To eliminate the inter-individual variability, the same animals were administered in an intra-individual two-way cross-over after an appropriate washout period.

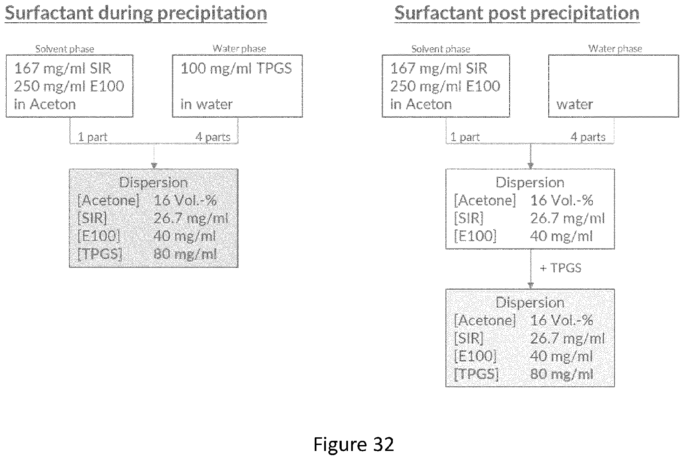

Two different approaches were followed in that a surfactant was included either during precipitation (**FLM04_025**) or after precipitation (**FLM02_b230**) as shown in Figure 32.

Formulations were freshly prepared on the day of application using the procedure as described herein.

*Table42: Preparation of formulation*

| | FLM04_025 | FLM02_b230 |
|---|---|---|
| Method | CF System | Batch |
| Solvent | Acetone | Acetone |
| Sirolimus in solvent | 167 mg/mL | 167 mg/mL |
| Components in solvent • Eudragit E100 | 250 mg/mL | 250 mg/mL |
| Non-solvent | Water | water |
| Components in non-solvent • TPGS1000 | 100 mg/ml | - |
| Mixing ratio | 1:4 (V/V) | 1:4 (V/V) |
| CF Reactor | Asymmetric cascade mixer | - |
| CF Reactor nozzle size | 500 $\mu$m | - |
| Flow rate solvent or volume | 6 mL/min | 1,0 mL |
| Flow rate non-solvent or volume | 24 mL/min | 4 mL |

(continued)

| | FLM04_025 | FLM02_b230 |
|---|---|---|
| Components in volumetric flask<br>• TPGS1000 | - | 80 mg/ml |

*Table: Composition of dispersion and characterisation*

| Formulation code | Composition | | | | Characterization | | | |
|---|---|---|---|---|---|---|---|---|
| | Acetone | [SIR] | [E100] | [TPGS] | pH | size | PDI | zeta |
| | v/v | mg/ml | mg/ml | mg/ml | | nm | | mV |
| FLM04_025 | 16 | mg/ml 26.7 | 40 | 80 | 6.53 | 134 | 0.274 | +24.6 |
| FLM02_b230 | 16 | 26.7 | 40 | 80 | 6.05 | 140 | 0.214 | +29.4 |

Test item formulations were administered sublingual to 5 animals with 7 days wash-out intervals at a dose of 5 mg per kg body weight. Application procedure, blood sampling and their as quantification was as described before.

*Results*

Sirolimus was detected in the blood of all rats after sublingual administration.

*Tabelle 43: Results of 2022NCM002*

| Formulation | FLM04_025 | FLM02_b230 |
|---|---|---|
| Time / h | mean blood concentration / ng/ml | mean blood concentration / ng/ml |
| 0 | 0.0 | 0.2 |
| 0.25 | 7.6 | 5.3 |
| 0.5 | 16.6 | 16.8 |
| 1 | 20.8 | 9.9 |
| 2 | 9.4 | 23.9 |
| 4 | 6.7 | 11.4 |
| 8 | 4.6 | 14.6 |
| **Pharmacokinetic analysis (mean)** | | |
| AUC(o-last) / ng*h/mL | 66.0 | 112.6 |
| AUC/dose/body weight / ng*h/mL/mg/kg | 13.5 | 22.8 |

*Conclusions*

The results of this study demonstrate (surprisingly) that sublingual absorption of Sirolimus from a nanoparticular dispersion based on Eudragit E100 is possible in rats after only (!) 5 min application time.

**15.3 Tacrolimus encapsulated in E100 in rats (2022NCM003)**

The pharmacokinetic profile of three different nanoparticular formulations of Tacrolimus after sublingual administration in five naive male Sprague Dawley rats with 14 days wash-out interval was evaluated. To eliminate the inter-individual variability, the same animals were administered in an intra-individual three-way cross-over after washout period. The animals were housed in a separate temperature-controlled room (20-24°C) and maintained in a 12h light/12h dark cycle. Food and water were available ad libitum throughout the duration of the study.

All experimental procedures were approved by and conducted in accordance with the regulations of the local Animal Welfare authorities (Landesamt für Gesundheit und Verbraucherschutz, Abteilung Lebensmittel- und Veterinärwesen, Saarbrücken, TV 2.4.2.2 14/2020).

Formulations were freshly prepared on the day of application using the procedure described herein and administered freshly after arrival.

*Table: Preparation of formulation*

| Formulation type | Tac/E100 | Tac/E100/TPGS |
|---|---|---|
| **Test item ID** | **FLM01_012** | **FLM01_b176** |
| Method | CF System | Batch |
| Solvent | Acetone | Acetone |
| Components in solvent<br>• Tacrolimus<br>• Eudragit E100 | 167 mg/ml<br>250 mg/mL | 167 mg/ml<br>250 mg/mL |
| Non-solvent | water | water |
| Components in non-solvent<br>• TPGS | - | 100 mg/ml |
| Mixing ratio | 1:4 (V/V) | 1:4 (V/V) |
| CF Reactor | Asymmetric cascade mixer | - |
| CF Reactor nozzle size | 500 μm | - |
| Flow rate solvent or volume | 6 mL/min | 1,0 mL |
| Flow rate non-solvent or volume | 24 mL/min | 4 mL |

*Table: Composition of dispersion and characterisation*

| Acetone | 16 % (V/V) | 16 % (V/V) |
|---|---|---|
| Tacrolimus | 26.7 mg/mL | 26.7 mg/mL |
| Eudragit E100 | 40 mg/mL | 40 mg/mL |
| TPGS1000 | | 80 mg/ml |
| HCl | 0.0374 mol/L | 0.0374 mol/L |
| Particle size (DLS) | 114 nm after preparation | 53.4 nm after preparation |
| PDI (DLS) | Multimodal after preparation (100x diluted. water) | Multimodal after preparation (10x diluted. water) |
| pH | 6.55 | 6.73 |
| Zeta-Potential | (+) 57.9 mV (10x diluted water) | (+) 24.3 mV (10x diluted water) |

Dose (and applied volume of the nanosuspension) was adjusted individually to the body weight of the animal. The treatment groups and the details of administration are presented in the table below.

*Table: Study design and schedule of 2022NCM003*

| Schedule | Day 1 | Day 15 |
|---|---|---|
| | 16.09.2022 | 30.09.2022 |
| | | |
| **Formulation type** | **Tac/E100** | **Tac/E100/TPGS** |
| Test item ID | **FLM01_012** | **FLM01_b176** |
| Dose/body weight / mg/kg | 5.00 | 5.10 |
| Body weight / g | 385 - 420 | 436 - 474 |

(continued)

| Formulation type | Tac/E100 | Tac/E100/TPGS |
|---|---|---|
| Test item ID | FLM01_012 | FLM01_b176 |
| Residence time / min | 5 | 5 |
| Application | sublingual | sublingual |

Test item formulations were administered sublingual to 5 animals with 7 days wash-out intervals at a dose of 5 mg per kg body weight. Application procedure and blood sampling was as described before.

Tacrolimus whole blood concentrations were analysed by LC-MS/MS and Cmax, tmax, and AUC were calculated.

**Liquid chromatography - mass spectrometry (LC-MS)**

*Preparation of working solutions for calibration standards and quality controls*

Individual stock solutions were used for preparation of calibration standards and quality control samples (QCs). Stock solutions of Sirolimus in DMSO (1 mg/mL) were diluted in DMSO to obtain a start solution and further working solutions.

*Preparation of blood samples*

All samples were kept on ice until the addition of the precipitation reagent. Calibration standards and quality controls (QCs) were prepared by spiking 20 μL blank blood with 2.4 μL of the corresponding working solution, then vortexed twice for 5 s with a 5-s break in between. Calibration standards and quality controls were prepared in duplicates.

A volume of 20 μL of each unknown sample, zero, blank and wash blank sample was spiked with 2.4 μL DMSO. The mixtures were vortexed twice for 5 s with a 5-s break in between. After 1 min of equilibration, a volume of 40 μL internal standard Sirolimus (1200 ng/ml) diluted in HFBA 0.2% in ACN was added to each calibration standard, QC sample, zero sample and unknown sample, while a volume of 40 μL plain ACN was added to all blanks and wash blanks. Samples were vortexed for 10 s and then centrifuged for 10 minutes at 8000 rpm and at room temperature.

Finally, an aliquot of 50 μL of the supernatant was diluted with 50 μL water in a fresh tube, vortex mixed for 10 seconds and an aliquot was transferred to LC-MS autosampler vials and subsequently subjected to LC-MS with an injection volume of 15 μl.

*LC-MS settings*

The HPLC system consisted of a Dionex UltiMate 3000 RS pump and Dionex UltiMate 3000 RS column compartment and an Accela Open Autosampler (Thermo Fisher Scientific, USA). Mass spectrometry was performed on a Q Exactive (Orbitrap) accurate mass spectrometer equipped with a heated electrospray (H-ESI) interface (Thermo Fisher Scientific, USA) connected to a PC running the standard software Chromeleon 7.2.

Analytes were separated on a Poroshell HPH-C18, 2.7u, 100×3 mm analytical column (Agilent Technologies) with a C6-Phenyl, 4×2.0 mm ID precolumn. The HPLC was performed in the gradient mode using Methanol as organic phase (A) and Ammonium acetate 10mM in water as aqueous phase (B) as given in table 45. The pump flow rate was set to 700 μl/min.

*Table: HPLC gradient*

| Mobile phase | 0 min | 0.1 min | 0.2 min | 2.4 min | 2.5 min | 4.5 min |
|---|---|---|---|---|---|---|
| A (%) | 25 | 25 | 97 | 97 | 25 | 25 |
| B (%) | 75 | 75 | 3 | 3 | 75 | 75 |

An optimized MS tune file was used and as a lock mass for internal mass calibration the [M+H]+ ion of the diisooctyl phthalate (m/z 391.28429), which is ubiquitously present in the solvent system was taken. The MS was operated in the PRM positive mode using collision energy of 25 and 35 for Sirolimus and Tacrolimus (ISTD), respectively; the accurate mass of the monitoring ions ±10 mDa were used for test item and internal standard peak integration. Further analyzer settings were as follows: max. injection time 120 ms, sheath gas 40, aux gas 10, sweep gas 2, spray voltage 4 kV, capillary temperature 350°C, heater 350°C. Table below gives an overview on the MS and chromatography parameters used for the test item and the internal standard.

Table: Overview on the MS conditions and retention time

| Items | Molecular weight (g/mol) | Precursor Mass m/z | Scan range (m/z) | Quantitation Ion m/z | Retention time (min) |
|---|---|---|---|---|---|
| Tacrolimus | 804.02 | 826.47 | 57.33-860.00 | 616.309 | 2.17 |
| Sirolimus (ISTD) | 914.17 | 936.54 | 65.00-975.00 | 409.234 | 2.19 |

**Results**

Tacrolimus was detected in the blood of all rats after sublingual administration; the maximum concentration was reached after 0.5 hours (t_max) as can be seen from the following table:

Table: Results of 2022NCM003

| Formulation type | Tac/E100 | Tac/E100/TPGS |
|---|---|---|
| Dose/body weight / mg/kg | 5.00 | 5.10 |
| | | |
| Time / h | mean blood concentration / ng/ml | mean blood concentration / ng/ml |
| 0 | 0.0 | 0.2 |
| 0.25 | 10.8 | 4.3 |
| 0.5 | 15.1 | 13.7 |
| 1 | 8.0 | 11.8 |
| 2 | 6.0 | 8.6 |
| 4 | 4.4 | 5.9 |
| 8 | 3.5 | 6.0 |
| | | |

| Pharmacokinetic analysis (mean) | | |
|---|---|---|
| c_max / ng/mL | 15.1 | 13.7 |
| t_max / h | 0.5 | 0.5 |
| AUC/dose/body weight | 8.74 | 11.32 |

**Conclusions**

The results of this study demonstrate (surprisingly) that sublingual absorption of Tacrolimus from a nanoparticular dispersion based on Eudragit E100 is possible in rats after only (!) 5 min application time.

**Claims**

1. A method of preparing an aqueous dispersion of nanoparticles of a hydrophobic active pharmaceutical ingredient (API) wherein said nanoparticles have a defined size range and comprise said hydrophobic active ingredient and at least one stabilizing agent, said stabilizing agent being a polymeric stabilizing agent having ionizable groups and/or ionic groups; said method comprising the steps:

   a) Providing in a single first space a first composition comprising

      - a hydrophobic active pharmaceutical ingredient (API) dissolved in a first amount of a suitable non-aqueous solvent;

- a polymeric stabilizing agent having ionizable groups that are ionizable by the addition of a suitable acid or suitable base, and/or having ionic groups, said polymeric stabilizing agent being also dissolved in said first amount of said non-aqueous solvent;

- a second amount of a suitable hydrophilic solvent, e.g. water or aqueous solution;

wherein, in said first composition, said suitable non-aqueous solvent and said suitable hydrophilic solvent become mixed, and wherein said first amount and said second amount in said first composition have a volume ratio in the range of from 20:1 to 2:1, preferably from 10:1 to 4:1;

wherein said second amount of a hydrophilic solvent additionally contains a third amount of a suitable acid or a suitable base, wherein said third amount of a suitable acid or suitable base is defined by the number of ionizable groups N in said polymeric stabilizing agent, such that said third amount of suitable acid or base achieves an ionization of 10% to 100% of N;

b) providing in a single second space a second composition comprising

- a suitable hydrophilic solvent, e.g. water or an aqueous solution; wherein, optionally, said suitable hydrophilic solvent further comprises one or several further solutes, such as a polymeric stabilizing agent which may be the same or different from the polymeric stabilizing agent provided in said first composition, and/or optionally further comprises one or several surfactants, and/or combinations of one or several further solutes and of one or several surfactants;

wherein said first and second space are separate from each other, and wherein the order of steps a) and b) is ab or ba, or both steps are performed synchronously or concomitantly;

c) mixing said first composition and said second composition together in a volume ratio of from 1:2 to 1:50, preferably from 1:3 to 1:20; more preferably from 1:4 to 1:10, thereby generating a stable aqueous dispersion of nanoparticles of said active pharmaceutical ingredient (API) stabilized by said polymeric stabilizing agent.

2. The method according to claim 1, wherein said polymeric stabilizing agent having ionizable groups and/or ionic groups is a poly(methacrylate), preferably selected from neutral protonizable poly(methacrylates), cationic poly(methacrylates), and anionic poly(methacrylates), or is a polyethyleneimine, poly(4-vinylpyridine), poly(L-lysine), or poly(L-arginine).

3. The method according to claim 2, wherein said polymeric stabilizing agent is a poly(methacrylate), and said poly(methacrylate) is a neutral protonizable or cationic poly(methacrylate) selected from poly(butyl methacrylate-co-(2-dimethylaminoethyl) methacrylate-co-methyl methacrylate), methyl methacrylate and diethylaminoethyl methacrylate copolymer, ammonio methacrylate copolymer type B, and ammonio methacrylate copolymer type A.

4. The method according to claim 2, wherein said polymeric stabilizing agent is a poly(methacrylate), and said poly(methacrylate) is an anionic poly(methacrylate) selected from methacrylic acid-methylmethacrylate-copolymers, preferably having a range of ratios of free carboxyl groups to ester groups of from 1:1 to 1:5, more preferably from 1:1 to 1:2.

5. The method according to any of claims 1 - 4, wherein said hydrophobic active pharmaceutical ingredient (API) is a drug that is insoluble in water and aqueous solutions and that is, preferably, selected from macrolides, non-steroidal antiinflammatory drugs, hormones, antibodies, steroids, anti-cancer agents, opioid drugs, and mixtures thereof, wherein more preferably said macrolide is selected from macrolide immune suppressants, macrolide antibiotics, and macrolide antifungals; wherein more preferably, said hydrophobic active pharmaceutical ingredient (API) is selected from tacrolimus, sirolimus, everolimus, pimecrolimus, erythromycin, clarithromycin, azithromycin, roxithromycin, josamycin, spiramycin, telithromycin, tylosin, fidaxomicin, nystatin, natamycin, amphotericin B; in particular, acetylsalicylic acid, ibuprofen, dexibuprofen, flurbiprofen, naproxen, ketoprofen, tiaprofenic acid, diclofenac, indomethacin, acemetacin, flufenamic acid, mefenamic acid, oxicams, e.g. piroxicam, tenoxicam, meloxicam, lornoxicam, nabumeton, rofecoxib, parecoxib, etoricoxib, celecoxib, alpha-atrial natriuretic peptide, arginine vasopressin, atropine, augrnerosen, atorvastatin, bevacizumab, calcitonins, chorionic gonadotropins, corticotropin, desmopressin, epibatidine, cetuximab, exenatide, trastuzumab, adalimumab, human insulin, ketoconazole, lanreotide, lutropin alpha, metoprolol, minoxidil, nesiritide, octreotide, paclitaxel, paracetamol, pegaptanib, recombinant follicle stimulating hormone, recombinant growth factors, infliximab, rituximab, sermorelin, somatotropin, a taxane derivative, taxol, teriparatide acetate, thyrotropin, triclosan, urofollitropin, omalizumab, actinomycin D, albendazole, aldosterone, alprazolam, amiodarone, amitriptyline, amprenavir, asimadoline, atorvastatin, bunitrolol, buspirone, camptothecin, carbamazepine, carvedilol, celiprolol, cyclosporine A, cimetidine, clotrimazole, colchicine, cortisone, daunorubicin, debrisoquine, dexamethasone, diazepam, digitoxin, digoxin, diltiazem, docetaxel, domperidone, doxorubicin, efavirenz, epirubicin, erythromycin, ergotamine, estradiol, estradiol glucuronide, erlotinib, etoposide, phenytoin, fentanyl, felodipine, phenothiazines, fexofenadine, fluoroquinolones, fluorouracil, gentamicin, griseofulvin, hydrocortisone, imatinib, indinavir, itraconazole, ivermectin, ketoconazole, kaempferol, levofloxacin, lidocaine, loperamide, losartan, lovastatin, mebendazole, methylprednisolone, methotrexate, mibefradil, midazolam, nisoldipine, morphine, nelfinavir, nicardipine, nitrendipine, nifedipine, ondansetron, paclitaxel, pentazocine, praziquantel, prednisolone, prednisone, quercetin, quinidine, ranitidine, rifabutin, rifampicin, ritonavir, saquinavir, sulfame-thizole, tamoxifen, talinolol, teniposide, terfenadine, tetracycline, topotecan, triamcinolone, valspodar, verapamil, vinblastine, vincristine, vindesine, zopiclone, and mixtures thereof.

6. The method according to claim 5, wherein the hydrophobic active pharmaceutical ingredient (API) is a macrolide, and the macrolide is selected from macrolide immune suppressants, macrolide antibiotics, and macrolide antifungals, wherein

   - the macrolide immune suppressant is selected from tacrolimus, sirolimus, everolimus and pimecrolimus;
   - the macrolide antibiotic is selected from erythromycin, clarithromycin, azithromycin, roxithromycin, josamycin, spiramycin, telithromycin, tylosin, and fidaxomicin; and
   - the macrolide antifungal is selected from polyenes, in particular nystatin, natamycin, and amphotericin B.

7. The method according to any of the foregoing claims, wherein said suitable non-aqueous solvent is selected from acetone, dimethyl sulfoxide, ethanol, methanol, 1-propanol, 2-propanol, tetrahydrofuran, acetic acid, acetonitrile, anisole, 1-butanol, 2-butanol, t-butyl alcohol, 2-methyl-1-propanol, 3-methyl-1-butanol, 1-pentanol, butyl acetate, chloroform, cyclohexane, 1,1,-diethoxypropane, 1,1-dimethoxymethane, 1,2-dimethoxyethane, 1,4-dioxane, 2,2-dimethoxypropane, dichloromethane, diethyl ether, di-iso-propyl ether, dimethylformamide, 2-ethoxyethanol, ethyl acetate, ethyl formate, ethylene glycol (1,2-ethanediol), formic acid, heptane, hexane, isobutyl acetate, isopropyl acetate, 2-methoxyethanol, 1-methyl-2-pyrrolidone, methyl acetate, methyl t-butyl ether, methyl butyl ketone, methyl cyclohexane, methyl ethyl ketone (MEK), methyl isobutyl ketone, methyl isopropyl ketone, methyl tetrahydrofuran, n-methyl pyrrolidone, pentane, petroleum ether, propyl acetate, pyridine, sulfolane, 2,2,4-trimethylpentan (i-octane), toluol, trichloroacetic acid, trichloroethylene, trifluoracetic acid, xylol, and any combination of the foregoing, wherein, preferably, said suitable non-aqueous solvent is miscible with water and, more preferably is selected from acetone, DMSO, ethanol, methanol, isopropanol, 1-propanol, acetonitrile. 1,4 dioxane, tetrahydrofuran, and mixtures of the foregoing, wherein, even more preferably, said suitable non-aqueous solvent is selected from acetone, DMSO, ethanol and isopropanol.

8. The method according to any of the foregoing claims, wherein said suitable acid is selected from inorganic and organic acids, such as hydrochloric acid, nitric acid, sulfuric acid, acetic acid, oleic acid, propionic acid, pentanoic acid, caprylic acid, succinic acid, benzoic acid, fumaric acid, citric acid, glutamic acid, aspartic acid, citric acid, malic acid; butyric acid, and aminopolycarboxylic acids, such as iminodiacetic acid (IDA), nitrilotriacetic acid (NTA), ethylenediaminetetraacetic acid (EDTA), diethylenetriaminepentaacetic acid (DTPA), Ethylene-bis(oxyethylene-nitrilo)-tetraacetic acid) (EGTA), (1,2-bis(*o*-aminophenoxy)ethane-*N,N,N',N'*-tetraacetic acid) (BAPTA), 2,2',2'',2'''-(1,4,7,10-Tetraazacyclododecane-1,4,7,10-tetrayl)tetraacetic acid (DOTA), ethylenediamine-*N,N'*-bis(2-hydroxyphenylacetic acid) (EDDHA), and Ethylenediamine-*N,N'*-disuccinic acid (EDDS); and said suitable base is selected from NaOH, KOH, triethylamine, arginine, L-lysine, L-histidine, ammonium hydroxide.

9. The method according to any of the foregoing claims, wherein said mixing step c) is performed by a suitable mixing technology allowing for the intimate and immediate mixing of said first and second compositions, wherein, preferably, said mixing technology is selected from split and recombine mixing (SAR), mixing by impinging-jet mixers, e.g. microjet reactor mixing (MJR), stirring in a vessel, e.g. a batch reactor, mixing by T- and/or Y shaped fluidic mixers, mixing by static mixers, in-line rotor stator mixing, dynamic in-line mixing, and ultrasonic mixing.

10. The method according to any of the foregoing claims, wherein said defined size range is from 10 nm to 500 nm, preferably from 20 nm to 350 nm, more preferably from 20 nm to 250 nm, even more preferably from 50 nm to 200 nm.

11. A plurality of nanoparticles, e.g. a dispersion of nanoparticles, produced by the method according to any of the foregoing claims, comprising a hydrophobic active pharmaceutical ingredient (API) and at least one stabilizing agent, said nanoparticles having a defined size range which is from 10 nm to 500 nm, preferably from 20 nm to 350 nm, more preferably from 20 nm to 250 nm, even more preferably from 50 nm to 200 nm and having one or several of the following structural features:

- Said hydrophobic active pharmaceutical ingredient (API) is present in said nanoparticles in amorphous form and does not show any XRPD signals attributable to crystalline form(s) of said API;

- said nanoparticles exhibit mucoadhesive properties, as measured by an increase in zeta-potential that is observed if said plurality of nanoparticles, e.g. of said dispersion of nanoparticles, are mixed with a 0.5 % (w/w) aqueous suspension of mucin, wherein a mixture of said plurality of nanoparticles, e.g. of said dispersion of nanoparticles, and of said aqueous suspension of mucin has a zeta potential that is increased in comparison to an aqueous suspension of mucin alone without any nanoparticles; and wherein, preferably, said increase in zeta potential is observed with increasing amounts of said plurality of nanoparticles, e.g. of said dispersion of nanoparticles, being mixed with said aqueous suspension of mucin, such that a first mixture of a first amount of said plurality of nanoparticles with a defined amount of a 0.5 % (w/w) aqueous suspension of mucin has a higher zeta potential than a second mixture of a second amount of said plurality of nanoparticles with the same defined amount of a 0.5 % (w/w) aqueous suspension of mucin, if said first amount of nanoparticles is greater than said second amount of nanoparticles

- said hydrophobic active pharmaceutical ingredient (API) is present in said nanoparticles in a solid or immobilized state, as measured by $^1$H-NMR spectroscopy, and does not show any $^1$H-NMR signals attributable to a liquid or dissolved or mobile state of said API.

12. A pharmaceutical composition comprising a plurality of nanoparticles, e.g. a dispersion of nanoparticles, according to claim 11.

13. A plurality of nanoparticles according to claim 11 or a pharmaceutical composition according to claim 12, for use in the treatment of a disease.

Figure 1

Figure 2

Figure 3

Figure 4

Figure 5

Figure 6

Figure 7

Figure 8

Figure 9

Figure 10

Figure 11

Figure 12

Figure 13

A                                                                    B

Figure 14

Figure 15

A

B

Figure 16

Figure 17

Figure 18

Figure 19

Figure 20

Figure 21

Figure 22

Figure 23

Figure 24

Figure 25

Figure 26

Figure 27

Figure 28

Figure 29

Figure 30

Figure 31

## Surfactant during precipitation

Solvent phase | Water phase

```
167 mg/ml SIR        100 mg/ml TPGS
250 mg/ml E100
in Aceton            in water
```

1 part | 4 parts

```
          Dispersion
[Acetone]    16 Vol.-%
[SIR]        26.7 mg/ml
[E100]       40 mg/ml
[TPGS]       80 mg/ml
```

## Surfactant post precipitation

Solvent phase | Water phase

```
167 mg/ml SIR
250 mg/ml E100
in Aceton            water
```

1 part | 4 parts

```
          Dispersion
[Acetone]    16 Vol.-%
[SIR]        26.7 mg/ml
[E100]       40 mg/ml
```

+ TPGS

```
          Dispersion
[Acetone]    16 Vol.-%
[SIR]        26.7 mg/ml
[E100]       40 mg/ml
[TPGS]       80 mg/ml
```

Figure 32

# EUROPEAN SEARCH REPORT

**Application Number**

EP 23 18 9189

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 251 006 A1 (TORAY INDUSTRIES [JP]) 17 November 2010 (2010-11-17) | 11-13 | INV. A61K9/51 |
| Y | * the whole document * <br> * claims 1-15; examples 1-31 * | 1-13 | A61K31/436 |
| X | WO 99/00113 A1 (VIVORX PHARMACEUTICALS INC [US]; DESAI NEIL P [US] ET AL.) 7 January 1999 (1999-01-07) * the whole document * * claims 1-65; examples 1-66 * | 11-13 | |
| X | EP 2 510 930 A1 (BIONANOPLUS S L [ES]) 17 October 2012 (2012-10-17) * the whole document * * claims 1-15 * | 11-13 | |
| X | EP 3 662 894 A1 (LEON NANODRUGS GMBH [DE]) 10 June 2020 (2020-06-10) | 11-13 | |
| Y | * the whole document * * claims 1-20 * | 1-13 | |
| A | BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368 * the whole document * | 1-13 | **TECHNICAL FIELDS SEARCHED (IPC)** <br><br> A61K |

The present search report has been drawn up for all claims

| Place of search | Date of completion of the search | Examiner |
|---|---|---|
| Munich | 18 January 2024 | Felder, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 23 18 9189

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2024

| Patent document cited in search report | | | Publication date | Patent family member(s) | | | Publication date |
|---|---|---|---|---|---|---|---|
| EP 2251006 | A1 | | 17-11-2010 | AU | 2009216216 | A1 | 27-08-2009 |
| | | | | BR | PI0905790 | A2 | 14-07-2015 |
| | | | | CA | 2715665 | A1 | 27-08-2009 |
| | | | | CN | 101932311 | A | 29-12-2010 |
| | | | | DK | 2251006 | T3 | 16-10-2017 |
| | | | | EP | 2251006 | A1 | 17-11-2010 |
| | | | | ES | 2635515 | T3 | 04-10-2017 |
| | | | | HU | E035331 | T2 | 02-05-2018 |
| | | | | JP | 5712485 | B2 | 07-05-2015 |
| | | | | JP | WO2009104706 | A1 | 23-06-2011 |
| | | | | KR | 20100120119 | A | 12-11-2010 |
| | | | | PL | 2251006 | T3 | 29-12-2017 |
| | | | | PT | 2251006 | T | 13-10-2017 |
| | | | | RU | 2010138925 | A | 27-03-2012 |
| | | | | US | 2011003007 | A1 | 06-01-2011 |
| | | | | WO | 2009104706 | A1 | 27-08-2009 |
| WO 9900113 | A1 | | 07-01-1999 | AU | 8266298 | A | 19-01-1999 |
| | | | | BR | 9810945 | A | 05-02-2002 |
| | | | | CA | 2294981 | A1 | 07-01-1999 |
| | | | | CN | 1267214 | A | 20-09-2000 |
| | | | | CN | 1515246 | A | 28-07-2004 |
| | | | | DK | 1023050 | T3 | 14-10-2013 |
| | | | | EP | 1023050 | A1 | 02-08-2000 |
| | | | | ES | 2435944 | T3 | 26-12-2013 |
| | | | | HK | 1030543 | A1 | 11-05-2001 |
| | | | | HU | 0003972 | A2 | 28-08-2001 |
| | | | | JP | 4865937 | B2 | 01-02-2012 |
| | | | | JP | 5405527 | B2 | 05-02-2014 |
| | | | | JP | 2002507976 | A | 12-03-2002 |
| | | | | JP | 2011219482 | A | 04-11-2011 |
| | | | | KR | 20010014254 | A | 26-02-2001 |
| | | | | KR | 20050042507 | A | 09-05-2005 |
| | | | | KR | 20070091051 | A | 06-09-2007 |
| | | | | MX | 337149 | B | 15-02-2016 |
| | | | | NO | 332166 | B1 | 09-07-2012 |
| | | | | NO | 340319 | B1 | 27-03-2017 |
| | | | | NZ | 502500 | A | 28-03-2002 |
| | | | | NZ | 525580 | A | 27-08-2004 |
| | | | | PT | 1023050 | E | 04-12-2013 |
| | | | | SG | 113402 | A1 | 29-08-2005 |
| | | | | US | 2008160095 | A1 | 03-07-2008 |
| | | | | US | 2008161382 | A1 | 03-07-2008 |
| | | | | WO | 9900113 | A1 | 07-01-1999 |
| EP 2510930 | A1 | | 17-10-2012 | AR | 087149 | A1 | 26-02-2014 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 23 18 9189

This annex lists the patent family members relating to the patent documents cited in the above-mentioned European search report.
The members are as contained in the European Patent Office EDP file on
The European Patent Office is in no way liable for these particulars which are merely given for the purpose of information.

18-01-2024

| Patent document cited in search report | | Publication date | Patent family member(s) | | Publication date |
|---|---|---|---|---|---|
| | | | AU | 2012241758 A1 | 14-11-2013 |
| | | | CA | 2833188 A1 | 18-10-2012 |
| | | | CN | 103619918 A | 05-03-2014 |
| | | | EP | 2510930 A1 | 17-10-2012 |
| | | | EP | 2699625 A1 | 26-02-2014 |
| | | | ES | 2730119 T3 | 08-11-2019 |
| | | | JP | 6001640 B2 | 05-10-2016 |
| | | | JP | 2014513082 A | 29-05-2014 |
| | | | MX | 346361 B | 15-03-2017 |
| | | | PT | 2699625 T | 24-06-2019 |
| | | | US | 2014161892 A1 | 12-06-2014 |
| | | | WO | 2012140252 A1 | 18-10-2012 |
| EP 3662894 A1 | 10-06-2020 | AU | 2019392718 A1 | 10-06-2021 |
| | | CA | 3063417 A1 | 04-06-2020 |
| | | CN | 113242732 A | 10-08-2021 |
| | | DK | 3662894 T3 | 20-09-2021 |
| | | DK | 3846783 T3 | 15-01-2024 |
| | | EP | 3662894 A1 | 10-06-2020 |
| | | EP | 3846783 A1 | 14-07-2021 |
| | | ES | 2894112 T3 | 11-02-2022 |
| | | HU | E056067 T2 | 28-01-2022 |
| | | IL | 283458 A | 29-07-2021 |
| | | JP | 2022513697 A | 09-02-2022 |
| | | KR | 20210100142 A | 13-08-2021 |
| | | PL | 3662894 T3 | 10-01-2022 |
| | | SG | 11202105778P A | 29-06-2021 |
| | | SI | 3662894 T1 | 30-11-2021 |
| | | US | 2020170934 A1 | 04-06-2020 |
| | | US | 2022079872 A1 | 17-03-2022 |
| | | WO | 2020115140 A1 | 11-06-2020 |

EPO FORM P0459

## REFERENCES CITED IN THE DESCRIPTION

**Non-patent literature cited in the description**

- **MARQUES, M. R. C.** ; **LOEBENBERG, R.** ; **ALMU-KAINZI, M.** Simulated Biological Fluids with Possible Application in Dissolution Testing. *Dissolution Technol.*, 2011, vol. 18, 15-28

- **SWINDELLS**. The X ray crystal structure of Sirolimus. *Canadian Journal of Chemistry*, 1978, vol. 56, 2491